# EUROPEAN PATENT SPECIFICATION

(11) **EP 1 090 023 B1**
(45) Date of publication and mention of the grant of the patent: **10.09.2003**
(21) Application number: 99934530.9
(22) Date of filing: 23.06.1999
(51) Int. Cl.: C07H 19/167, A61K 31/70, C07H 15/04

(54) **2-(PURIN-9-YL)-TETRAHYDROFURAN-3,4-DIOL DERIVATIVES**
2-(PURIN-9-YL)-TETRAHYDROFURAN-3,4-DIOL DERIVATE
DERIVES DU 2-(PURIN-9-YL)-TETRAHYDROFURAN-3,4 DIOL

(30) Priority: 23.06.1998 GB 9813565
(43) Date of publication of application: 11.04.2001
(73) Proprietor: GLAXO GROUP LIMITED, Greenford, Middlesex UB6 0NN (GB)
(72) Inventor: ALLEN, David George, Stevenage, Hertfordshire SG1 2NY (GB); CHAN, Chuen, Stevenage, Hertfordshire SG1 2NY (GB); COOK, Caroline Mary, Stevenage, Hertfordshire SG1 2NY (GB); COUSINS, Richard Peter Charles, Stevenage, Hertfordshire SG1 2NY (GB); COX, Brian, Stevenage, Hertfordshire SG1 2NY (GB); DYKE, Hazel Joan, Chiroscience Ltd., Cambridge CB4 4WE (GB); ELLIS, Frank, Stevenage, Hertfordshire SG1 2NY (GB); GEDEN, Joanna Victoria, OSI Pharm. (Aston Molec.), Birmingham B7 4EJ (GB); HOBBS, Heather, Stevenage, Hertfordshire SG1 2NY (GB); REDGRAVE, Alison Judith, Stevenage, Hertfordshire SG1 2NY (GB); SWANSON, Stephen, Stevenage, Hertfordshire SG1 2NY (GB); BAYS, David, Ware, Hertfordshire SG12 9PE (GB)
(74) Representative: Teuten, Andrew John
(86) International application number: EP9904267
(87) International publication number: WO99067264

(56) References cited:
- WO-A-94/17090
- WO-A-98/01459
- WO-A-98/28319
- WO-A-99/38877
- WO-A-99/41267

## Description

This invention relates to new chemical compounds, processes for their preparation, pharmaceutical formulations containing them and their use in therapy.

Inflammation is a primary response to tissue injury or microbial invasion and is characterised by leukocyte adhesion to the endothelium, diapedesis and activation within the tissue. Leukocyte activation can result in the generation of toxic oxygen species (such as superoxide anion), and the release of granule products (such as peroxidases and proteases). Circulating leukocytes include neutrophils, eosinophils, basophils, monocytes and lymphocytes. Different forms of inflammation involve different types of infiltrating leukocytes, the particular profile being regulated by the profile of adhesion molecule, cytokine and chemotactic factor expression within the tissue.

The primary function of leukocytes is to defend the host from invading organisms such as bacteria and parasites. Once a tissue is injured or infected a series of events occurs which causes the local recruitment of leukocytes from the circulation into the affected tissue. Leukocyte recruitment is controlled to allow for the orderly destruction and phagocytosis of foreign or dead cells, followed by tissue repair and resolution of the inflammatory infiltrate. However in chronic inflammatory states, recruitment is often inappropriate, resolution is not adequately controlled and the inflammatory reaction causes tissue destruction.

There is evidence from both *in vitro* and *in vivo* studies to suggest that compounds active at the adenosine A2a receptor will have anti-inflammatory actions. The area has been reviewed by Cronstein (1994). Studies on isolated neutrophils show an A2 receptor-mediated inhibition of superoxide generation, degranulation, aggregation and adherence (Cronstein et al, 1983 and 1985; Burkey and Webster, 1993; Richter, 1992; Skubitz et al, 1988. When agents selective for the A2a receptor over the A2b receptor (eg CGS21680) have been used, the profile of inhibition appears consistent with an action on the A2a receptor subtype (Dianzani et al, 1994). Adenosine agonists may also down-regulate other classes of leucocytes (Elliot and Leonard, 1989; Peachell et al, 1989). Studies on whole animals have shown the anti-inflammatory effects of methotrexate to be mediated through adenosine and A2 receptor activation (Asako et al, 1993; Cronstein et al, 1993 and 1994). Adenosine itself, and compounds that raise circulating levels of adenosine also show anti-inflammatory effects *in vivo* (Green et al, 1991; Rosengren et al, 1995). In addition raised levels of circulating adenosine in man (as a result of adenosine deaminase deficiency) results in immunosuppression (Hirschorn, 1993).

Certain substituted 4'-carboxamido and 4'-thioamido adenosine derivatives which are useful for the treatment of inflammatory diseases are described in International Patent Application Nos. WO94/17090, WO96/02553, WO96/02543 (Glaxo Group). Substituted 4'-carboxamidoadenosine derivatives useful in the treatment of dementia are described in AU 8771946 (Hoechst Japan). Substituted 4'-hydroxymethyl adenosine derivatives which are useful for the treatment of gastrointestinal motility disorders are described in EP-A-423776 and EP-A-423777 (Searle). Substituted 4'-hydroxymethyl adenosine derivatives which are useful as platelet aggregation inhibitors are described in BE-768925 (Takeda). 4'-Hydroxymethyl adenosine derivatives and 4'-esters thereof which are useful as anti-hypertensive agents or have other cardiovascular activity are described in US 4663313, EP 139358 and US 4767747 (Warner Lambert), US 4985409 (Nippon Zoki) and US 5043325 (Whitby Research). 4-Hydroxymethyladenosine derivatives useful in the treatment of autoimmune disorders are described in US 5106837 (Scripps Research Institute). 4'-Hydroxymethyladenosine derivatives useful as anti-allergic agents are described in US 4704381 (Boehringer Mannheim). Certain 4'-tetrazolylalkyl adenosine derivatives which are useful in the treatment of heart and circulatory disorders are generically described in DT-A-2621470 (Pharma-Waldhof). Other 4'-carboxamidoadenosine derivatives useful in the treatment of cardiovascular conditions are described in US 5219840, GB 2203149 and GB 2199036 (Sandoz), WO94/02497 (US Dept. Health), US 4968697 and EP 277917 (Ciba Geigy), US 5424297 (Univ. Virginia) and EP 232813 (Warner Lambert). Other 4'-carboxamidoadenosine derivatives lacking substitution on the purine ring in the 2-position are described in DT 2317770, DT 2213180, US 4167565, US 3864483 and US 3966917 (Abbott Labs), DT 2034785 (Boehringer Mannheim), JP 58174322 and JP 58167599 (Tanabe Seiyaku), WO92/05177 and US 5364862 (Rhone Poulenc Rorer), EP 66918 (Procter and Gamble), WO86/00310 (Nelson), EP 222330, US 4962194, WO88/03147 and WO88/03148 (Warner Lambert) and US 5219839, WO95/18817 and WO93/14102 (Lab UPSA). 4'-Hydroxymethyladenosine derivatives lacking substitution on the purine ring in the 2-position are described in WO95/11904 (Univ Florida). 4'-Substituted adenosine derivatives useful as adenosine kinase inhibitors are described in WO94/18215 (Gensia). Other 4'-halomethyl, methyl, thioalkylmethyl or alkoxymethyl adenosine derivatives are described in EP 161128 and EP 181129 (Warner Lambert) and US 3983104 (Schering). Other 4'-carboxamidoadenosine derivatives are described in US 7577528 (NIH), WO91/13082 (Whitby Research) and WO95/02604 (US Dept Health).

Certain tetrazole containing deoxynucleotides which were found to lack antiinfective activity are described in Baker et al (1974) Tetrahedron 30, 2939-2942. Other tetrazole containing adenosine derivatives which show activity as platelet aggregation inhibitors are described in Mester and Mester (1972) Pathologie-Biologie, 20 (Suppl) 11-14. Certain nitrile containing ribose derivatives are described in Schmidt et al (1974) Liebigs. Ann. Chem. 1856-1863.

Other publications include: WO 98/16539 (Novo Nordisk A/S) which describes adenosine derivatives for the treatment of myocardial and cerebral ischaemia and epilepsy; WO 98/01426 (Rhone-Poulenc Rorer Pharmaceuticals Inc.) which relates to adenosine derivatives possessing antihypertensive, cardioprotective, anti-ischaemic and antilipolytic properties; and WO 98/01459 (Novo Nordisk A/S) which describes *N*,9-disubstituted adenine derivatives which are substituted in the 4' position by unsubstituted oxazolyl or isoxazolyl and the use of such compounds for the treatment of disorders involving cytokines in humans. WO 98/28319 (Glaxo Group Limited) was published subsequent to the earliest priority date of this application and describes 4'-substituted tetrazole 2-(purin-9-yl)-tetrahydrofuran-3,4-diol derivatives;

We have now found a novel group of compounds with broad anti-inflammatory properties which inhibit leukocyte recruitment and activation and which are agonists of the adenosine 2a receptor. The compounds are therefore of potential therapeutic benefit in providing protection from leukocyte-induced tissue damage in diseases where leukocytes are implicated at the site of inflammation. The compounds of the invention may also represent a safer alternative to corticosteroids in the treatment of inflammatory diseases, whose uses are severely limited by their side-effect profiles.

More particularly, the compounds of this invention may show an improved profile over known A2a-selective agonists in that they generally lack agonist activity at the human A3 receptor. This profile can be considered of benefit as A3 receptors are also found on leucocytes (eg eosinophil) and other inflammatory cells (eg mast cell) and activation of these receptors may have pro-inflammatory effects (Kohno et al, 1996; Van Schaick et al 1996). It is even considered that the bronchoconstrictor effects of adenosine in asthmatics may be mediated via the adenosine A3 receptor (Kohno et al, 1996).

Thus, according to the invention we provide compounds of formula (I): 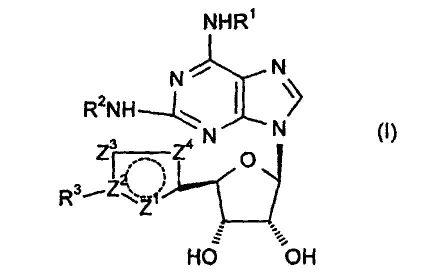 wherein R¹ and R² independently represent a group selected from:
(i) C₃₋₈cycloalkyl-;
(ii) hydrogen;
(iii) aryl₂CHCH₂-;
(iv) C₃₋₈cycloalkylC₁₋₆alkyl-;
(v) C₁₋₈alkyl-;
(vi) arylC₁₋₆alkyl-;
(vii) R⁴R⁵N-C₁₋₆alkyl-;
(viii) C₁₋₆alkyl-CH(CH₂OH)-;
(ix) arylC₁₋₅alkyl-CH(CH₂OH)-;
(x) arylC₁₋₅alkyl-C(CH₂OH)₂-;
(xi) C₃₋₈cycloalkyl independently substituted by one or more (e.g. 1, 2 or 3) -(CH₂)ₚR⁶ groups;
(xii) H₂NC(=NH)NHC₁₋₆alkyl-;
(xiii) a group of formula 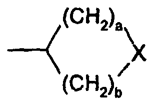 or such a group in which one methylene carbon atom adjacent to X, or both if such exist, is substituted by methyl;
(xiv) -C₁₋₆alkyl-OH;
(xv) -C₁₋₈haloalkyl;
(xvi) a group of formula 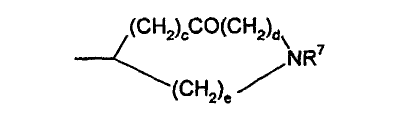
(xvii) aryl; and
(xviii) -(CH₂)_{f}SO₂NH_{g}(C₁₋₄alkyl-)_{2-g} or -(CH₂)_{f}SO₂NH_{g}(arylC₁₋₄alkyl-)_{2-g} where f is 2 or 3 and g is an integer 0 to 2;
Z² represents C or N;
Z¹, Z³ and Z⁴ together with Z² and the carbon atom form a 5-membered heterocyclic aromatic ring;
R³ represents C₁₋₃alkyl or cyclopropyl, save that where Z² represents C, R³ may also represent CH₂OH.
R⁴ and R⁵ independently represent hydrogen, C₁₋₆alkyl, aryl, arylC₁₋₆alkyl- or NR⁴R⁵ together may represent pyridinyl, pyrrolidinyl, piperidinyl, morpholinyl, azetidinyl, azepinyl, piperazinyl or N-C₁₋₆alkylpiperazinyl;
R⁶ represents OH, NH₂, NHCOCH₃ or halogen;
R⁷ represents hydrogen, C₁₋₆alkyl, -C₁₋₆alkylaryl or -COC₁₋₆alkyl;
X represents NR⁷, O, S, SO or SO₂;
p represents 0 or 1;
a and b independently represent an integer 0 to 4 provided that a + b is in the range 3 to 5;
c, d and e independently represent an integer 0 to 3 provided that c + d + e is in the range 2 to 3;
   with the proviso that the moiety 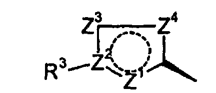 does not represent one of the following groups: 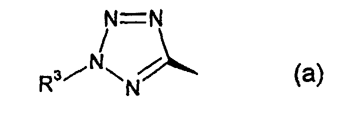 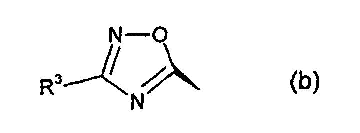 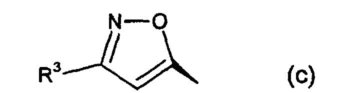 and salts and solvates thereof.

Z¹, Z³ and Z⁴ will independently represent C, N, O or S and, in the case of C and N, together with a sufficient number of hydrogen atoms to provide the ring with aromatic character. At least one of Z¹, Z², Z³, Z⁴ and Z⁵ will represent a heteroatom. Preferably at least one of Z¹, Z³ and Z⁴ will represent a nitrogen atom. More preferably at least one of Z¹, Z³ and Z⁴ will represent a nitrogen atom and at least one of the remainder will represent C or N. We prefer that two or three of Z¹, Z², Z³ and Z⁴ are heteroatoms.

References to C_{x-y}alkyl include references to an aliphatic hydrocarbon grouping containing x to y carbon atoms which may be straight chain or branched and may be saturated or unsaturated. References to alkoxy may also be interpreted similarly.

References to aryl include references to mono- and bicyclic carbocyclic aromatic rings (e.g. phenyl, naphthyl) and heterocyclic aromatic rings, for example containing 1-3 hetero atoms selected from N, O and S (e.g. pyridinyl, pyrimidinyl, thiophenyl, imidazolyl, quinolinyl, furanyl, pyrrolyl, oxazolyl) all of which may be optionally substituted, e.g. by C₁₋₆alkyl, halogen, hydroxy, nitro, C₁₋₆alkoxy, cyano, amino, SO₂NH₂ or -CH₂OH.

Examples of C₃₋₈cycloalkyl for R¹ and R² include monocyclic alkyl groups (e.g. cyclopentyl, cyclohexyl) and bicyclic alkyl groups (e.g. norbornyl such as exo-norborn-2-yl).
Examples of (aryl)₂CHCH₂- for R¹ and R² include Ph₂CHCH₂- or such a group in which one or both phenyl moieties is substituted, e.g. by halogen or C₁₋₄alkyl. Examples of C₃₋₈cycloalkylC₁₋₆alkyl- for R¹ and R² include ethylcyclohexyl. Examples of C₁₋₈alkyl for R¹ and R² include -(CH₂)₂C(Me)₃, -CH(Et)₂ and CH₂=C(Me)CH₂CH₂-.
Examples of arylC₁₋₆alkyl- for R¹ and R² include -(CH₂)₂Ph, -CH₂Ph or either in which Ph is substituted (one or more times) by halogen (e.g. iodine), amino, methoxy, hydroxy, -CH₂OH or SO₂NH₂; -(CH₂)₂ pyridinyl (e.g. -(CH₂)₂pyridin-2-yl) optionally substituted by amino; (CH₂)₂imidazolyl (e.g. 1H-imidazol-4-yl) or this group in which imidazoyl is N-substituted by C₁₋₆alkyl (especially methyl). Examples of R⁴R⁵N-C₁₋₆alkyl- for R¹ and R² include ethyl-piperidin-1-yl, ethyl-pyrrolidin-1-yl, ethyl-morpholin-1-yl, -(CH₂)₂NH(pyridin-2-yl) and -(CH₂)₂NH₂. Examples of C₁₋₆alkyl-CH(CH₂OH)- for R¹ and R² include Me₂CHCH(CH₂OH)-. Examples of arylC₁₋₅alkyl-CH(CH₂OH)- for R¹ and R² include PhCH₂CH(CH₂OH)-particularly

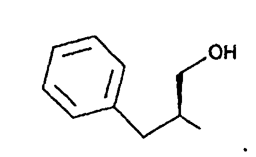

Examples of arylC₁₋₅alkyl-C(CH₂OH)₂- for R¹ and R² include PhCH₂C(CH₂OH)₂-.

Examples of C₃₋₈ cycloalkyl independently substituted by one or more -(CH₂)ₚR⁶ groups (eg 1, 2 or 3 such groups) for R¹ and R² include 2-hydroxy-cyclopentyl and 4-aminocyclohexyl (especially trans-4-amino-cyclohexyl).
Examples of H₂NC(=NH)NHC₁₋₆alkyl for R¹ and R² include H₂NC(=NH)NH(CH₂)₂-Examples of groups of formula 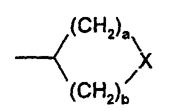 for R¹ and R² include pyrrolidin-3-yl, piperidin-3-yl, piperidin-4-yl, tetrahydro-1,1-dioxide thiophen-3-yl, tetrahydropyran-4-yl, tetrahydrothiopyran-4-yl and 1,1-dioxo-hexahydro-1.lamda.6-thiopyran-4-yl, or a derivative in which the ring nitrogen is substituted by C₁₋₆alkyl (e.g. methyl), C₁₋₆alkylacyl (e.g. acetyl), arylC₁₋₆alkyl- (e.g. benzyl).
Examples of -C₁₋₆alkyl-OH groups for R¹ and R² include -CH₂CH₂OH and -CH(CH₂OH)CH(CH₃)₂.
Examples of C₁₋₈haloalkyl for R¹ and R² include -CH₂CH₂Cl and (CH₃)₂ClC(CH₂)₃-.
Examples of groups of formula 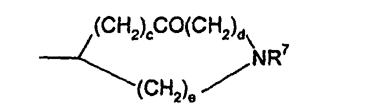 for R¹ and R² include 2-oxopyrrolidin-4-yl, 2-oxopyrrolidin-3-yl or a derivative in which the ring nitrogen is substituted by C₁₋₆alkyl (e.g. methyl) or benzyl. Examples of aryl for R¹ and R² include phenyl optionally substituted by halogen (e.g. fluorine, especially 4-fluorine).
An example of a -(CH₂)_{f}SO₂NH_{g}(C₁₋₄alkyl)_{2-g} group for R¹ and R² is -(CH₂)₂SO₂NHMe, and an example of a -(CH₂)_{f}SO₂NH_{g}(arylC₁₋₄alkyl)_{2-g} group for R¹ and R² is -(CH₂)₂SO₂NHCH₂Ph.

An example of C₁₋₆alkyl for R⁷ is methyl, an example of C₁₋₆alkylaryl for R⁷ is benzyl, and an example of -COC₁₋₆alkyl for R⁷ is acetyl.

We prefer that R¹ and R² do not both represent hydrogen.
We prefer R¹ to represent aryl₂CHCH₂- C₁₋₈alkyl-, hydrogen or arylC₁₋₆alkyl-.
We prefer R² to represent -CH(CH₂OH)C₁₋₃alkyl, 4-aminocyclohexyl, pyrrolidinyl or arylCH₂CH₂-, especially where aryl represents (1-C₁₋₃alkyl-1H-imidazoyl-4-yl).
We prefer R³ to represent methyl, ethyl, n-propyl, isopropyl, cyclopropyl or CH₂OH (when Z² represents C), particularly methyl, ethyl or cyclopropyl, especially ethyl.
We prefer R⁴ and R⁵ independently to represent hydrogen or aryl or NR⁴R⁵ together to represent pyrrolidinyl, piperidinyl, morpholinyl, azetidinyl, azepinyl, piperazinyl or N-methylpiperazinyl.
We prefer that p represents 0. We prefer that R⁶ represents OH or NH₂.
We prefer that a represents 2 and that b represents 1 or 2. We prefer X to represent NR⁷ (e.g. NH), O, S or SO₂, particularly O, S or NH.
We prefer that c represents 0, and either that d represents 1 and e represents 1 or d represents 0 and e represents 2. We prefer that R⁷ represents hydrogen.

We particularly prefer R¹ to represent Ph₂CHCH₂-, hydrogen or CH(Et)₂, especially Ph₂CHCH₂-.
We particularly prefer R² to represent ethyl-piperidin-1-yl, PhCH₂CH(CH₂OH)-, -CH(CH₂OH)(CH(CH₃)₂, trans-4-amino-cyclohexyl, 2-(1-methyl-1H-imidazoyl-4-yl)CH2CH2-, ethyl-morpholin-1-yl, pyrrolidin-3-yl, ethyl-pyridin-2-yl, H₂NC(=NH)NH(CH₂)₂-, cyclopentyl or ethylcyclohexyl.

We prefer Z² to represent C. We prefer Z⁴ to represent N.

We prefer that the moiety 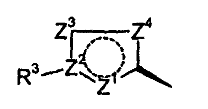 represents one of the following groups:

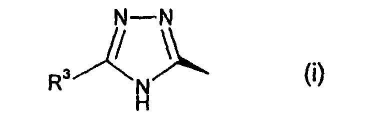

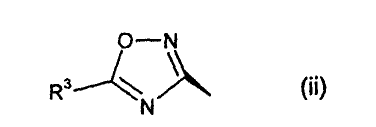

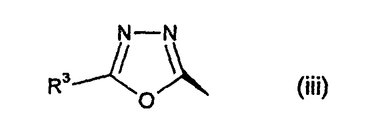

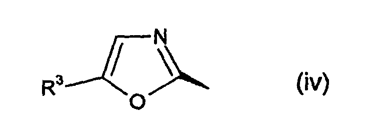

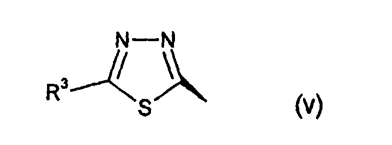

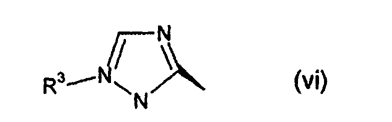

The above groups may be referred to hereinafter as (i) = triazolyl; (ii) = 4'-1,2,4 oxadiazolyl; (iii) = 4'-1,3,4 oxadiazolyl; (iv) = 1,3 oxazolyl; (v) = 1,3,4 thiadiazolyl; and (vi) = N-alkyl triazolyl. The groups above illustrated as (i) triazolyl, (ii) 4'-1,2,4 oxadiazolyl, (iii) 4'-1,3,4 oxadiazolyl and (vi) N-alkyl triazolyl are preferred. The group above illustrated as (i) triazolyl is most preferred.

The representation of formula (I) indicates the absolute stereochemistry. When sidechains contain chiral centres the invention extends to mixtures of enantiomers (including racemic mixtures) and diastereoisomers as well as individual enantiomers. Generally it is preferred to use a compound of formula (I) in the form of a purified single enantiomer.

We also provide a first process for the preparation of compounds of formula (I) including the step of reacting a compound of formula (II) 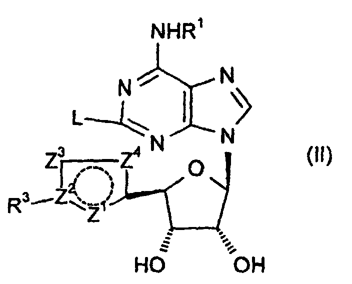 wherein L represents a leaving group, e.g. halogen, particularly chlorine; or a protected derivative thereof with a compound of formula R²NH₂ or a protected derivative thereof. Said reaction will generally involve heating the reagents to a temperature of 50°C-150°C in the presence of an inert solvent such as DMSO. The compound of formula (II) may be used in a form which the two hydroxyl groups are protected e.g. with acetonide or acetyl groups. Compounds of formula R²NH₂ are either known or may be prepared by conventional methods known *per se.*

This first process is particularly suitable for making the 1,3 oxazolyl and N-substituted triazolyl compounds herein.

Compounds of formula (II) or a protected derivative thereof may be prepared by reacting a compound of formula (III) 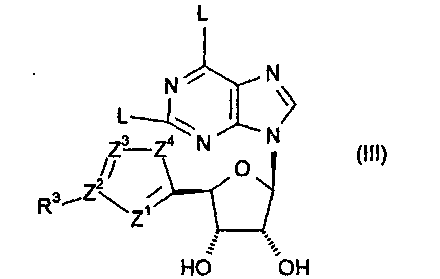 or a protected derivative thereof with a compound of formula R¹NH₂. This reaction will preferably be performed in the presence of a base such as an amine base (e.g. diisopropyl ethylamine in a solvent such as an alcohol (e.g. isopropanol) at elevated temperature (e.g. 50°C).

The compound of formula (III) or a protective derivative thereof may be prepared by reacting a compound of formula (IV) 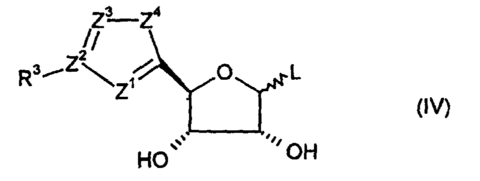 wherein L represents a leaving group, or a protected derivative thereof with 2,6,dichloropurine.

We prefer to use the compound of formula (IV) when the ribose 2- and 3-hydroxyl groups are protected for example by acetyl. Leaving group L may represent OH but will preferably represent C₁₋₆alkoxy (e.g. methoxy or ethoxy) an ester moiety (e.g. acetyloxy or benzoyloxy) or halogen. The preferred group L is acetyloxy. The reaction may be formed by combining the reactants in an inert solvent such as MeCN in the presence of a Lewis Acid (e.g. TMSOTf) and DBU.

The compounds of formula (IV) or a protected derivatives thereof may be prepared from a compound of formula (V) 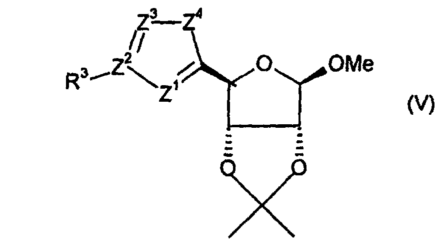 by treating the compound of formula (V) with trifluoroacetic acid in water followed by acetic anhydride in a solvent such as pyridine, DMAP, Et₃N, DCM or a combination of these.

Compounds of formula (IV) in which L represents halogen may be prepared for the corresponding 1'-alcohol or a 1'-ester such as the acetate. Reaction will generally occur on treatment with anhydrous HCl or HBr. 1'-lodides may be prepared directly on treatment with trimethylsilyliodide and 1'-fluorides may be prepared on treatment with DAST. An inert solvent, e.g. diethylether, DCM, THF or CCl₄ will generally be suitable.

Compounds of formula (V) may be prepared from D-ribose using methods analogous to those described at Scheme 1 of PCT Application No. PCT/EP97/07197, wherein the heterocyclic ring formation is achieved by conventional methods known *per se*.

We also provide a second process for the preparation of compounds of formula (II) including the step of reacting a compound of formula (VI) 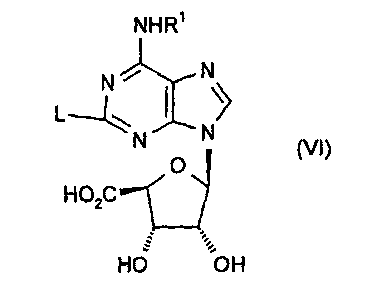 with reagents to enable formation of the appropriate heterocyclic ring (comprising Z¹, Z², Z³, and Z⁴) using conventional heterocyclic ring formation methods known *per se.* The compound of formula (VI) wherein R¹ represents Ph₂CHCH₂ and L represents chlorine is known and described at preparation 4. of PCT Patent Application No. WO94/17090. Other compounds of formula (VI) may be prepared by analogous or conventional methods.

This second process is suitable for making the triazolyl, 4'-1,2,4 oxadiazolyl, 4'-1,3,4 oxadiazolyl and 1,3,4 thiadiazolyl compounds herein.

Preferred routes to compounds of formula (I) wherein the 5-membered heterocyclic aromatic ring is one of the groups (i) -(vi) as described above, are now provided. The R¹ and R³ sidechains shown in the schemes are illustrative.

### (i) triazolyl

The following reaction scheme is provided:

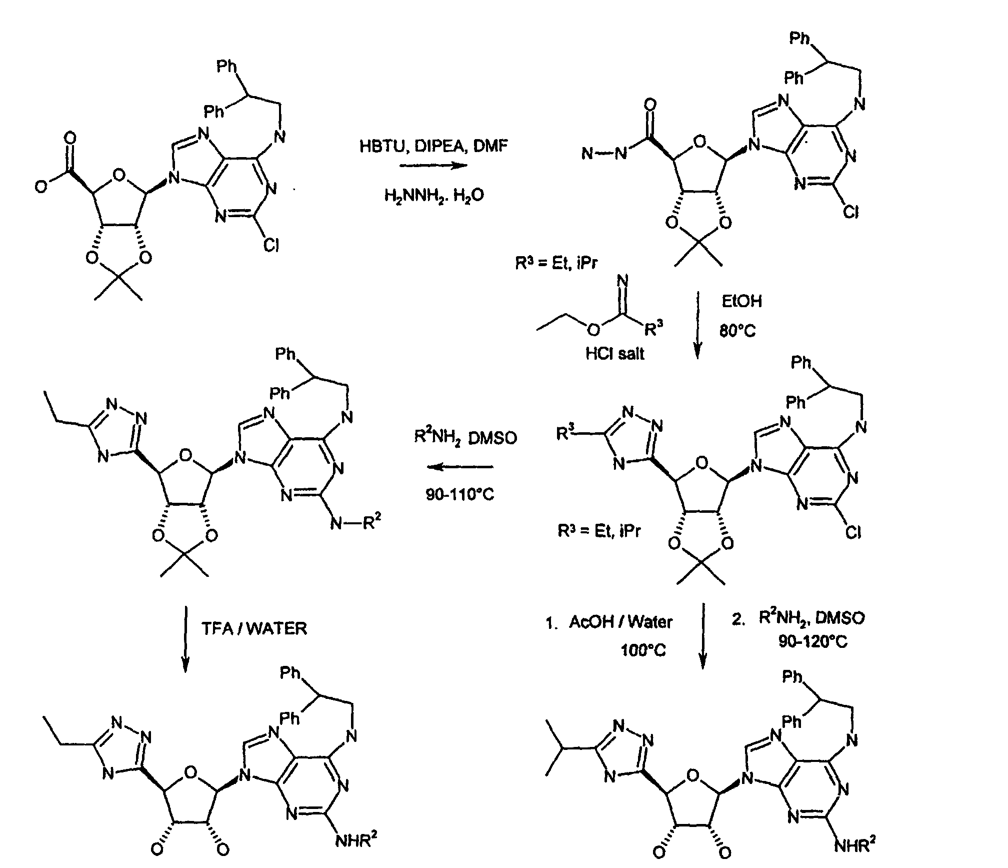

The above reaction scheme is particularly suitable where

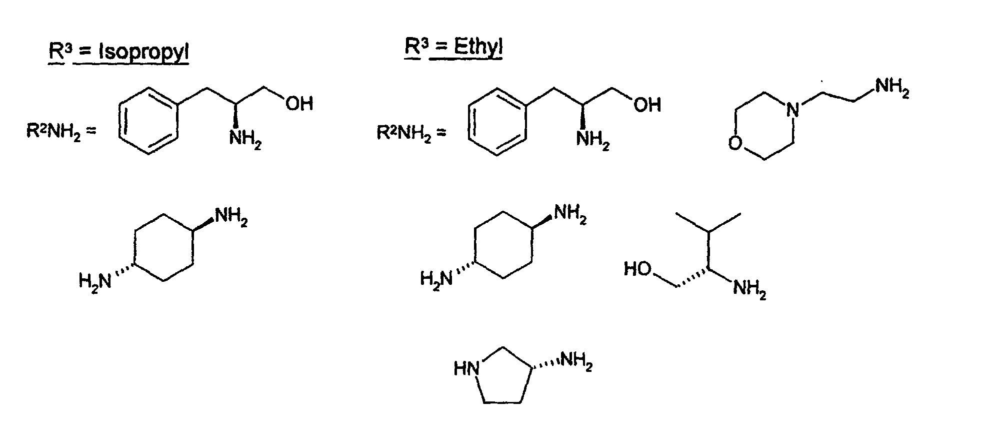

### (ii) 4'-1,2,4 oxadiazolyl

The following reaction scheme is provided:

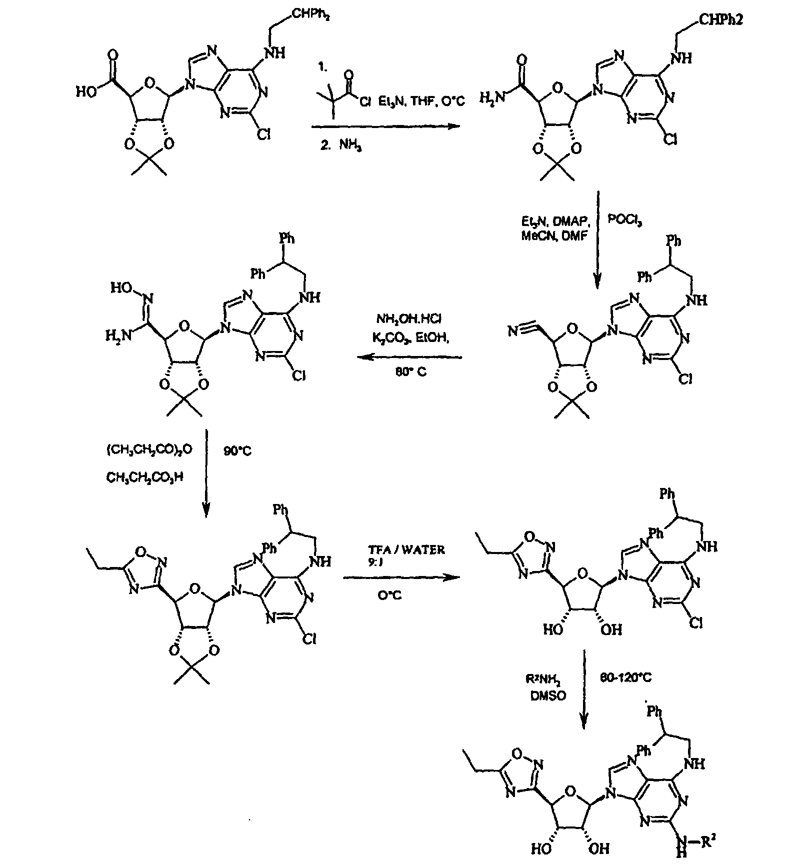

The above reaction is particularly suitable where

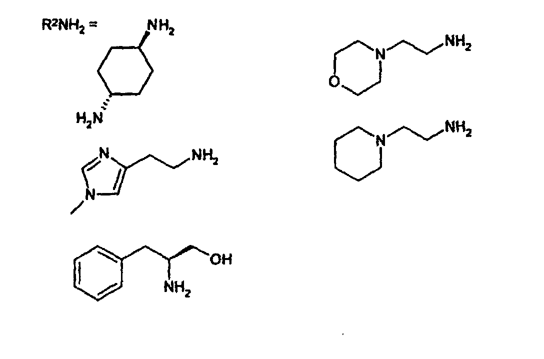

### (iii) 4'-1,3,4 oxadiazolyl

The following reaction scheme is provided:

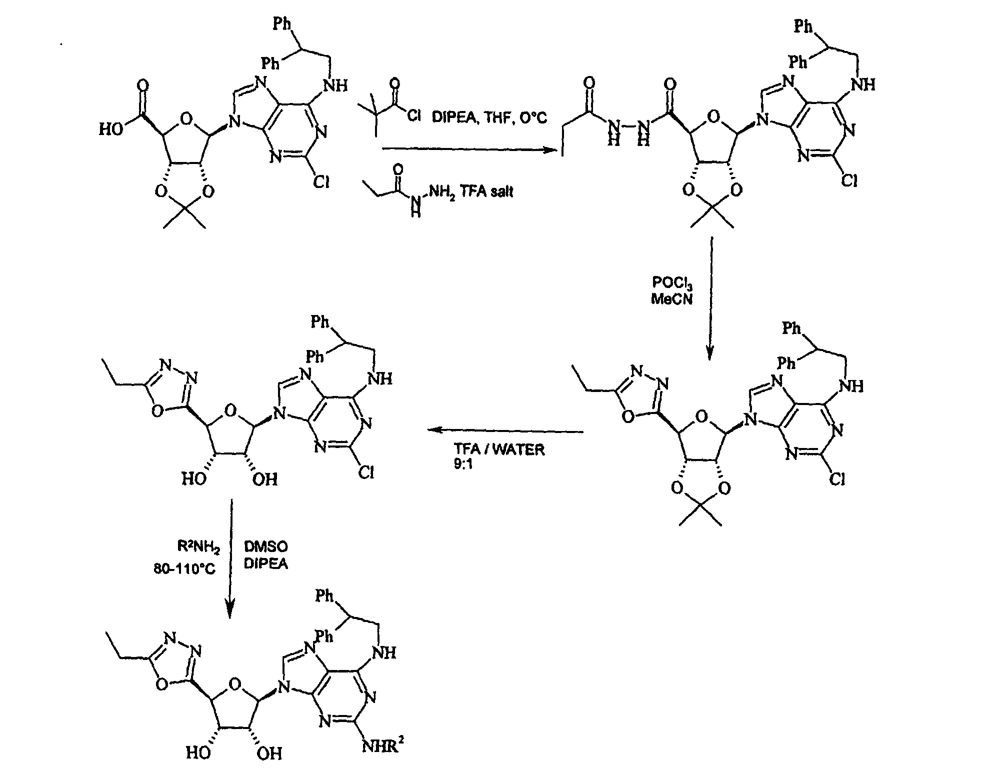

The above scheme is particularly suitable where

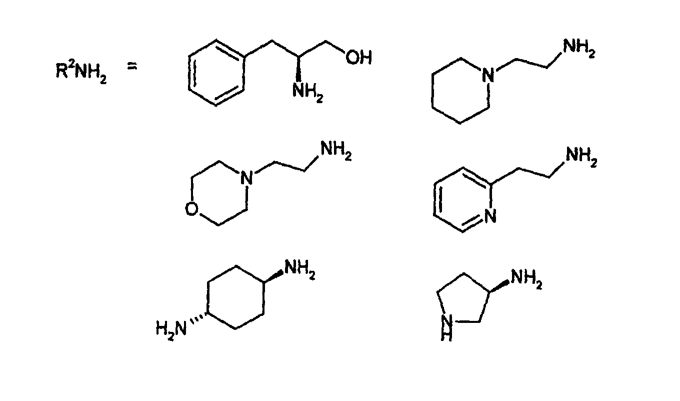

### (iv) 1,3 oxazolyl

The following reaction scheme is provided:

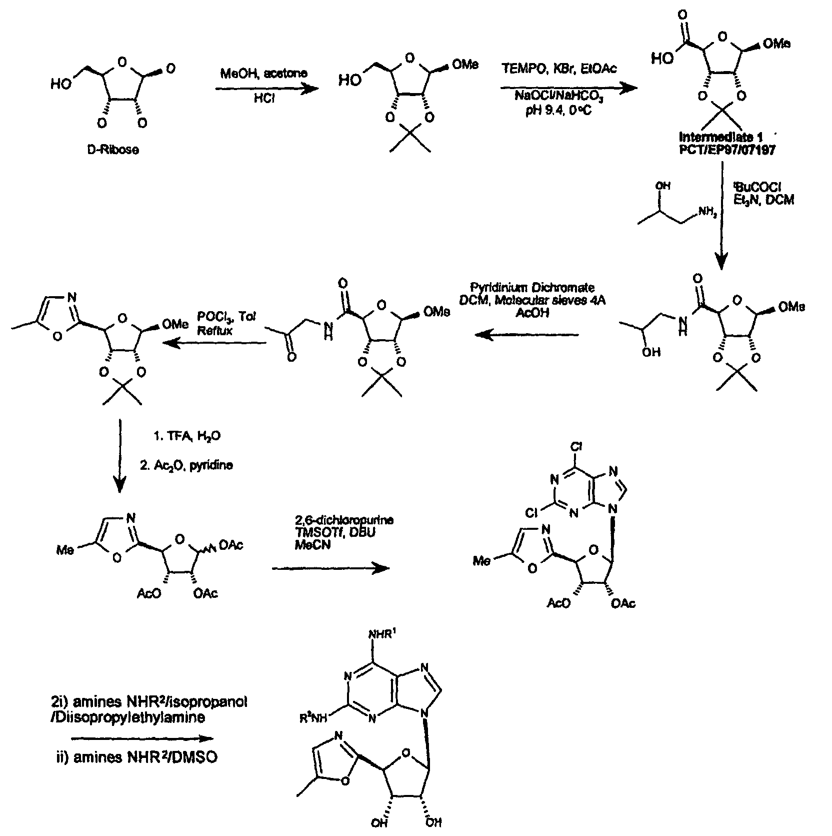

The above is particularly suitable for making:

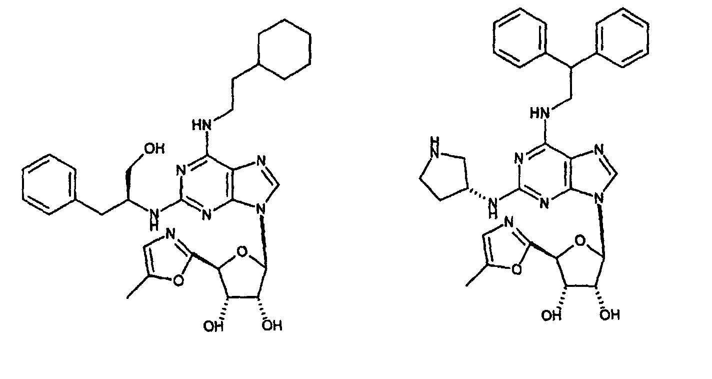

### (v) 1,3,4 thiadiazolyl

The following reaction scheme is provided:

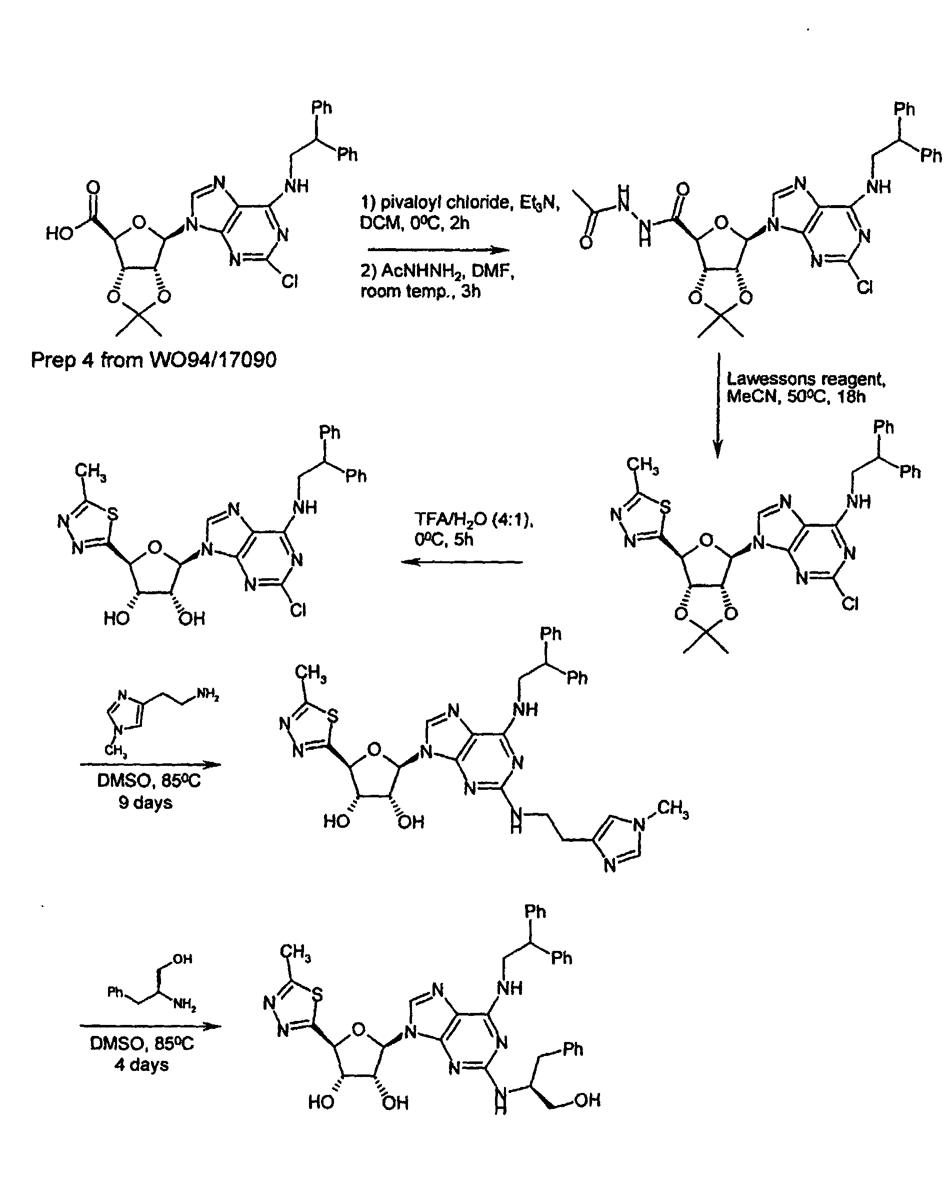

### (vi) N-substituted triazolyl

The following reaction scheme is provided:

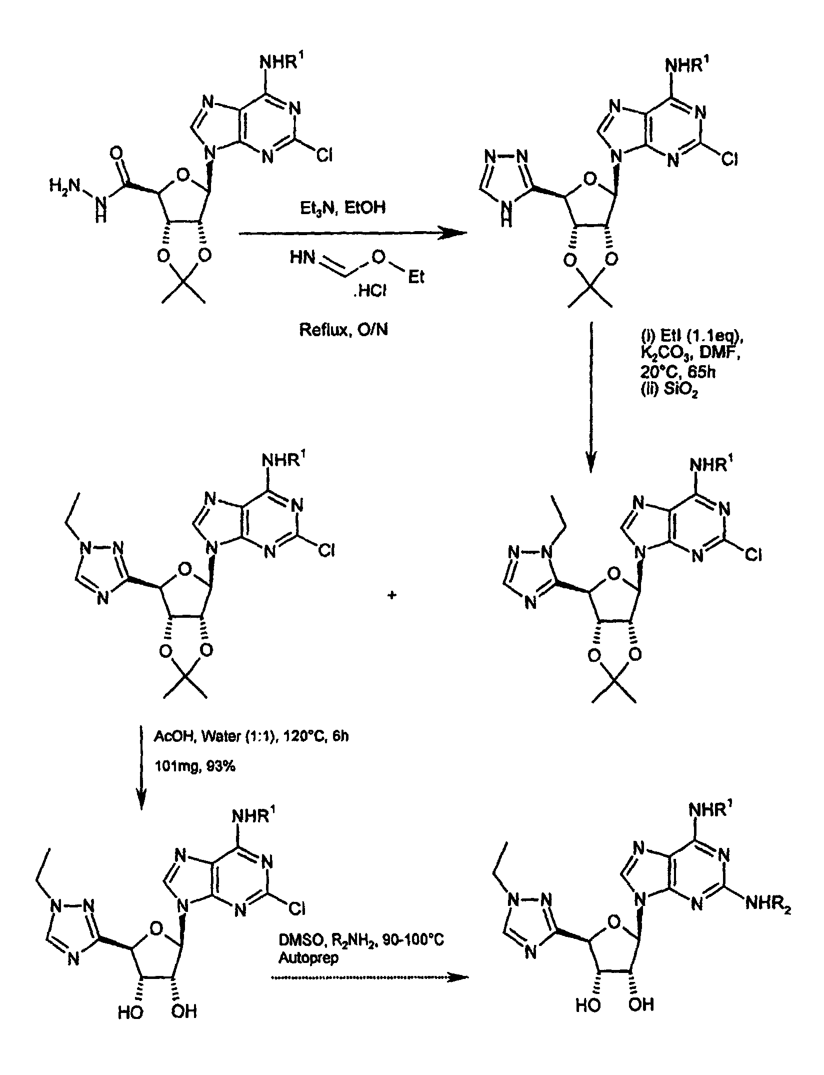

We also provide a further exemplary scheme for the preparation of compounds wherein the moiety: 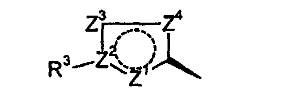 represents a substituted reverse isoxazole moiety: 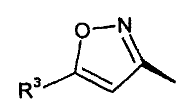 as follows:

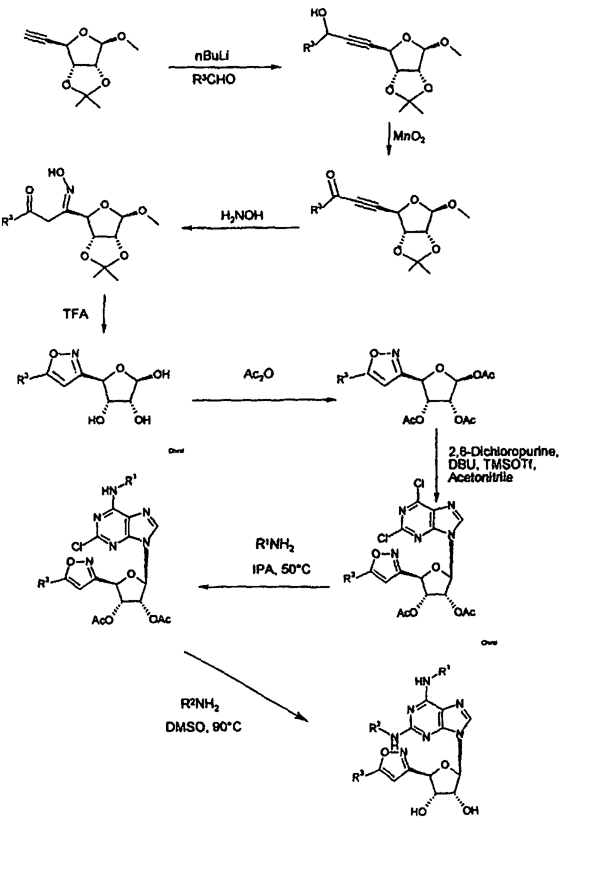

Examples of protecting groups and the means for their removal can be found in T W Greene "Protective Groups in Organic Synthesis" (J Wiley and Sons, 1991). Suitable hydroxyl protecting groups include alkyl (e.g. methyl), acetal (e.g. acetonide) and acyl (e.g. acetyl or benzoyl) which may be removed by hydrolysis, and arylalkyl (e.g. benzyl) which may be removed by catalytic hydrogenolysis. Suitable amine protecting groups include sulphonyl (e.g. tosyl), acyl e.g. benzyloxycarbonyl or t-butoxycarbonyl) and arylalkyl (e.g. benzyl) which may be removed by hydrolysis or hydrogenolysis as appropriate.

Suitable salts of the compounds of formula (I) include physiologically acceptable salts such as acid addition salts derived from inorganic or organic acids, for example hydrochlorides, hydrobromides, sulphates, phosphates, acetates, benzoates, citrates, succinates, lactates, tartrates, fumarates, maleates, 1-hydroxy-2-naphthoates, methanesulphonates, and if appropriate, inorganic base salts such as alkali metal salts, for example sodium salts. Other salts of the compounds of formula (I) include salts which are not physiologically acceptable but may be useful in the preparation of compounds of formula (I) and physiologically acceptable salts thereof. Examples of such salts include trifluoroacetates and formates.

Examples of suitable solvates of the compounds of formula (I) include hydrates. Acid-addition salts of compounds of formula (I) may be obtained by treating a free-base of formula (I) with an appropriate acid.

The potential for compounds of formula (I) to inhibit leukocyte function may be demonstrated, for example, by their ability to inhibit superoxide (O₂⁻) generation from neutrophils stimulated with chemoattractants such as N-formylmethionyl-leucyl-phenylalanine (fMLP). Accordingly, compounds of formula (I) are of potential therapeutic benefit in providing protection from leukocyte-induced tissue damage in diseases where leukocytes are implicated at the site of inflammation.

Examples of disease states in which the compounds of the invention have potentially beneficial anti-inflammatory effects include diseases of the respiratory tract such as adult respiratory distress syndrome (ARDS), bronchitis (including chronic bronchitis), cystic fibrosis, asthma (including allergen-induced asthmatic reactions), emphysema, rhinitis and septic shock. Other relevant disease states include diseases of the gastrointestinal tract such as intestinal inflammatory diseases including inflammatory bowel disease (e.g. Crohn's disease or ulcerative colitis), Helicobacter-pylori induced gastritis and intestinal inflammatory diseases secondary to radiation exposure or allergen exposure, and non-steroidal anti-inflammatory drug-induced gastropathy. Furthermore, compounds of the invention may be used to treat skin diseases such as psoriasis, allergic dermatitis and hypersensitivity reactions and diseases of the central nervous system which have an inflammatory component e.g. Alzheimer's disease and multiple sclerosis.

Further examples of disease states in which compounds of the invention have potentially beneficial effects include cardiac conditions such as peripheral vascular disease, post-ischaemic reperfusion injury and idiopathic hypereosinophilic syndrome.

Compounds of the invention which inhibit lymphocyte function may be useful as immunosuppressive agents and so have use in the treatment of auto-immune diseases such as rheumatoid arthritis and diabetes.

Compounds of the invention may also be useful in inhibiting metastasis.

It will be appreciated by those skilled in the art that reference herein to treatment extends to prophylaxis as well as the treatment of established conditions.

As mentioned above, compounds of formula (I) are useful in human or veterinary medicine, in particular as anti-inflammatory agents.

There is thus provided as a further aspect of the invention a compound of formula (I) or a physiologically acceptable salt or solvate thereof for use in human or veterinary medicine, particularly in the treatment of patients with inflammatory conditions who are susceptible to leukocyte-induced tissue damage.

According to another aspect of the invention, there is provided the use of a compound of formula (I) or a physiologically acceptable salt or solvate thereof for the manufacture of a medicament for the treatment of patients with inflammatory conditions who are susceptible to leukocyte-induced tissue damage.

In a further or alternative aspect there is provided a method for the treatment of a human or animal subject with an inflammatory condition who is susceptible to leukocyte-induced tissue damage, which method comprises administering to said human or animal subject an effective amount of a compound of formula (I) or a physiologically acceptable salt or solvate thereof.

The compounds according to the invention may be formulated for administration in any convenient way, and the invention therefore also includes within its scope pharmaceutical compositions for use in anti-inflammatory therapy, comprising a compound of formula (I) or a physiologically acceptable salt or solvate thereof together, if desirable, with one or more physiologically acceptable carriers or excipients.

There is also provided a process for preparing such a pharmaceutical formulation which comprises mixing the ingredients.

The compounds according to the invention may, for example, be formulated for oral, buccal, parenteral, topical or rectal administration, preferably for parenteral or topical (e.g. by aerosol) administration.

Tablets and capsules for oral administration may contain conventional excipients such as binding agents, for example syrup, acacia, gelatin, sorbitol, tragacanth, mucilage of starch, cellulose or polyvinyl pyrrolidone; fillers, for example, lactose, microcrystalline cellulose, sugar, maize- starch, calcium phosphate or sorbitol; lubricants, for example, magnesium stearate, stearic acid, talc, polyethylene glycol or silica; disintegrants, for example, potato starch, croscarmellose sodium or sodium starch glycollate; or wetting agents such as sodium lauryl sulphate. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, for example, sorbitol syrup, methyl cellulose, glucose/sugar syrup, gelatin, hydroxymethyl cellulose, carboxymethyl cellulose, aluminium stearate gel or hydrogenated edible fats; emulsifying agents, for example, lecithin, sorbitan mono-oleate or acacia; non-aqueous vehicles (which may include edible oils), for example almond oil, fractionated coconut oil, oily esters, propylene glycol or ethyl alcohol; or preservatives, for example, methyl or propyl p- hydroxybenzoates or sorbic acid. The preparations may also contain buffer salts, flavouring, colouring and/or sweetening agents (e.g. mannitol) as appropriate.

For buccal administration the compositions may take the form of tablets or lozenges formulated in conventional manner.

The compounds may also be formulated as suppositories, e.g. containing conventional suppository bases such as cocoa butter or other glycerides.

The compounds according to the invention may also be formulated for parenteral administration by bolus injection or continuous infusion and may be presented in unit dose form, for instance as ampoules, vials, small volume infusions or pre-filled syringes, or in multi-dose containers with an added preservative. The compositions may take such forms as solutions, suspensions, or emulsions in aqueous or non-aqueous vehicles, and may contain formulatory agents such as anti-oxidants, buffers, antimicrobial agents and/or tonicity adjusting agents. Alternatively, the active ingredient may be in powder form for constitution with a suitable vehicle, e.g. sterile, pyrogen-free water, before use. The dry solid presentation may be prepared by filling a sterile powder aseptically into individual sterile containers or by filling a sterile solution aseptically into each container and freeze-drying.

By topical administration as used herein, we include administration by insufflation and inhalation. Examples of various types of preparation for topical administration include ointments, creams, lotions, powders, pessaries, sprays, aerosols, capsules or cartridges for use in an inhaler or insufflator, solutions for nebulisation or drops (e.g. eye or nose drops).

Ointments and creams may, for example, be formulated with an aqueous or oily base with the addition of suitable thickening and/or gelling agents and/or solvents. Such bases may thus, for example, include water and/or an oil such as liquid paraffin or a vegetable oil such as arachis oil or castor oil or a solvent such as a polyethylene glycol. Thickening agents which may be used include soft paraffin, aluminium stearate, cetostearyl alcohol, polyethylene glycols, microcrystalline wax and beeswax.

Lotions may be formulated with an aqueous or oily base and will in general also contain one or more emulsifying agents, stabilising agents, dispersing agents, suspending agents or thickening agents.

Powders for external application may be formed with the aid of any suitable powder base, for example, talc, lactose or starch. Drops may be formulated with an aqueous or non-aqueous base also comprising one or more dispersing agents, solubilising agents or suspending agents.

Spray compositions may be formulated, for example, as aqueous solutions or suspensions or as aerosols delivered from pressurised packs, with the use of a suitable propellant, e.g. dichlorodifluoromethane, trichlorofluoromethane, dichlorotetra-fluoroethane, 1,1,1,2,3,3,3-heptafluoropropane, 1,1,1,2-tetrafluoroethane, carbon dioxide or other suitable gas.

Intranasal sprays may be formulated with aqueous or non-aqueous vehicles with the addition of agents such as thickening agents, buffer salts or acid or alkali to adjust the pH, isotonicity adjusting agents or anti-oxidants.

Capsules and cartridges of for example gelatin, or blisters of for example laminated aluminium foil, for use in an inhaler or insufflator may be formulated containing a powder mix of a compound of the invention and a suitable powder base such as lactose or starch.

Solutions for inhalation by nebulation may be formulated with an aqueous vehicle with the addition of agents such as acid or alkali, buffer salts, isotonicity adjusting agents or antimicrobials. They may be sterilised by filtration or heating in an autoclave, or presented as a non-sterile product.

The pharmaceutical compositions according to the invention may also be used in combination with other therapeutic agents, for example anti-inflammatory agents (such as corticosteroids (e.g. fluticasone propionate, beclomethasone dipropionate, mometasone furoate, triamcinolone acetonide or budesonide) or NSAIDs (eg sodium cromoglycate)) or beta adrenergic agents (such as salmeterol, salbutamol, formoterol, fenoterol or terbutaline and salts thereof) or antiinfective agents (eg antibiotics, antivirals).

The invention thus provides, in a further aspect, a combination comprising a compound of formula (I) or a physiologically acceptable salt or solvate thereof together with another therapeutically active agent, for example an anti-inflammatory agent such as a corticosteroid or NSAID.

The combination referred to above may conveniently be presented for use in the form of a pharmaceutical formulation and thus pharmaceutical formulations comprising a combination as defined above together with a pharmaceutically acceptable carrier thereof represent a further aspect of the invention.

The individual components of such combinations may be administered either sequentially or simultaneously in separate or combined pharmaceutical formulations. Appropriate doses of known therapeutic agents will be readily appreciated by those skilled in the art.

Compounds of the invention may conveniently be administered in amounts of, for example, 0.01 to 500mg/kg body weight, preferably 0.01 to 100mg/kg body weight, 1 to 4 times daily. The precise dose will of course depend on the age and condition of the patient and the particular route of administration chosen.

Certain intermediate compounds described herein are new and these are also provided as an aspect of the invention.

The compounds of the invention have the advantage that they may be more efficacious, show greater selectivity, have fewer side effects, have a longer duration of action, be more bioavailable by the preferred route, show less systemic activity when administered by inhalation or have other more desirable properties than similar known compounds.

In particular the compounds of the invention have the advantage that they may show greater selectivity for the adenosine 2a receptor subtype over other adenosine receptor subtypes (especially the A1 and A3 receptor subtypes) than hitherto known compounds.

Compounds of the invention were tested for in vitro and in vivo biological activity in accordance with the following screens:
(1) Agonist activity against adenosine 2a, adenosine 1 and adenosine 3 receptor subtypes.

Agonist selectivity of compounds against other human adenosine receptors was determined using Chinese hamster ovary (CHO) cells transfected with the gene for the relevant human adenosine receptor following a method based on that of Castanon and Spevak, 1994 . The CHO cells were also transfected with cyclic AMP response elements promoting the gene for secreted placental alkaline phosphatase (SPAP) (Wood, 1995). The effect of test compounds was determined by their effects on basal levels of cAMP (A2a) or on forskolin-enhanced cAMP (A1 and A3) as reflected by changes in levels of SPAP. EC₅₀ values for compounds were then determined as a ratio to that of the non-selective agonist N-ethyl carboxamide adenosine (NECA).

### References:

Asako H, Wolf, RE, Granger, DN (1993), Gastroenterology 104, pp 31-37;
Bedford CD, Howd RA, Dailey OD, Miller A, Nolen HW III, Kenley RA, Kern JR, Winterle JS, (1986), J. Med. Chem. 29, pp2174-2183;
Burkey TH, Webster, RO, (1993), Biochem. Biophys Acta 1175, pp 312-318;
Castanon MJ, Spevak W, (1994), Biochem. Biophys Res. Commun. 198, pp 626-631;
Cronstein BN, Kramer SB, Weissmann G, Hirschhom R, (1983), Trans. Assoc. Am. Physicians 96, pp 384-91;
Cronstein BN, Kramer SB, Rosenstein ED, Weissmann G, Hirschhorn R, (1985), Ann N.Y. Acad. Sci. 451, pp 291-301;
Cronstein BN, Naime D, Ostad E, (1993), J. Clin. Invest. 92, pp 2675-82;
Cronstein BN, Naime D, Ostad E, (1994), Adv. Exp. Med. Biol., 370, pp 411-6;
Cronstein BN, (1994), J. Appl. Physiol. 76, pp 5-13;
Dianzani C, Brunelleschi S, Viano I, Fantozzi R, (1994), Eur. J. Pharmacol 263, pp 223-226;
Elliot KRF, Leonard EJ, (1989), FEBS Letters 254, pp 94-98;
Flora KP, van't Riet B, Wampler GL, (1978), Cancer Research, 38, pp1291-1295;
Green PG, Basbaum Al, Helms C, Levine JD, (1991), Proc. Natl. Acad Sci. 88, pp 4162-4165;
Hirschom R, (1993), Pediatr. Res 33, pp S35-41;
Kohno Y; Xiao-duo J; Mawhorter SD; Koshiba M; Jacobson KA. (1996).Blood 88 p3569-3574.
Peachell PT, Lichtenstein LM, Schleimer RP, (1989), Biochem Pharmacol 38, pp 1717-1725;
Richter J, (1992), J. Leukocyte Biol. 51, pp 270-275;
Rosengren S, Bong GW, Firestein GS, (1995), J. Immunol. 154, pp 5444-5451;
Sanjar S, McCabe PJ, Fattah D, Humbles AA, Pole SM, (1992), Am. Rev. Respir. Dis. 145, A40;
Skubitz KM, Wickman NW, Hammerschmidt DE, (1988), Blood 72, pp 29-33 Van Schaick EA; Jacobson KA; Kim HO; Ijzerman AP; Danhof M. (1996) Eur J Pharmacol 308 p311-314.
Wood KV. (1995) Curr Opinion Biotechnology 6 p50-58.

Throughout the specification and the claims which follow, unless the context requires otherwise, the word 'comprise', and variations such as 'comprises' and 'comprising', will be understood to imply the inclusion of a stated integer or step or group of integers but not to the exclusion of any other integer or step or group of integers or steps.

### The invention is illustrated by the following Examples:

### Examples

### General experimental details

Where products were purified by column chromatography, 'flash silica' refers to silica gel for chromatography, 0.040 to 0.063mm mesh (e.g. Merck Art 9385), where column elution was accelerated by an applied pressure of nitrogen at up to 5 p.s.i. Where thin layer chromatography (TLC) has been used it refers to silica gel TLC using 5 x 10 cm silica gel 60 F₂₅₄ plates (e.g. Merck Art 5719), visualised using UV light unless otherwise indicated. Where products were purified by preparative HPLC, this was carried out on a C18-reverse-phase column (1" Dynamax), eluting with a gradient of acetonitrile (containing 0.1% trifluoroacetic acid) in water (containing 0.1% trifluoroacetic acid) and the compounds isolated as their trifluoroacetate salts unless otherwise specified.

### Standard Automated Preparative HPLC column, conditions & eluent

Automated preparative high performance liquid chromatography (autoprep. HPLC) was carried out using a Supelco ABZ+ 5µm 100mmx22mm i.d. column eluted with a mixture of solvents consisting of i) 0.1% formic acid in water and ii) 0.05% formic acid in acetonitrile, the eluent being expressed as the percentage of ii) in the solvent mixture, at a flow rate of 4ml per minute. Unless otherwise stated the eluent was used as a gradient of 5-95 % over 20 minutes.

### LC/MS System

The Liquid Chromatography Mass Spectroscopy (LC/MS) systems used:
LC/MS System A - A Supelco ABZ+, 3.3cm x 4.6mm i.d. column eluting with solvents: A - 0.1%v/v formic acid + 0.077% w/v ammonium acetate in water, and B - 95:5 acetonitrile:water + 0.05% v/v formic acid. The following gradient protocol was used: 100% A for 0.7 mins; A+B mixtures, gradient profile 0 - 100% B over 3.5mins; hold at 100% B for 3.5mins; return to 0% B over 0.3mins. Positive and negative electrospray ionization was employed.
LC/MS System B - A Supelco ABZ+, 5cm x 2.1mm i.d. column eluting with solvents: A - 0.1%v/v formic acid + 0.077% w/v ammonium acetate in water, and B - 95:5 acetonitrile:water + 0.05% v/v formic acid. The following gradient protocol was used: 0 - 100% B over 3.5mins; hold at 100% B for 1.50mins; return to 0% B over 0.50mins. Positive and negative electrospray ionization was employed.
LC/MS System C - A Supelco ABZ+, 3.3cm x 4.6mm i.d. column eluting with solvents: A - 0.1%v/v formic acid + 10mmol ammonium acetate in water, and B - 95:5 acetonitrile:water + 0.05% v/v formic acid. The following gradient protocol was used: 100% A for 0.7 mins; A+B mixtures, gradient profile 0- 100% B over 3.7mins; hold at 100% B for 0.9mins; return to 0% B over 0.2mins. Positive and negative electrospray ionization was employed.

### Intermediate 1: (3aS,4S,6R,6aR)-6-Methoxy-2,2-dimethyl-tetrahydrofuro[3,4-d][1,3]dioxole-4-carboxylic acid (2-hydroxy-propyl)-amide.

Trimethylacetyl chloride (4.9mL, 39.8mmol) was added dropwise to a cooled solution in an ice/water bath of [Intermediate 1 in PCT Application No. PCT/EP97/07197] (8.69g, 39.8mmol) and triethylamine (6.1ml, 43.8mmol) in dichloromethane (120ml) under nitrogen with stirring. After 45 mins. isopropanolamine (3.7ml, 77.8mmol), and left to warm to 20°C and stirred for 20hrs.. Saturated sodium bicarbonate solution (100ml) was added and the aqueous mixture was extracted with further dichloromethane (3 x 100ml). The combined organic phases were washed with brine (60ml), dried (MgSO₄) and solvent was removed *in vacuo* leaving the title compound as a pale yellow gum (11.8g). TLC SiO₂ (neat ethyl acetate) Rf = 0.30

### Intermediate 2: (3aS,4S,6R,6aR)-6-Methoxy-2,2-dimethyl-tetrahydro-furo[3,4-d][1,3]dioxole-4-carboxylic acid (2-oxo-propyl)-amide.

To a cooled, 0°C (ice/water bath), mixture of Intermediate 1 (1.68g, 6.1mmol), acetic acid (1.2ml) and 4A molecular sieves (2.52g) in anhydrous dichloromethane (45ml) was added portionwise pyridinium dichromate (3.68g, 9.8mmol) with stirring. After 15 mins. The ice bath was removed and the reaction mixture was stirred at 20°C for 2hrs. Further pyridinium dichromate (0.46g, 1.2mmol) was added and the reaction mixture stirred for 30 mins., whereupon isopropanol (15ml) was added and the reaction mixture stirred for 15 mins before filtering through a pad of filter aid Habourlite J2 and concentrating *in vacuo*. The product was purified by column chromatography on flash silica eluting with cyclohexane, ethyl acetate mixtures (2:1 and 1:1) furnishing the title compound as a colourless oil (1.213g) TLC SiO₂ (neat ethyl acetate) Rf = 0.36

### Intermediate 3: 2-(6R-Methoxy-2,2-dimethyl-tetrahydro-(3aR,6aR)-furo[3,4-d][1,3]dioxol-4S-yl)-5-methyl-oxazole

Intermediate 2 (1.213g, 4.4mmol) was dissolved in dry toluene (15ml) under nitrogen and to this was added POCl₃ (2.48ml, 26.6mmol). The reaction mixture was warmed to reflux for 2.5hrs., then allowed to cool for 2hrs, and further cooled in an ice bath whereupon a cautious addition of aqueous saturated sodium bicarbonate solution (50ml) was made. The resultant mixture was stirred vigorously for 1 hr, phases separated and the aqueous phase further extracted with ethyl acetate (3 x 50ml). The combined organic phases were dried (MgSO₄) and solvent was removed *in vacuo*. The crude product was purified using column chromatography on flash silica eluting with cyclohexane, ethyl acetate (3:1) to furnish the title compound as a pale yellow oil (0.616g) TLC SiO₂ (cyclohexane, ethyl acetate (1:1)) Rf = 0.40

### Intermediate 4: Acetic acid 4R,5-diacetoxy-2S-(5-methyl-oxazol-2-yl)-tetrahydro-furan-3R-yl ester

Intermediate 3 (6.307g, 24.7mmol) was treated with trifluoroacetic acid (32.4ml) and water (3.6ml), left to stand at 20° C for 3 hrs. and then solvent was removed *in vacuo*. The residue was dissolved in pyridine (40ml) under nitrogen and acetic anhydride (28ml) was added and the reaction mixture was left to stir for 16 hrs. before concentration *in vacuo*. The resultant oil was dissolved in ethyl acetate (20ml), washed with aqueous hydrochloric acid (1M, 20ml), saturated aqueous sodium bicarbonate (3 x 20ml), brine (20ml), dried (MgSO₄) and solvent was removed in vacuo. The crude product was purified using column chromatography on flash silica eluting with cyclohexane, ethyl acetate (1:1) to furnish the title compound as a pale yellow oil (7.640g) TLC SiO₂ (cyclohexane, ethyl acetate (1:1)) Rf = 0.31

### Intermediate 5: Acetic acid 4S-acetoxy-2R-(2,6-dichloro-purin-9-yl)-5S-(5-methyl-oxazol-2-yl)-tetrahydro-furan-3R-yl ester

Intermediate 4 (2.25g, 6.9mmol) was dissolved in anhydrous acetonitrile (35ml) at 20 C under nitrogen and 2,6dichloropurine (1.83g, 9.7mmol), DBU (1.24ml, 8.3mmol) and TMSOTf (1.73ml 8.9mmol) were added successively and stirred at 20°C for 16.5 hrs. Further DBU (0.62, 4.2mmol) and TMSOTf (0.87ml 4.5mmol) were added and after 2 hrs. at 20°C the reaction mixture was heated at 90°C for 1.5hrs. The cooled mixture was diluted with ethyl acetate (50ml), washed with water (2 x 50ml). The combined aqueous phases were reextracted with ethyl acetate (2 x 50ml). The combined organic phases were dried (Na2SO4) and solvent removed *in vacuo.* The crude product was purified using column chromatography on flash silica eluting with ethyl acetate, cyclohexane (1:1) furnishing the title compound as a colourless foam (2.695g) TLC SiO₂ (cyclohexane, ethyl acetate (1:1)) Rf = 0.24

### Intermediate 6: (3aS,4S,6R,6aR)-6-[2-Chloro-6-(2,2-diphenyl-ethylamino)-purin-9-yl]-2,2-dimethyl-tetrahydro-furo[3,4-d][1,3]dioxole-4-carboxylic acid N'-acetyl hydrazide

(5.00g, 9.33mmol) was dissolved in anhydrous dichloromethane (100ml) and cooled to 0°C under nitrogen. Triethylamine (1.43ml, 10.26mmol) was then added followed by pivaloyl chloride (1.26ml, 10.26mmol) and the mixture stirred at 0°C for 2h. Acetyl hydrazine (1.10g, 14.85mmol) was then added and the mixture allowed to warm to 20°C over 3h with stirring. The reaction mixture was concentrated *in vacuo* and partitioned between ethyl acetate (150ml) and water (30ml). The organic layer was washed further with water (30ml), then dried over MgSO₄, filtered and concentrated *in vacuo* to afford a pale yellow solid. Recrystallisation from hot dichloromethane afforded the title compound as a white solid (5.17g). TLC SiO₂ (ethyl acetate) R_{f} =0.26

### Intermediate 7: {2-Chloro-9-[2,2-dimethyl-6S-(5-methyl-[1,3,4]thiadiazol-2-yl)-tetrahydro-(3aR,6aS)-furo[3,4-d][1,3]dioxol-4R-yl]-9H-purin-6-yl}-(2,2-diphenylethyl)-amine

A solution of Intermediate 6 (0.70g, 1.18mmol) in acetonitrile (15ml) was treated with a solution of Lawessons reagent (0.53g, 1.31mmol) also in acetonitrile (15ml) and stirred at 20°C for 18h. The mixture was heated at 50°C for 6h, followed by stirring for a further 66h at 20°C. Acetonitrile was removed by evaporation under reduced pressure and the residue purified by column chromatography on flash silica eluting first with toluene, then with 50% ethyl acetate in cyclohexane to afford the title compound as a white solid (0.43g). TLC SiO₂ (ethyl acetate) R_{f} =0.60

### Intermediate 8: (2R,3R,4S,5S)-2-[2-Chloro-6-(2,2-diphenyl-ethylamino)-purin-9-yl]-5-(5-methyl-[1,3,4]thiadiazol-2-yl)-tetrahydro-furan-3,4-diol

Intermediate 7 (0.42g, 0.71mmol) was dissolved in 80% TFA in water (15ml) at 0°C and stirred for 5h at this temperature. The mixture was concentrated *in vacuo* and the residue partitioned between ethyl acetate (40ml) and saturated aqueous sodium bicarbonate solution (5ml). The organic layer was further washed with brine (5ml), then water (5ml), dried (MgSO₄), filtered and evaporated under reduced pressure to afford the title compound as an off-white solid (0.34g). TLC SiO₂ (ethyl acetate) R_{f} =0.38

### Intermediate 9: (3aS,4S,6R,6aR)-6-[2-Chloro-6-(2,2-diphenyl-ethylamino)-purin-9-yl]-2,2-dimethyl-tetrahydro-furo[3,4-d][1,3]dioxole-4-carboxylic acid hydrazide

(3aS,4S,6R,6aR)-6-[2-Chloro-6-(2,2-diphenyl-ethylamino)-purin-9-yl]-2,2-dimethyl-tetrahydro-furo[3,4-d][1,3]dioxole-4-carboxylic acid [Preparation 4 from PCT Patent Application No. WO94/17090] (200mg, 0.4mmol) in dry dimethylformamide (2mL) was treated with HBTU (152mg, 0.4mmol) and diisopropylethylamine (129mg, 0.18mL, 1mmol) and the reaction stirred at room temperature under nitrogen for 15 minutes. Hydrazine hydrate (20mg, 0.019mmol) was added and the reaction stirred at room temperature for a further 20 hours. The reaction mixture was partitioned between ethyl acetate (100mL) and saturated ammonium chloride solution (100mL). The organic phase was washed with a further portion of saturated ammonium chloride solution, 2N citric acid (2 x 100mL), saturated sodium bicarbonate (2 x 100mL), dried (MgSO₄) and concentrated *in vacuo* to afford the title compound as pale coloured foam (0.158g). LC-MS System A Rt.= 4.73 min., *m*/*z* 550 (MH⁺)

### Intermediate 10: {2-Chloro-9-[2,2-dimethyl-6R-(5-methyl-4H-[1,2,4]triazol-3-yl)-tetrahydro-(3aR,6aR)-furo[3,4-d][1,3]dioxol-4R-yl]-9H-purin-6-yl}-(2,2-diphenylethyl)-amine

A solution of Intermediate 9 (780mg, 1.4mmol) in ethanol (25mL) was treated with ethylacetimidate hydrochloride (275mg, 2.1mmol) and triethylamine (1mL, 7mmol) and the reaction mixture was stirred at reflux for 16 hours prior to cooling. The reaction mixture was concentrated *in vacuo* and the residue partitioned between ethyl acetate (200mL) and 2N HCI (200mL). The organic phase was washed with brine (2 x 200mL), dried (MgSO₄) and concentrated in vacuo. Purification by column chromatography on flash silica eluted with ethyl acetate afforded the title compound as a white solid (0.410g).
LC-MS System A Rt.= 3.40 min., *m*/*z* 573 (MH⁺)

### Intermediate 11: {2-Chloro-9-[6R-(5-ethyl-4H-[1,2,4]triazol-3-yl)-2,2-dimethyltetrahydro-(3aR,6aR)-furo[3,4-d][1,3]dioxol-4R-yl]-9H-purin-6-yl}-(2,2-diphenylethyl)-amine

To a solution of Intermediate 9 (0.696g, 1.27mmol) in ethanol (25ml), triethylamine (0.89ml, 6.4mmol) and ethyl propionimidate hydrochloride (0.260g, 1.9mmol) were added. The reaction mixture was stirred at 80°C under nitrogen for 17h.. The solution was allowed to cool, then concentrated *in vacuo* and the residue partitioned between ethyl acetate (50ml), and hydrochloric acid solution (2N, 50ml). The organic phase was washed with brine (50ml), dried (MgSO₄), and concentrated *in vacuo.* Purification by column chromatography on flash silica eluted with DCM: methanol (25:1) afforded the title compound as a cream solid (0.290g). TLC SiO₂ (Dichloromethane, methanol, 25:1) Rf = 0.36

### Intermediate 12: {2-Chloro-9-[6R-(5-isopropyl-4H-[1,2,4]triazol-3-yl)-2,2-dimethyl-tetrahydro-(3aR,6aR)-furo[3,4-d][1,3]dioxol-4R-yl]-9H-purin-6-yl}-(2,2-diphenyl-ethyl)-amine

To a solution of Intermediate 9 (0.6g, 1.09mmol) in ethanol (25ml), triethylamine (0.77ml, 5.5mmol) and 2-methylpropionimidic acid ethyl ester hydrochloride salt (0.230g, 1.97mmol) were added. The solution was stirred at 80°C under nitrogen for 20h.. 2-Methylpropionimidic acid ethyl ester hydrochloride salt (0.063g, 0.546mmol) was added and the solution was heated for a further 3h.. The solution was allowed to cool, then concentrated *in vacuo* and the residue partitioned between ethyl acetate (50ml), and hydrochloric acid solution (2N, 50ml). The organic phase was washed with brine (50ml), dried (MgSO₄), and concentrated *in vacuo.* Purification by column chromatography on flash silica eluted with dichloromethane: methanol (40:1 to 25:1) afforded the title compound as an orange foam (0.410g). TLC SiO₂ (Dichloromethane, methanol, 25:1) Rf = 0.43

### Intermediate 13: (2R,3R,4S,5R)-2-[2-Chloro-6-(2,2-diphenyl-ethylamino)-purin-9-yl]-5-(5-isopropyl-4H-[1,2,4]triazol-3-yl)-tetrahydro-furan-3,4-diol

A solution of Intermediate 12 (0.410g, 0.683mmol) in glacial acetic acid / water solution (4:1, 25ml), was heated at 100°C under nitrogen for 4.5h.. The cooled solution was concentrated *in vacuo*, then partitioned between ethyl acetate (50ml) and saturated sodium bicarbonate solution (50ml). The aqueous was back extracted with ethyl acetate (50ml). The organics were dried (MgSO₄) and concentrated *in vacuo* to afford the title compound as a pale orange foam (0.278g). LC/MS SYSTEM B Rₜ = 3.21 min, *m*/*z* = 561 MH⁺

### Intermediate 14: (3aS,4S,6R,6aR)-6-[2-Chloro-6-(2,2-diphenyl-ethylamino)-purin-9-yl]-2,2-dimethyl-tetrahydro-furo[3,4-d][1,3]dioxole-4-carboxylic acid amide

To a cool solution of (3aS,4S,6R,6aR)- 6-[2-chloro-6-(2,2-diphenyl-ethylamino)-purin-9-yl]-2,2-dimethyl- tetrahydro-furo[3,4-d][1,3]dioxole-4-carboxylic acid [Preparation 4 in PCT Patent Application No. WO94/17090] (6.03g, 11.3mmol) in dichloromethane (48ml), at 0°C, triethylamine (1.73ml, 12.4mmol) and pivaloyl chloride (1.53ml, 12.4mmol) were added. The resultant solution was stirred at 0°C for 1.5h. Ammonia gas was bubbled through the cool solution for 40mins. The white slurry was concentrated *in vacuo*, dissolved in ethyl acetate (50ml) and washed with water (3x 50ml), then the aqueous was extracted with ethyl acetate (50ml). The combined organics were dried (MgSO₄), then concentrated *in vacuo*. The cream solid was triturated with dichloromethane, the resulting solid was filtered off and dried to afford the title compound as a white solid (3.82g). TLC SiO₂ (neat ethyl acetate) Rf = 0.75

### Intermediate 15: N-[2-Chloro-9-(6R-cyano-2,2-dimethyl-tetrahydro-(3aR,6aR)-furo[3,4-d][1,3]dioxol-4R-yl)-9H-purin-6-yl]-N-(2,2-diphenyl-ethyl)-formamide

Triethylamine (0.69ml, 4.96mmol) and 4,4-dimethylaminopyridine (0.023g, 0.19mmol), were added to a cool, 0°C, stirring suspension, of Intermediate 14 (0.511g, 0.953mmol) in anhydrous acetonitrile (12ml). Phosphorous oxychloride (0.45ml, 4.77mmol) was added cautiously to the cooled mixture over 10min. The solution was stirred at room temperature for 30mins, then cooled to 0°C, and dimethylformamide (4ml) was added. The resultant brown slurry was heated at 95°C with stirring under nitrogen for 20h. The cooled mixture was concentrated *in vacuo,* then partitioned between ethyl acetate (25ml) and water (30ml). The aqueous layer was back extracted with ethyl acetate (2x 25ml). The combined organics were dried (MgSO₄), then concentrated *in vacuo.* Purification by column chromatography on flash silica eluted with 30-50% ethyl acetate - cyclohexane afforded the title compound as a beige foam (0.43g). TLC SiO₂ (40% ethyl acetate in cyclohexane), Rf = 0.55

### Intermediate 16: (3aR,4R,6R,6aR)-6-[2-Chloro-6-(2,2-diphenyl-ethylamino)-purin-9-yl]-N-hydroxy-2,2-dimethyl-tetrahydro-furo[3,4-d][1,3]dioxole-4-carboxamidine

To a solution of the Intermediate 15 (0.5g, 0.965mmol) in ethanol (12ml), potassium carbonate (0.267g, 1.93mmol), and hydroxylamine hydrochloride (0.246g, 3.57mmol) was added. The reaction mixture was refluxed at 80°C for 19h, under nitrogen. The solution was concentrated *in vacuo*, then dissolved dichloromethane (50ml), and washed with water (50ml). The aqueous was back extracted with dichloromethane (50ml), the combined organics were dried (MgSO₄), then concentrated *in vacuo* to afford the title compound as a beige foam (0.458g). TLC SiO₂ (50% ethyl acetate in cyclohexane) Rf = 0.34.

### Intermediate 17: {2-Chloro-9-[6R-(5-ethyl-[1,2,4]oxadiazol-3-yl)-2,2-dimethyl-tetrahydro-(3aR,6aR)-furo[3,4-d][1,3]dioxol-4R-yl]-9H-purin-6-yl}-(2,2-diphenylethyl)-amine

Intermediate 16 (0.525g, 0.954mmol) in propionic acid (7.5ml), was treated with propionic anhydride (0.147ml, 1.145mmol), then stirred at room temperature for 2h under nitrogen. The mixture was heated at 90°C for 7h, then concentrated *in vacuo*, and azeotroped with toluene (2x 20ml). The residue was purified by column chromatography on flash silica eluted with 50% ethyl acetate-cyclohexane to yield the title compound as a white solid (0.46g).
TLC SiO₂ (50% ethyl acetate in cyclohexane) Rf = 0.44

### Intermediate 18: (2R,3R,4S,5R)-2-[2-Chloro-6-(2,2-diphenyl-ethylamino)-purin-9-yl]-5-(5-ethyl-[1,2,4]oxadiazol-3-yl)-tetrahydro-furan-3,4-diol

Intermediate 17 (0.46g, 0.784mmol) in trifluoroacetic acid / water (4:1, 8ml), was stirred at 0°C for 4.5h. The mixture was concentrated *in vacuo*, azeotroped with toluene (2 x 15ml). Purification with Solid Phase Extraction (SPE) cartridge (NH2 aminopropyl Bondelute) (2mL cartridge) eluted with dichloromethane (20ml), ethyl acetate (20ml), acetonitrile (20ml) and methanol (20ml). Evaporation of the methanol fraction *in vacuo* afforded the title compound as a cream solid (0.416g). LC/MS system A Rₜ = 4.56min. *m*/*z* = 548 MH⁺.

### Intermediate 19: (3aS,4S,6R,6aR)-6-[2-Chloro-6-(2,2-diphenyl-ethylamino)-purin-9-yl]-2,2-dimethyl-tetrahydro-furo[3,4-d][1,3]dioxole-4-carboxylic acid N'-propionyl-hydrazide

The (3aS,4S,6R,6aR)- 6-[2-chloro-6-(2,2-diphenyl-ethylamino)-purin-9-yl]-2,2-dimethyl-tetrahydro-furo[3,4-d][1,3]dioxole-4-carboxylic acid [Preparation 4 from PCT Patent Application No. WO94/17090 (2.15g, 4.0mmol) in anhydrous tetrahydrofuran (40ml) at 0°C was treated with diisopropylethylamine (2.44ml, 14mmol), and pivaloyl chloride (0.493ml, 4.0mmol). The resultant solution was stirred for 2.5h at 0°C, then propionic hydrazide trifluoroacetate (0.840g, 4.16mmol) was added in tetrahydrofuran (8ml). The solution was stirred at room temperature for 3 days. The reaction mixture was concentrated *in vacuo,* then the residue dissolved in ethyl acetate (50ml), washed with saturated sodium bicarbonate solution (50ml). The aqueous was back extracted with ethyl acetate (50ml), the combined organics were washed with brine (80ml), dried (MgSO₄), then concentrated *in vacuo* to afford the title compound as a cream solid (2.189g). LC/MS system B R, = 3.33min, *m*/*z* = 606 MH⁺.

### Intermediate 20: {2-Chloro-9-[6S-(5-ethyl-[1,3,4]oxadiazol-2-yl)-2,2-dimethyl-tetrahydro-(3aR,6aS)-furo[3,4-d][1,3]dioxol-4R-yl]-9H-purin-6-yl}-(2,2-diphenylethyl)-amine

To a solution of the Intermediate 19 (0.250g, 0.413mmol) in dimethylformamide (2ml) at 0°C, phosphorous oxychloride (0.06ml, 0.661mmol) was added. The solution was stirred at 0°C for 4h. The solution was concentrated *in vacuo*, then the residue was dissolved in ethyl acetate (30ml), and washed with saturated sodium bicarbonate solution (2x 30ml). The aqueous was back extracted with ethyl acetate (30ml), and the combined organics were washed with brine (50ml), dried (MgSO₄), then concentrated *in vacuo* to give a yellow oil. Purification by column chromatography on flash silica eluted with 50% ethyl acetate - cyclohexane yielded the title compound as a cream solid (0.119g).
TLC SiO₂ (50% ethyl acetate in cyclohexane) Rf = 0.35.

### Intermediate 21: (2R,3R,4S,5S)-2-[2-Chloro-6-(2,2-diphenyl-ethylamino)-purin-9-yl]-5-(5-ethyl-[1,3,4]oxadiazol-2-yl)-tetrahydro-furan-3,4-diol

Intermediate 20 (0.35g, 0.596mmol) in trifluoroacetic acid / water solution (10:1, 4ml), was stirred at 0°C for 2 h, then at 25°C for 2h. The mixture was concentrated *in vacuo*, azeotroped with toluene (3 x 10ml), to afford the title compound as a white solid (0.290g). LC/MS system B Rₜ = 3.20min, *m*/*z* = 548 MH⁺.

### Intermediate 22: {2-Chloro-9-[2,2-dimethyl-6R-(2H-[1,2,4]triazol-3-yl)-tetrahydro-(3aR,6aR)-furo[3,4-d][1,3]dioxol-4R-yl]-9H-purin-6-yl)-(2,2-diphenyl-ethyl)-amine

Intermediate 9 (2.500g), formimidate hydrochloride (0.748g) and triethylamine (25.8ml) in ethanol (20ml) were heated to reflux for 68 hrs. Solvent was removed *in vacuo* and the residue was purified by column chromatography on flash silica twice firstly with ethylacetate-cyclohexane (1:1 to neat ethyl acetate), then with cyclohexane-ethyl acetate (10:1, 5:1, 2:1, 1:1, 1:2 and then neat ethyl acetate) to give the title compound as a crisp orange foam (0.185g).
TLC SiO₂ (neat ethyl acetate) Rf = 0.27

### Intermediate 23: {2-Chloro-9-[6R-(1-ethyl-1H-[1,2,4]triazol-3-yl)-2,2-dimethyl-tetrahydro-(3aR,6aR)-furo[3,4-d][1,3]dioxol-4R-yl]-9H-purin-6-yl}-(2,2-diphenylethyl)-amine

Intermediate 22 (0.185mg, 0.33mM), iodoethane (0.057g) and potassium carbonate (0.055g) in DMF were stirred at 20°C for 65 hrs. The reaction mixture was separated between ethyl acetate (40ml) and water (20ml), washed with water (20ml), brine (20ml), dried (MgSO4) and solvent was removed *in vacuo.* The residue was purified by column chromatography on flash silica (ethylacetate-cyclohexane 2:1 then neat ethyl acetate) to give the title compound as an orange coloured glass (0.122g). TLC SiO₂ (neat ethyl acetate) Rf = 0.34

### Intermediate 24: (2R,3R,4S,5R)-2-[2-Chloro-6-(2,2-diphenyl-ethylamino)-purin-9-yl]-5-(1-ethyl-1H-[1,2,4]triazol-3-yl)-tetrahydro-furan-3,4-diol

Intermediate 23 (0.117g, 0.2mM) was heated at 120°C for 6hrs. in a mixture of glacial acetic acid (2ml) and water (2ml). Solvent was removed *in vacuo*, the residue was azetroped with toluene (3 x 10ml) and left under high vacuum for 16 hrs. furnishing the title compound as a cream coloured solid (0.101g).
TLC SiO₂ (neat ethyl acetate Rf = 0.25

### Intermediate 25: 2-Chloro-N-(1-Ethylpropyl)-adenosine

A mixture of 2,6-dichloro-9-(2,3,5-tri-O-acetyl-β-D-ribofuranosyl)-9H-purine (described in M.J. Robins and B. Uznanski, Canad. J. Chem., 1981, 59(17), 2608) (10.1g, 22.6mM), iso-propanol (300ml), K₂CO₃ (5g) and 1-ethylpropylamine (2.17g, 24.84mM) was stirred at 20°C for 24hrs. The reaction mixture was heated at 54 C for 73 hrs. Solvent was removed in vacuo, water (50ml) was added, extracted with ethyl acetate (3 x 80ml), the combined extracts were dried (Mg₂SO₄) affording the title compound as a creamy light brown foam (9.44g) LC/MS system A Rₜ = 2.66 min, *m*/*z* = 372 MH⁺.

### Intermediate 26: {6R-[2-Chloro-6-(1-ethyl-propylamino)-purin-9-yl]-2,2-dimethyl-tetrahydro-(3aR,6aR)-furo[3,4-d][1,3]dioxol-4R-yl}-methanol

A mixture of Intermediate 25 (9.3g, 22.6mmol), 2,2-dimethoxypropane (35ml), actone (250ml) and para-toluenesulfonic acid (8.1g) was stirred for 22 hrs. at 20°C. The solvent was removed *in vacuo* and the residue taken up in ethyl acetate (200ml), washed with sodium bicarbonate (aqueous, saturated, 3 x 70ml). The aqueous washings were back extracted with ethyl acetate (50ml). The combined organic layers were dried (MgSO₄) and solvent was removed in vacuo. The residue was purified by column chromatography on flash silica (50%, 60% and then 70% ethyl acetate - cyclohexane) to afford the title compound as a white foam (5.67g). TLC SiO₂ (50% ethyl acetate in cyclohexane) Rf = 0.17

### Intermediate 27: (3aS,4S,6R,6aR)-6-[2-Chloro-6-(1-ethyl-propylamino)-purin-9-yl]-2,2-dimethyl-tetrahydro-furo[3,4-d][1,3]dioxole-4-carboxylic acid

A mixture of Intermediate 26 (5.431g, 13.2mmol), KBr (0.157g, 1.32mmol), TEMPO, (0.010g, 0.07mmol) in ethyl acetate (205ml) and saturated aqueous NaHCO₃ (138ml) was vigorously stirred for 20 mins. at 0°C. A mixture made up of sodium hypochlorite (13% active chloride, 7.3ml) solid NaHCO₃ (0.420g) and water (2ml) was added dropwise over 5 mins. After 30 mins. more reagents (KBr, TEMPO, sodium hypochlorite, solid NaHCO₃, and water in the same quantities as above) were added. This addition was repeated after a further 30 mins had elapsed. One hour later the reaction mixture was poured into aqueous solution of Na₂SO₃ (28g) in water (400ml), diluted with ethyl acetate (100ml). The mixture was vigorously shaken and the organic phase washed with water (100ml). The combined aqueous layers were cooled to 0°C and acidified to pH 3 with 2M hydrochloric acid, extracted with ethyl acetate (3 x 200ml), dried (MgSO₄) and solvent was removed in vacuo leaving the title compound as a white foam (5.03g) LC/MS system B Rₜ = 3.25min, *m*/*z* = 426 MH⁺.

### Intermediate 28: Cyclopropanecarboxylic acid N'-{6R-[2-chloro-6-(1-ethylpropylamino)-purin-9-yl]-2,2-dimethyl-tetrahydro-(3aS,6aR)-furo[3,4-d][1,3]dioxole-4S-carbonyl}-hydrazide

Trimethylacetyl chloride (0.52ml, 4.2mmol) was added to a stirred solution of Intermediate 27 (1.5g, 3.5mmol) and N,N-Diisopropylethylamine (2.4ml, 14mmol) in tetrahydrofuran (18ml) under nitrogen at 0°C and stirring continued for 2 h. Mixture cooled again to 0°C and cyclopropanecarboxylic acid hydrazide [Ref: Roberts, J. Amer.Chem.Soc., 1951, 73, 2959] (0.62g, 4.5mmol) added in tetrahydrofuran (8ml) and mixture stirred for 16h. allowing to warm to room temperature. The mixture was poured into saturated sodium bicarbonate solution (50ml) and extracted with ethyl acetate (3 x 100ml). The organic layers were combined and washed with brine (100ml), dried over sodium sulphate and concentrated *in vacuo* to give crude product as yellow oil. Purification with Solid Phase Extraction (SPE) cartridge (Varian NH2 aminopropyl Bondelute, 10ml cartridge) eluted with ethyl acetate/cyclohexane 1:9 - 1:1 to afford title compound as white solid (1.567g) after concentration *in vacuo*.
LC/MS SYSTEM B R_{T}=3.07mins, *M*/*Z*=508 MH⁺

### Intermediate 29: {2-Chloro-9-[6S-(5-cyclopropyl-[1,3,4]oxadiazol-2-yl)-2,2-dimethyl-tetrahydro-(3aR,6aS)-furo[3,4-d][1,3]dioxol-4R-yl]-9H-purin-6-yl}-(1-ethyl-propyl)-amine

Phosphorous oxychloride (0.46ml,4.92mmol) was added to a stirred suspension of Intermediate 28 (1.567g, 3.08mmol) in acetonitrile(15ml,anhydrous) under nitrogen at room temperature. Mixture heated to reflux (90°C) for 3h with stirring. Reaction was cooled and phosphorous oxychloride (0.3ml, 3.2mmol) was added and reaction heated back to reflux for 2.5h. Reaction carefully quenched by addition of aqueous saturated sodium bicarbonate solution (100ml) and extracted with dichloromethane (3 x 50ml), washed with brine (50ml) and dried over sodium sulphate, concentrating *in vacuo.* Purification by column chromatography on flash silica eluting with 1:1 ethyl acetate/ cyclohexane to afforded title compound as pale yellow oil (0.77g) LCMS SYSTEM B Rₜ=3.41mins *m*/*z*=490 MH⁺

### Intermediate 30: (2R,3R,4S,5S)-2-[2-Chloro-6-(1-ethyl-propylamino)-purin-9-yl]-5-(5-cyclopropyl-[1,3,4]oxadiazol-2-yl)-tetrahydro-furan-3,4-diol

Intermediate 29 (0.65g, 1.32mmol) was dissolved in trifluoroacetic acid/water (10:1, 5.5ml) at 0°C under nitrogen with stirring for 4h and left in refrigerator (4°C) for 16h. The mixture was concentrated in vacuo and poured slowly on to saturated sodium bicarbonate solution (100ml), extracting with dichloromethane(3x 50ml), washing with brine, and drying with sodium sulphate. Concentrating *in vacuo* afforded the title compound as an off-white solid(0.65g). LCMS SYSTEM B Rₜ=3.04mins *m*/*z*=450 MH⁺

### Intermediate 31: {2-(2-Piperidin-1-yl-ethylamino-9-[2,2-dimethyl-6S-(5-methyl-[1,3,4]thiadiazol-2-yl)-tetrahydro-(3aR,6aS)-furo[3,4-d][1,3]dioxol-4R-yl]-9H-purin-6-yl}-(2,2-diphenyl-ethyl)-amine

A solution of Intermediate 7 (0.04g, 0.06mmol) in DMSO (0.05ml) was treated with 2-piperidinoethylamine (0.04ml, 0.30mmol) and heated at 80°C in a sealed a sealed vial (eg Reacti-vial™) for 72h. After cooling, purification with Solid Phase Extraction (SPE) cartridge (Varian NH2 aminopropyl Bondelute, 2mL cartridge) eluting with dichloromethane. Concentration *in vacuo* afforded the title compound as a brown solid (0.04g). LC/MS SYSTEM B Rₜ = 2.74 min; *m*/*z* = 682 (MH⁺)

### Intermediate 32: (2R,3R,4S,5R)-2-[2-(2-Amino-ethylamino)-6-(2,2-diphenylethylamino)-purin-9-yl]-5-(5-ethyl-[1,2,4]oxadiazol-3-yl)-tetrahydro-furan-3,4-diol diformate

Intermediate 18 (0.038g, 0.069mmol) and ethylenediamine (0.023ml, 0.345mmol) were dissolved in DMSO (0.03ml) and heated at 80° C for 18h. in a sealed vial (eg Reacti-vial™). Purification by Autoprep. HPLC to give the title compound after freeze drying as a cream solid (0.02g).
LC/MS system B Rₜ = 2.56min, *m*/*z* = 572 MH⁺.

### Intermediate 33: 1-[(3aR,4R,6R,6aR)-6-methoxy-2,2-dimethyltetrahydrofuro[3,4-d][1,3]dioxol-4-yl]pent-1-yn-3-ol

A solution of 4R-ethynyl-6R-methoxy-2,2-dimethyl-tetrahydro-(3aR,6aR)-furo[3,4-d][1,3]dioxole [lit. compound; Ref: Helv. Chim. Acta 1980, 63, 1181-1189] (1.5g) in tetrahydrofuran (20ml) was cooled to -78°C for 15 minutes under nitrogen. A solution of propionaldehyde (1.09ml) in tetrahydrofuran (0.5ml) was added via syringe and stirring continued for 5h. The mixture was allowed to warm to 22°C and stirred for a further 16h. The solvents were removed in vacuo and the resultant orange oil partitioned between ether and aqueous ammonium chloride. The organic layers were washed with further aqueous ammonium chloride, dried (MgSO₄), and concentrated *in vacuo* to afford a yellow oil. Purification by chromatography on silica gel (Varian Bondelut cartridge), eluting with (i) cyclohexane, (ii) dichloromethane, (iii) ether, (iv) ethyl acetate afforded the title compound as a colourless oil (1.33g).
TLC SiO₂ (ether:cyclohexane 1:1) R_{f} 0.39

### Intermediate 34: 1-[(3aR,4R,6R,6aR)-6-methoxy-2,2-dimethyltetrahydrofuro[3,4-d][1,3]dioxol-4-yl]pent-1-yn-3-one

A solution of Intermediate 33 (1.3g) in dichloromethane (100ml) was added to a stirred suspension of manganese dioxide (60g) in dichloromethane at 0°C. The mixture was stirred at 0°C for 3h, filtered through magnesium sulphate (50g) and the solvent removed *in vacuo* to give the title compound as a colourless oil (550mg). TLC SiO₂ (ether:cyclohexane 1:1) R_{f} 0.68

### Intermediate 35: 1-[(3aR,4R,6R,6aR)-6-methoxy-2,2-dimethyltetrahydrofuro[3,4-d][1,3]dioxol-4-yl]pentane-1,3-dione 1-oxime

A mixture of Intermediate 34 (550mg) and hydroxylamine (50% solution in water) (0.2ml) in ethanol (10ml) was stirred overnight at 22°C. The mixture was concentrated in *vacuo* to afford the title compound as a yellow oil (554mg, 89%). TLC SiO₂ (ether:cyclohexane 1:1) R_{f} 0.36

### Intermediate 36: (3R,4S,5R)-5-(5-ethylisoxazol-3-yl)tetrahydrofuran-2,3,4-triol isomer 1

Intermediate 35 (0.5g) was dissolved in aqueous acetic acid (18mg) and the mixture heated at 100°C for 2h. The solution was cooled and concentrated *in vacuo* to afford a brown oil which was azeotroped with toluene. Purification by chromatography on silica gel (Varian Bondelut silica gel cartridge), eluting with (i) dichloromethane, (ii) ether, (iii) ethyl acetate, (iv) methanol, gave the title compound (150mg). TLC SiO₂ (ether) R_{f} 0.17

### Intermediate 37: (2R,3R,4R)-4,5-bis(acetyloxy)-2-(5-ethylisoxazol-3-yl)tetrahydrofuran-3-yl acetate isomer 1

Intermediate 36 (150mg) was dissolved in pyridine (4ml) and the mixture treated with acetic anhydride (0.983ml). The resulting solution was stirred at 22°C overnight. The mixture was concentrated *in vacuo* to afford a brown oil. Purification by chromatography on silica gel (Varian Bondelut SiO₂ cartridge), eluting with (i) dichloromethane, (ii) ether (iii) ethyl acetate, afforded the title compound as a pale yellow solid. TLC SiO₂ (ether) R_{f} 0.53

### Intermediate 38: (2R,3R,4R,5R)-4-(acetyloxy)-2-(2,6-dichloro-9H-purin-9-yl)-5-(5-ethylisoxazol-3-yl)tetrahydrofuran-3-yl acetate

Intermediate 37 (193mg) was dissolved in acetonitrile (5ml) and treated sequentially with 2,6-dichloropurine (213mg), 1,8-diazabicyclo[5.4.0]undec-7-ene (DBU) (0.186ml) and trimethylsilyl trifluoromethanesulphonate (TMSOTf) (0.225ml) via a syringe over 5 min. The clear yellow solution was stirred at 22°C for 40h, at 60°C for 21h, and at 80°C for 6h. The mixture was cooled to room temperature and more DBU (0.186ml) and TMSOTf (0.225ml) were added. After stirring at 22°C for 36h the yellow mixture was heated at 60°C overnight and at 80°C for 6h. The solvents were removed *in vacuo* and the resultant brown oily solid taken up in ethyl acetate and washed with water (20ml, 3:1). The aqueous layer was extracted with ethyl acetate and the combined organic layers dried (MgSO₄) and evaporated *in vacuo* to afford a brown oily solid. The residue was triturated with dichloromethane and a white solid removed by filtration. Evaporation of the filtrate afforded a tan solid. Purification by flash chromatography on silica gel eluting with ether:cyclohexane (1:1) afforded the title compound as a white solid (161mg). LC/MS (System C) Rₜ 3.34min.
Mass spectrum *m*/*z* 470/2 [MH⁺]

### Intermediate 39: (2R,3R,4R,5R)-4-(acetyloxy)-2-(2-chloro-6-[(1-ethylpropyl)amino]-9H-purin-9-yl}-5-(5-ethylisoxazol-3-yl)tetrahydrofuran-3-yl acetate

Intermediate 38 (125mg) was dissolved in isopropanol (5ml) and the solution was treated with diisopropylethylamine (0.06ml) followed by 1-ethylpropylamine (0.044ml). The mixture was heated at 50°C with stirring under nitrogen for 16h. The solvent was removed *in vacuo* and the mixture partitioned between ethyl acetate and 1M hydrochloric acid (3:1). The organic layers were washed with sodium bicarbonate solution and brine, dried (MgSO₄) and evaporated *in vacuo.* Purification by chromatography on silica gel (Varian Bondelut cartridge), eluting with (i) dichloromethane, (ii) ether and (iii) ethyl acetate, gave the title compound as a colourless oil (108mg). TLC SiO₂ (ether) R_{f} 0.26.

### Example 1: (2R,3R,4S,5R)-2-[6-(2,2-Diphenyl-ethylamino)-2-(2-pyrrolidin-1-yl-ethylamino)-purin-9-yl]-5-(5-methyl-4H-[1,2,4]triazol-3-yl)-tetrahydro-furan-3,4-diol formate

Intermediate 10 (0.035g, 0.07mmol) was treated with a solution of 2-N-aminoethylpyrrolidine (0.33mmol) in DMSO (5 drops) in a sealed vial (eg Reactivial™) and heated at 100°C for 48 hours. The reaction mixture was cooled and loaded directly onto a Solid Phase Extraction (SPE) cartridge (NH2 aminopropyl Bondelute) (2mL cartridge). The column was flushed with column volumes of cyclohexane, dichloromethane and acetonitrile. Material in two column volumes of methanol was combined, solvent was removed *in vacuo* and treated with a mixture of trifluoroacetic acid:water (9:1, 1ml), stirred at 20°C for 1 hour prior to concentration *in vacuo*. The residue was co-evaporated with methanol prior to purification by Autoprep. HPLC to afford the title compound as a solid product (0.011g). LC-MS System A Rt.= 3.83 min., *m*/*z* 611 (MH⁺)

### Example 2: (2R,3R,4S,5R)-2-[6-(2,2-Diphenyl-ethylamino)-2-(1S-hydroxymethyl-2-phenyl-ethylamino)-purin-9-yl]-5-(5-methyl-4H-[1,2,4]triazol-3-yl)-tetrahydro-furan-3,4-diol formate

Example 2 was prepared in an analogous manner to Example 1 using (S)-(-)-2-amino-3-phenyl-1-propanol (0.33mmol). The title compound was obtained as a solid (0 011g) LC-MS System A Rt.= 3.02 min., *m*/*z* 648 (MH⁺)

### Example 3: (2R,3R,4S,5R)-2-{6-(2,2-Diphenyl-ethylamino)-2-[2-(1-methyl-1H-imidazol-4-yl)-ethylamino]-purin-9-yl}-5-(5-methyl-4H-[1,2,4]triazol-3-yl)-tetrahydro-furan-3,4-diol formate

Example 3 was prepared in an analogous manner to Example 1 using 1-methylhistamine (0.33mmol). The title compound was obtained as a solid (0 002g) LC-MS System A Rt.= 3.79 min., *m*/*z* 622 (MH⁺)

### Example 4: (2R,3R,4S,5R)-2-[2-(trans-4-Amino-cyclohexylamino)-6-(2,2-diphenyl-ethylamino)-purin-9-yl]-5-(5-methyl-4H-[1,2,4]triazol-3-yl)-tetrahydro-furan-3,4-diol formate

Example 4 was prepared in an analogous manner to Example 1 using trans-1,4-diaminocyclohexane (0.33mmol). The title compound was obtained as a solid (0.009g) LC-MS System A Rt.= 3.83 min., *m*/*z* 611 (MH⁺)

### Example 5: (2R,3R,4S,5R)-2-[6-(2,2-Diphenyl-ethylamino)-2-(1S-hydroxymethyl-2-phenyl-ethylamino)-purin-9-yl]-5-(5-ethyl-4H-[1,2,4]triazol-3-yl)-tetrahydro-furan-3,4-diol formate

A mixture of Intermediate 11 (0.035g, 0.06mmol) in DMSO (0.04ml) and 3-(S)-(-)-2-amino-3-phenyl propanol (0.045g, 0.3mmol) was heated at 90-120°C in a sealed vial (eg Reactivial™) for 5 days. The brown residue was dissolved in trifluoroacetic acid / water solution (9:1, 1 ml). The solution was stirred for 1.5h. at room temperature, then the TFA blown off with nitrogen. Purification by Autoprep. HPLC afforded the title compound after freeze drying as a cream solid (0.004g). LC/MS SYSTEM A Rₜ = 4.36min, *m*/*z* = 662 MH⁺

### Example 6: (2R,3R,4S,5R)-2-[2-(trans-4-Amino-cyclohexylamino)-6-(2,2-diphenyl-ethylamino)-purin-9-yl]-5-(5-ethyl-4H-[1,2,4]triazol-3-yl)-tetrahydro-furan-3,4-diol formate

A mixture of Intermediate 11 (0.035g, 0.06mmol) in DMSO (0.04ml), and *trans*-1,4-diaminocyclohexane (0.034g, 0.3mmol) was heated at 90-100°C in a sealed vial (eg Reactivial™) for 4 days. The residue was dissolved in trifluoroacetic acid / water solution (9:1, 1ml). The solution was stirred for 1.5h at room temperature, then the TFA blown off with nitrogen. Purification by Autoprep. HPLC afforded the title compound after freeze drying as a beige coloured solid (0.004g). LC/MS SYSTEM A Rₜ = 3.60min, *m*/*z* = 625 MH⁺

### Example 7: (2R,3R,4S,5R)-2-[6-(2,2-Diphenyl-ethylamino)-2-(1S-hydroxymethyl-2-methyl-propylamino)-purin-9-yl]-5-(5-ethyl-4H-[1,2,4]triazol-3-yl)-tetrahydro-furan-3,4-diol formate

A mixture of Intermediate 11 (0.035g, 0.06mmol) in DMSO (0.04ml), and L-2-amino-3-methylbutanol (0.031g, 0.3mmol) was heated at 90-120°C in a sealed vial (eg Reactivial™) for 5 days. The residue was dissolved up trifluoroacetic acid / water solution (9:1, 1ml). The solution was stirred for 1.5h at room temperature, then the TFA blown off with nitrogen. Purification by Autoprep. HPLC afforded the title compound after freeze drying as a white solid (0.003g). LC/MS SYSTEM A Rₜ = 4.26min, *m*/*z* = 614 MH⁺

### Example 8: (2R,3R,4S,5R)-2-[6-(2,2-Diphenyl-ethylamino)-2-(2-morpholin-4-yl-ethylamino)-purin-9-yl]-5-(5-ethyl-4H-[1,2,4]triazol-3-yl)-tetrahydro-furan-3,4-diol formate

A mixture of Intermediate 11 (0.035g, 0.06mmol) in DMSO (0.04ml), and 4-(2-aminoethyl)morpholine (0.039ml, 0.3mmol) was heated at 90°C in a sealed vial (eg Reacti vial™) for 48h.. The residue was dissolved up in trifluoroacetic acid / water solution (9:1, 1ml). The solution was stirred for 1.5h at room temperature, then the TFA blown off with nitrogen. Purification by Autoprep. HPLC afforded the title compound after freeze drying as a pale brown gum (0.004g).
LC/MS SYSTEM A Rₜ = 3.63min, *m*/*z* = 642 MH⁺

### Example 9: (2R,3R,4S,5R)-2-[6-(2,2-Diphenyl-ethylamino)-2-(pyrrolidin-3R-ylamino)-purin-9-yl]-5-(5-ethyl-4H-[1,2,4]triazol-3-yl)-tetrahydro-furan-3,4-diol formate

A mixture of Intermediate 11 (0.035g, 0.06mmol) in DMSO (0.04ml), and (3R)-(+)-3-aminopyrrolidine (0.029ml, 0.3mmol) was heated at 90°C in a sealed vial (eg Reactivial™) for 48h. The residue was dissolved in trifluoroacetic acid / water solution (9:1, 1ml). The solution was stirred for 1.5h at room temperature, then the TFA blown off with nitrogen. Purification by Autoprep. HPLC afforded the title compound after freeze drying as a brown gum (0.003g).
LC/MS SYSTEM B Rₜ = 2.44min, *m*/*z* = 598 MH⁺

### Example 10: (2R,3R,4S,5R)-2-[2-(trans-4-Amino-cyclohexylamino)-6-(2,2-diphenyl-ethylamino)-purin-9-yl]-5-(5-isopropyl-4H-[1,2,4]triazol-3-yl)-tetrahydro-furan-3,4-diol formate

Intermediate 13 (0.028g, 0.05mmol) and *trans*-1,4-diaminocyclohexane (0.028g, 0.248mmol) were dissolved in DMSO (0.05ml) and heated at 90° C in a sealed vial (eg Reactivial™) for 2 days. Purification by Autoprep. HPLC afforded the title compound after freeze drying as a beige coloured solid (0.017g). LC/MS SYSTEM B R, = 2.48 min, *m*/*z* = 639 MH⁺

### Example 11: (2R,3R,4S,5R)-2-[6-(2,2-Diphenyl-ethylamino)-2-(1S-hydroxymethyl-2-phenyl-ethylamino)-purin-9-yl]-5-(5-isopropyl-4H-[1,2,4]triazol-3-yl)-tetrahydro-furan-3,4-diol formate

Intermediate 13 (0.028g, 0.05mmol) and 3-(S)-(-)-2-amino-3-phenyl propanol (0.037g, 0.248mmol) were dissolved in DMSO (0.05ml) and heated at 90-120° C in a sealed vial (eg Reactivial™) for 3 days. Purification by Autoprep. HPLC afforded the title compound after freeze drying as a cream solid (0.014g).
LC/MS SYSTEM B Rₜ = 3.17 min, *m*/*z* = 676 MH⁺

### Example 12: (2R,3R,4S,5R)-2-[2-(trans-4-Amino-cyclohexylamino)-6-(2,2-diphenyl-ethylamino)-purin-9-yl]-5-(5-ethyl-[1,2,4]oxadiazol-3-yl)-tetrahydro-furan-3,4-diol diformate

Intermediate 18 (0.038g, 0.069mmol) and trans -1,4-diaminocyclohexane (0.039g, 0.345mmol) were dissolved in DMSO (0.03ml) and heated at 80° C for 3.5 days in a sealed vial (eg Reactivial™). Purification by Autoprep HPLC followed by freeze-drying yielded the title compound as a white solid (0.007g) LC/MS system A Rₜ = 3.71 min, *m*/*z* = 626 MH⁺.

### Example 13: (2R,3R,4S,5R)-2-{6-(2,2-Diphenyl-ethylamino)-2-[2-(1-methyl-1H-imidazol-4-yl)-ethylamino]-purin-9-yl}-5-(5-ethyl-[1,2,4]oxadiazol-3-yl)-tetrahydro-furan-3,4-diol formate

Intermediate 18 (0.038g, 0.069mmol) and 1-methylhistamine (0.043g, 0.345mmol) were dissolved in DMSO (0.03ml) and heated at 80-120° C for 4.5 days in a sealed vial (eg Reactivial™). Purification by Autoprep HPLC followed by freeze-drying yielded the title compound as a cream coloured solid (0.006g) LC/MS system B Rₜ = 2.59min, *m*/*z* = 637 MH⁺.

### Example 14: (2R,3R,4S,5R)-2-[6-(2,2-Diphenyl-ethylamino)-2-(2-piperidin-1-yl-ethylamino)-purin-9-yl]-5-(5-ethyl-[1,2,4]oxadiazol-3-yl)-tetrahydro-furan-3,4-diol formate

Intermediate 18 (0.025g, 0.046mmol) and 2-piperidinoethylamine (0.032ml, 0.23mmol) were dissolved in DMSO (0.1ml) and heated at 85° C under nitrogen for 44h.. Purification by Autoprep. HPLC afforded the title compound after freeze drying as a white solid (0.014g). LC/MS system B Rₜ = 2.64min, *m*/*z* = 640 MH⁺.

### Example 15: (2R,3R,4S,5R)-2-[6-(2,2-Diphenyl-ethylamino)-2-(2-morpholin-4-yl-ethylamino)-purin-9-yl]-5-(5-ethyl-[1,2,4]oxadiazol-3-yl)-tetrahydro-furan-3,4-diol formate

Intermediate 18 (0.038g, 0.069mmol) and 4-(2-aminoethyl) morpholine (0.045ml, 0.345mmol) were dissolved in DMSO (0.03ml) and heated at 80° C for 18h in a sealed vial (eg Reactivial™). Purification by Autoprep HPLC followed by freeze-drying yielded the title compound as a white solid (0.017g)
LC/MS system B Rₜ = 2.56min, *m*/*z* = 642 MH⁺.

### Example 16: (2R,3R,4S,5R)-2-[6-(2,2-Diphenyl-ethylamino)-2-(1S-hydroxymethyl-2-phenyl-ethylamino)-purin-9-yl]-5-(5-ethyl-[1,2,4]oxadiazol-3-yl)-tetrahydro-furan-3,4-diol formate

Intermediate 18 (0.038g, 0.069mmol) and 3-(S)-(-)-2-amino-3-phenyl propanol (0.0529, 0.345mmol) were dissolved in DMSO (0.03ml) and heated at 80-100° C for 3.5 days in a sealed vial (eg Reactivial™). Purification by Autoprep HPLC followed by freeze-drying yielded the title compound as a white solid (0.004g). LC/MS system A Rₜ = 4.43min, *m*/*z* = 663 MH⁺.

### Example 17: (2R,3R,4S,5S)-2-[6-(2-Cyclohexyl-ethylamino)-2-(1S-hydroxymethyl-2-phenyl-ethylamino)-purin-9-yl]-5-(5-methyl-oxazol-2-yl)-tetrahydro-furan-3,4-diol formate

Intermediate 5 (0.012g), N,N-diisopropylethylamine (0.004g), 2-cyclohexyethylamine (0.003g) in isopropanol (0.75ml) were left to stand at room temperature for 16hrs. The solvent was removed , (S)-(-)-2-amino-3-phenyl-1-propanol (0.030g) and DMSO (0.03ml) added and the mixture heated in sealed vials (eg Reactivial™) at 90°C for 32hrs, then at 120°C for 16hrs. (S)-(-)-2-amino-3-phenyl-1-propanol (0.025g) and DMSO (0.1ml) were added and the vials heated at 120°C for 16hrs. Purification by Autoprep followed by freeze-drying gave the title compound as a yellow solid (0.002g)
LC-MS System A Rt=4.46min *m*/*z* 578 (MH⁺)

### Example 18: (2R,3R,4S,5S)-2-[6-(2,2-Diphenyl-ethlyamino)-2-(pyrrolidin-3R-ylamino)-purin-9-yl]-5-(5-methyl-oxazol-2-yl)-tetrahydro-furan-3,4-diol diformate

Intermediate 5 (0.012g), N,N-diisopropylethylamine (0.025mmolol) 2,2-diphenylethylamine (0.018g) in isopropanol (0.75ml) were left to stand at room temperature for 16hrs. The solvent was removed, (3R)-(+)-3-aminopyrrolidine (0.1ml) and DMSO (0.05ml) added and the mixture heated in a sealed vial (eg Reactivial™) at 90°C for 27h. Purification by Autoprep HPLC followed by freeze-drying gave the title compound as a cream coloured solid (0.002g)
LC-MS System A Rt=4.27min *m*/*z* 583 (MH⁺)

### Example 19: (2R,3R,4S,5S)-2-{6-(2,2-Diphenyl-ethylamino)-2-[2-(1-methyl-1H-imidazol-4-yl)-ethylamino]-purin-9-yl}-5-(5-methyl-[1,3,4]thiadiazol-2-yl)-tetrahydro-furan-3,4-diol diformate

A solution of 1-methylhistamine bishydrochloride (0.06g, 0.30mmol) in methanol (1ml) was treated with sodium hydroxide (0.02g, 0.54mmol) and stirred at 20°C for 1h. The supernatant of the resultant mixture was added to a solution of Intermediate 8 0.04g, 0.06mmol) in DMSO (0.5ml) and methanol was removed by nitrogen flow. The solution was heated at 85°C in a sealed vial (eg Reactivial™) for 216h then allowed to cool. The crude reaction product was purified using Autoprep. HPLC to afford the title compound after freeze-drying, as a yellow solid (0.024g). LC/MS SYSTEM B Rₜ = 2.53 min; *m*/*z* 639 (MH⁺)

### Example 20: (2R,3R,4S,5S)-2-[6-(2,2-Diphenyl-ethylamino)-2-(1S-hydroxymethyl-2-phenyl-ethylamino)-purin-9-yl]-5-(5-methyl-[1,3,4]thiadiazol-2-yl)-tetrahydro-furan-3,4-diol formate

A solution of Intermediate 8 (0.04g, 0.06mmol) in DMSO (0.5ml) was treated with (S)-(-)-2-amino-3-phenyl-1-propanol (0.05g, 0.30mmol) and heated at 85°C in a sealed vial (eg Reactivial™) for 96h then allowed to cool. The crude reaction product was purified using Autoprep. HPLC to afford the title compound after freeze-drying, as a yellow solid (0.010g). LC/MS SYSTEM B Rₜ = 3.13 min; *m*/*z* 665 (MH⁺)

### Example 21: (2R,3R,4S,5S)-2-[6-(2,2-Diphenyl-ethylamino)-2-(1S-hydroxymethyl-2-phenyl-ethylamino)-purin-9-yl]-5-(5-ethyl-[1,3,4]oxadiazol-2-yl)-tetrahydro-furan-3,4-diol formate

Intermediate 21 (0.041g, 0.075mmol) and 3-(S)-(-)-2-amino-3-phenyl propanol (0.057g, 0.375mmol) were dissolved in DMSO (0.03ml) and diisopropylethylamine (0.03ml) then heated at 110° C for 2 days in a sealed vial (eg Reactivial™). Purification by Autoprep. HPLC afforded the title compound after freeze drying as a white solid (0.009g). LC/MS system A Rₜ = 4.58 min, *m*/*z* = 663 MH⁺. R2421/122/4

### Example 22: (2R,3R,4S,5S)-2-[6-(2,2-Diphenyl-ethylamino)-2-(2-piperidin-1-yl-ethylamino)-purin-9-yl]-5-(5-ethyl-[1,3,4]oxadiazol-2-yl)-tetrahydro-furan-3,4-diol formate

Intermediate 21 (0.041 g, 0.075mmol) and 2-piperidinoethylamine (0.053ml, 0.375mmol) were dissolved in DMSO (0.03ml) and diisopropylethylamine (0.03ml) then heated at 80-85° C for 29h in a sealed vial (eg Reactivial™), Purification by Autoprep. HPLC afforded the title compound after freeze drying as a white solid (0.004g). LC/MS system A Rₜ = 3.75min, *m*/*z* = 640 MH⁺.

### Example 23: (2R,3R,4S,5S)-2-[6-(2,2-Diphenyl-ethylamino)-2-(2-morpholin-4-yl-ethylamino)-purin-9-yl]-5-(5-ethyl-[1,3,4]oxadiazol-2-yl)-tetrahydro-furan-3,4-diol formate

Intermediate 21 (0.041g, 0.075mmol) and 4-(2-aminoethyl)morpholine (0.049ml, 0.375mmol) were dissolved in DMSO (0.03ml) and diisopropylethylamine (0.03ml) then heated at 80-85° C for 9h in a sealed vial (eg Reactivial™). Purification by Autoprep. HPLC afforded the title compound after freeze drying as a white solid (0.008g). LC/MS system A Rₜ = 3.64 min, *m*/*z* = 642 MH⁺.

### Example 24: (2R,3R,4S,5S)-2-[6-(2,2-Diphenyl-ethylamino)-2-(2-pyridin-2-yl-ethylamino)-purin-9-yl]-5-(5-ethyl-[1,3,4]oxadiazol-2-yl)-tetrahydro-furan-3,4-diol formate

Intermediate 21 (0.041g, 0.075mmol) and 2-(2-aminoethyl) pyridine (0.045ml, 0.375mmol) were dissolved in DMSO (0.03ml) and diisopropylethylamine (0.03ml) then heated at 80-85° C for 29h in a sealed vial (eg Reactivial™). Purification by Autoprep. HPLC afforded the title compound after freeze drying as a white solid (0.003g). LC/MS system A Rₜ = 3.97 min, *m*/*z* = 634 MH⁺.

### Example 25: (2R,3R,4S,5S)-2-[2-(trans-4-Amino-cyclohexylamino)-6-(2,2-diphenyl-ethylamino)-purin-9-yl]-5-(5-ethyl-[1,3,4]oxadiazol-2-yl)-tetrahydro-furan-3,4-diol formate

Intermediate 21 (0.041g, 0.075mmol) and *trans*-1,4-diaminocyclohexane (0.043g, 0.375mmol) were dissolved in DMSO (0.03ml) and diisopropylethylamine (0.03ml) then heated at 80-85° C for 29h in a sealed vial (eg Reactivial™). A further portion of *trans* -1,4-diaminocyclohexane (0.043g, 0.375mmol) was added and the mixture heated for a further 5h. Purification by Autoprep. HPLC afforded the title compound after freeze drying as a pink solid (0.011g). LC/MS system B Rₜ = 2.51 min, *m*/*z* = 626 MH⁺.

### Example 26: (2R,3R,4S,5S)-2-[6-(2,2-Diphenyl-ethylamino)-2-(pyrrolidin-3R-ylamino)-purin-9-yl]-5-(5-ethyl-[1,3,4]oxadiazol-2-yl)-tetrahydro-furan-3,4-diol formate

Intermediate 21 (0.041g, 0.075mmol) and (3R)-(+)-3-aminopyrrolidine (0.036ml, 0.375mmol) were dissolved in DMSO (0.03ml) and diisopropylethylamine (0.03ml) then heated at 80° C for 5h in a sealed vial (eg Reactivial™). Purification by Autoprep. HPLC afforded the title compound after freeze drying as a white solid (0.006g). LC/MS system A Rₜ = 3.65min, *m*/*z* = 598 MH⁺.

### Example 27: (2R,3R,4S,5R)-2-[6-(2,2-Diphenyl-ethylamino)-2-(2-pyridin-2-yl-ethylamino)-purin-9-yl]-5-(5-ethyl-4H-[1,2,4]triazol-3-yl)-tetrahydro-furan-3,4-diol formate

A mixture of Intermediate 11 (0.035g, 0.06mmol) in DMSO (0.04ml), and 2-(2-aminoethyl)pyridine (0.036ml, 0.3mmol) was heated at 90°C in a sealed vial (eg Reactivial™) for 48h. The resultant compound was dissolved up in TFA / water solution (9:1, 1ml). The solution was stirred for 1.5h. at room temperature, then the TFA blown off with nitrogen. Purification by Autoprep. HPLC afforded the title compound after freeze drying as a cream coloured solid (0.003g).
LC/MS SYSTEM A Rₜ = 3.99 min, *m*/*z* = 633 MH⁺

### Example 28: (2R,3R,4S,5S)-2-[6-(2,2-Diphenyl-ethylamino)-2-(2-piperidin-1-yl-ethylamino)-purin-9-yl]-5-[5-methyl-[1,3,4]thiadiazol-2-yl)-tetrahydro-furan-3,4-diol diformate

Intermediate 31 (0.04g, 0.06mmol) was dissolved in TFA (0.9ml) and water (0.2ml) at 0°C and stirred for 2h. at this temperature. The mixture was concentrated *in vacuo* and the residue purified by Autoprep. HPLC to afford the title compound after freeze-drying as an off-white solid (0.004g).
LC/MS SYSTEM B Rₜ = 2.56 min; *m*/*z* = 686 (MH⁺)

### Example 29: N-(2-{6-(2,2-Diphenyl-ethylamino)-9-[5R-(5-ethyl-[1,2,4]oxadiazol-3-yl)-3R,4S-dihydroxy-tetrahydro-furan-2R-yl]-9H-purin-2-ylamino}-ethyl)-guanidine diformate

A solution of the Intermediate 32 (0.02g, 0.035mmol) in ethanol / water (1:1), (0.5ml), was treated with imidazole (0,05g, 0.07mmol), and 1N-pyrazole-1-carboxamidine -monohydrochloride (0.01g, 0.07mmol), the heated at 60°C for 4 days. The solvent was removed by evaporation. The product was purified by Autoprep. HPLC to give the title compound after freeze drying as a white solid (0.005g). LC/MS system B Rₜ = 2.61min, *m*/*z* = 614 MH⁺.

### Example 30: (2R,3R,4S,5R)-2-[2-(trans-4-Amino-cyclohexylamino)-6-(2,2-diphenyl-ethylamino)-purin-9-yl]-5-(1-ethyl-1H-[1,2,4]triazol-3-yl)-tetrahydro-furan-3,4-diol diformate

Intermediate 24 (0.017g, 0.03mM) and *trans*-1,4-diaminocyclohexane (0.032g, 0.28mM) in anhydrous DMSO (0.5ml) in a sealed vial (e.g. Reacti-vial™) were heated at 90°C for 225 hrs. and then at 100°C for 91 hrs. The reaction mixture was diluted with acetonitrile and water (4ml, 1:1) containing 0.1% formic acid. and purified with using Autoprep. HPLC to afford the title compound after freeze drying as a brown coloured solid (0.005g). LC/MS system A Rₜ= 3.52 mins, *m*/*z* = 625 MH⁺

### Example 31: (2R,3R,4S,5R)-2-{6-(2,2-Diphenyl-ethylamino)-2-[2-(1-methyl-1H-imidazol-4-yl)-ethylamino]-purin-9-yl}-5-(1-ethyl-1H-[1,2,4]triazol-3-yl)-tetrahydro-furan-3,4-diol diformate

Example 31 was formed in an analogous manner to Example 30 using 1-methylhistamine (0.038g, 0.3mmol). in anhydrous DMSO (0.5ml) in a sealed vial (e.g. Reacti-vial™) were heated at 90°C for 225 hrs. Further methylhistamine (0.038g, 0.3mM) was added, heated at 100°C for 203 hrs. The reaction mixture was diluted to a volume of 4ml with a 1:1 mixture of acetonitrile and water containing 0.1% formic acid and purified with using Autoprep. HPLC to afford the title compound after freeze drying as a yellowish coloured solid (0.004g). LC/MS system A Rₜ = 3.58 mins, *m*/*z* = 636 MH⁺

### Example 32: (2R,3R,4S,5R)-2-[6-(2,2-Diphenyl-ethylamino)-2-(2-piperidin-1-yl-ethylamino)-purin-9-yl]-5-(1-ethyl-1H-[1,2,4]triazol-3-yl)-tetrahydro-furan-3,4-diol diformate

Intermediate 24 (0.017g, 0.03mM) and 2-piperidinoethylamine (0.038g, 0.30mM) in anhydrous DMSO (0.5ml) in a sealed vial (e.g. Reacti-vial™) were heated at 90°C for 110 h. The reaction mixture was diluted to a volume of 4ml with a 1:1 mixture of acetonitrile and water containing 0.1% formic acid. and purified with using Autoprep. HPLC to afford the title compound after freeze drying as an off-white solid (0.009g). LC/MS system A Rₜ = 3.63 mins, *m*/*z* = 639 MH⁺

### Example 33: (2R,3R,4S,5R)-2-[6-(2,2-Diphenyl-ethylamino)-2-(2-pyridin-2-yl-ethylamino)-purin-9-yl]-5-(1-ethyl-1H-[1,2,4]triazol-3-yl)-tetrahydro-furan-3,4-diol diformate

Example 33 was formed in an analogous manner to Example 32 using 2-(2-aminoethyl)pyridine (0.037g, 0.3mmol). to afford the title compound after freeze drying as an off-white solid (0.011g). LC/MS system A Rₜ = 3.81 mins, *m*/*z* = 633 MH⁺

### Example 34: (2R,3R,4S,5R)-2-[6-(2,2-Diphenyl-ethylamino)-2-(pyrrolidin-3R-ylamino)-purin-9-yl]-5-(1-ethyl-1H-[1,2,4]triazol-3-yl)-tetrahydro-furan-3,4-diol diformate

Example 34 was formed in an analogous manner to Example 33 using (3R)-(+)-3-aminopyrrolidine (0.038g, 0.3mmol). to afford the title compound after freeze drying as an off-white solid (0.012g). LC/MS system A Rₜ = 3.58 mins, *m*/*z* = 597 MH⁺

### Example 35: (2R,3R,4S,5R)-2-[6-(2,2-Diphenyl-ethylamino)-2-(1R-hydroxy-2-phenyl-ethylamino)-purin-9-yl]-5-(1-ethyl-1H-[1,2,4]triazol-3-yl)-tetrahydro-furan-3,4-diol formate

Example 35 was formed in an analogous manner to Example 30 using 3-(S)-(-)2-amino-3-phenyl propanol (0.045g, 0.3mmol) to afford the title compound after freeze drying as an off-white solid (0.007g). LC/MS system A Rₜ = 4.37 mins, *m*/*z* = 662 MH⁺

### Example 36: (2R,3R,4S,5S)-2-[2-(trans-4-Amino-cyclohexylamino)-6-(1-ethyl-propylamino)-purin-9-yl]-5-(5-cyclopropyl-[1,3,4]oxadiazol-2-yl)-tetrahydro-furan-3,4-diol diformate

A mixture of Intermediate 30 (0.05g, 0.11mmol) and *trans*-1,4-diamino cyclohexane (0.063g, 0.5mmol) in DMSO (0.3ml) in a sealed vial (e.g. Reactivial™) was heated with stirring at 90°C for 4d. The resultant crude product was purified by Autoprep. HPLC to afford the title compound after freeze-drying as a brown solid (0.005g). LC/MS SYSTEM C Rₜ = 2.12 mins, *m*/*z* = 528 MH⁺

### Example 37: (2S,3S,4R,5R)-2-(5-Cyclopropyl-[1,3,4]oxadiazol-2-yl)-5-{6-(1-ethyl-propylamino)-2-[2-(1-methyl-1H-imidazol-4-yl)-ethylamino]-purin-9-yl}-tetrahydro-furan-3,4-diol diformate

Example 37 was prepared in an analogous manner to Example 36 using 1-methylhistamine (0.07g, 0.55mmol; generated from the corresponding bishydrochloride by neutralisation with slight deficient of solid sodium hydroxide in methanol and evaporation of any volatile matters under a jet of nitrogen) at 90°C for 4d. The title compound was afforded after freeze drying as a pale brown solid (0.012g). LCMS SYSTEM C Rₜ = 2.16 mins, *m*/*z* = 539 MH⁺

### Example 38: (2S,3S,4R,5R)-2-(5-Cyclopropyl-[1,3,4]oxadiazol-2-yl)-5-[6-(1-ethyl-propylamino)-2-(2-piperidin-1-yl-ethylamino)-purin-9-yl]-tetrahydro-furan-3,4-diol diformate

Example 38 was prepared in an analogous manner to Example 36 using 2-piperidinoethylamine (0.078ml, 0.55mmol) at 90°C for 4 days. The title compound was afforded after freeze drying as a pale brown solid (0.007g). LCMS SYSTEM C Rₜ = 2.25 mins, *m*/*z* = 542 MH⁺

### Example 39: (2R,3R,4S,5S)-2-[2-Cyclopentylamino-6-(1-ethyl-propylamino)-purin-9-yl]-5-(5-cyclopropyl-[1,3,4]oxadiazol-2-yl)-tetrahydro-furan-3,4-diol diformate

Example 39 was prepared in an analogous manner to Example 36 using cyclopentylamine (0.055ml, 0.55mmol) at 90°C for 4 days. The title compound was afforded after freeze drying as a pale brown solid (0.015g).
LCMS SYSTEM C Rₜ = 2.94 mins, *m*/*z* = 499 MH⁺

### Example 40: (2S,3S,4R,5R)-2-(5-Cyclopropyl-[1,3,4]oxadiazol-2-yl)-5-[6-(1-ethyl-propylamino)-2-(pyrrolidin-3R-ylamino)-purin-9-yl]-tetrahydro-furan-3,4-diol diformate

Example 40 was prepared in an analogous manner to Example 36 using pyrrolidin-3R-ylamine (0.060ml, 0.55mmol) at 90°C for 4 days. The title compound was afforded after freeze drying as a pale brown solid (0.009g).
LCMS SYSTEM ARₜ = 3.24 mins, *m*/*z* = 500 MH⁺

### Example 41:(2R,3R,4S,5S)-2-[2-(2-Cyclohexyl-ethylamino)-6-(1-ethyl-propylamino)-purin-9-yl]-5-(5-cyclopropyl-[1,3,4]oxadiazol-2-yl)-tetrahydro-furan-3,4-diol diformate

Example 41 was prepared in an analogous manner to Example 36 using cyclohexyl-ethylamine (0.082ml, 0.55mmol) at 90°C for 4 days. The title compound was afforded after freeze drying as a pale brown solid (0.02g).
LCMS SYSTEM C Rₜ = 4.88 mins, *m*/*z* = 541 MH⁺

### Example 42: (2R,3R,4S,5S)-2-[6-(1-Ethyl-propylamino)-2-(1S-hydroxymethyl-2-methyl-propylamino)-purin-9-yl]-5-(5-cyclopropyl-[1,3,4]oxadiazol-2-yl)-tetrahydro-furan-3,4-diol formate

Example 42 was prepared in an analogous manner to Example 36 L-2-amino-3-methylbutanol (0.062ml, 0.55mmol) at 90°C for 4 days. The title compound was afforded after freeze drying as a pale brown solid(0.007g).
LCMS SYSTEM C Rₜ = 2.41 mins, *m*/*z* = 517 MH⁺

### Example 43: (2R,3R,4S,5R)-2-[6-(1-Ethyl-propylamino)-2-(2-piperidin-1-yl-ethylamino)-purin-9-yl]-5-(5-ethylisoxazol-3-yl)tetrahydrofuran-3,4-diol diformate

A mixture of Intermediate 39 (30mg) and 2-piperidinoethylamine (0.043ml) was heated at 90°C for 24h in dimethylsulphoxide (0.5ml). Heating was continued for 96h at 90°C. Purification by preparative HPLC (gradient profile 5-95% (ii) over 18.25min) gave the title compound as a brown gum (4 mg).
LC/MS SYSTEM C: Rₜ = 2.50 mins, *m*/*z* = 529 MH⁺

The compounds of the Examples were tested in screen (1) (agonist activity against receptor sub-types) and the results obtained were as follows:

| Example No. | A2a | A3 | A1 |
|---|---|---|---|
| 1 | 14.6 | >1088 | >8325 |
| 2 | 2.46 | >1087 | >=7728 |
| 3 | 3.54 | >698 | >9058 |
| 4 | 5.1 | >1052 | 4686 |
| 5 | 1 | >319 | >=5194 |
| 6 | 12.3 | >183 | 6739 |
| 7 | 2.94 | >183 | 5327 |
| 8 | 19.4 | >183 | >10735 |
| 9 | 3.25 | >147 | >6032 |
| 10 | 16.85 | >326 | 1453.5 |
| 11 | 17.97 | >257 | 2202 |
| 12 | 4.77 | >194 | >8841 |
| 13 | 1.29 | >194 | 6620 |
| 14 | 12.86 | >190 | >=4762 |
| 15 | 13.62 | >190 | >=8649 |
| 16 | 5.75 | >257 | 4514.96 |
| 17 | 5.45 | >518 | 538 |
| 18 | 18.9 | >223 | 5515 |
| 19 | 4.05 | >293 | 3172 |
| 20 | 17.7 | >470 | 2625 |
| 21 | 3.04 | >173 | 568.06 |
| 22 | 12.28 | >180 | 101.96 |
| 23 | 6.16 | >180 | 101.96 |
| 24 | 6.04 | >175 | 390.97 |
| 25 | 4.81 | >136 | 398.28 |
| 26 | 5.57 | >162 | 432 |
| 27 | 21.8 | >183 | 135.9 |
| 28 | 37.3 | >245 | 3371 |
| 29 | 30.7 | >284 | >2147 |
| 30 | 13.27 | >206 | 2948.1 |
| 31 | 8.79 | >206 | 1753.5 |
| 32 | 11.85 | >206 | 1217.4 |
| 33 | 34.25 | >206 | 4999.7 |
| 34 | 10.97 | >231 | 1980.8 |
| 35 | 6.33 | >240 | 5261.1 |
| 36 | 26.3 | >173 | 1105.6 |
| 37 | 6.39 | >173 | 581.9 |
| 38 | 45.64 | >173 | 365.6 |
| 39 | 129.5 | >173 | >=1067 |
| 40 | 56.86 | >173 | 5084.2 |
| 41 | 74.29 | >249 | 1921.5 |
| 42 | 41.04 | >87 | 306.9 |
| 43 | 3.25 | >1124 | 21.82 |

Values given in the Table are EC₅₀ values as a ratio of that of NECA.

### ABBREVIATIONS

- TMS: trimethylsilyl
- TFA: trifluoroacetic acid
- DMF: N,N-dimethylformamide
- NECA: N-ethylcarboxamideadenosine
- DMAP: 4-dimethylaminopyridine
- TEMPO: 2,2,6,6-tetramethyl-1-piperidinyloxy, free radical
- TMSOTf: Trimethylsilyltrifluoromethylsulphonate
- DBU: 1,8-diazabicyclo[5.4.0]undec-7-ene
- BSA: bistrimethylsilylacetamide
- DCM: dichloromethane
- DAST: diethylaminosulphur trifluoride
- Ph: phenyl
- CDI: carbonyldiimidazole
- EEDQ: 2-ethoxy-1-ethoxycarbonyl-1,2 dihydroquinone
- NSAID: non-steroidal antiinflammatory drug
- HBTU: 2-(IH-Benzotriazole-1-yl)-1,1,3,3-tetramethyluronium hexafluorophosphate
- DMSO: dimethylsulphoxide
- DEAD: diethylazocarboxylate

## Claims

1. A compound of formula (I): 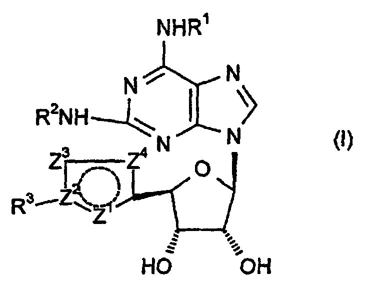 wherein R¹ and R² independently represent a group selected from:
(i) C₃₋₈cycloalkyl-;
(ii) hydrogen;
(iii) aryl₂CHCH₂-;
(iv) C₃₋₈cycloalkylC₁₋₆alkyl-;
(v) C₁₋₆alkyl-;
(vi) arylC₁₋₆alkyl-;
(vii) R⁴R⁵N-C₁₋₆alkyl-;
(viii) C₁₋₆alkyl-CH(CH₂OH)-;
(ix) arylC₁₋₅alkyl-CH(CH₂OH)-;
(x) arylC₁₋₅alkyl-C(CH₂OH)₂-;
(xi) C₃₋₈cycloalkyl independently substituted by one or more (e.g. 1, 2 or 3) -(CH₂)ₚR⁶ groups;
(xii) H₂NC(=NH)NHC₁₋₆alkyl-;
(xiii) a group of formula 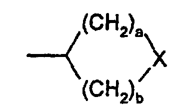 or such a group in which one methylene carbon atom adjacent to X, or both if such exist, is substituted by methyl;
(xiv) -C₁₋₆alkyl-OH;
(xv) -C₁₋₈haloalkyl;
(xvi) a group of formula 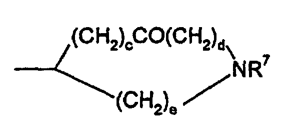
(xvii) aryl; and
(xviii) -(CH₂)_{f}SO₂NH_{g}(C₁₋₄alkyl-)_{2-g} or -(CH₂)_{f}SO₂NH_{g}(arylC₁₋₄alkyl-)_{2-g} where f is 2 or 3 and g is an integer 0 to 2;
Z² represents C or N;
Z¹, Z³ and Z⁴ together with Z² and the carbon atom form a 5-membered heterocyclic aromatic ring;
R³ represents C₁₋₃alkyl or cyclopropyl, save that where Z² represents C, R³ may also represent CH₂OH;
R⁴ and R⁵ independently represent hydrogen, C₁₋₆alkyl, aryl, arylC₁₋₆alkyl- or NR⁴R⁵ together may represent pyridinyl, pyrrolidinyl, piperidinyl, morpholinyl, azetidinyl, azepinyl, piperazinyl or N-C₁₋₆alkylpiperazinyl;
R⁶ represents OH, NH₂, NHCOCH₃ or halogen;
R⁷ represents hydrogen, C₁₋₆alkyl, -C₁₋₆alkylaryl or -COC₁₋₆alkyl;
X represents NR⁷, O, S, SO or SO₂;
p represents 0 or 1;
a and b independently represent an integer 0 to 4 provided that a + b is in the range 3 to 5;
c, d and e independently represent an integer 0 to 3 provided that c + d + e is in the range 2 to 3;
with the proviso that the moiety 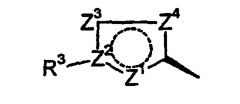 does not represent one of the following groups: 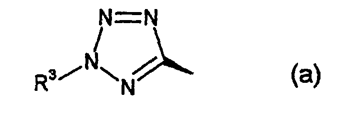 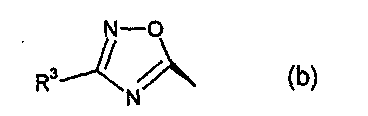 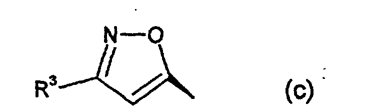 and any salts and solvates thereof.

2. A compound of formula (I) according to claim 1 wherein R¹ and R² do not both represent hydrogen.

3. A compound according to claim 1 or claim 2 wherein R¹ represents aryl₂CHCH₂- C₁₋₈alkyl-, hydrogen or arylC₁₋₆alkyl-.

4. A compound according to any one of claims 1 to 3 wherein R¹ represents Ph₂CHCH₂-.

5. A compound according to any one of claims 1 to 4 wherein R² represents ethyl-piperidin-1-yl, PhCH₂CH(CH₂OH)-, -CH(CH₂OH)(CH(CH₃)₂, trans-4-amino-cyclohexyl, 2-(1-methyl-1H-imidazoyl-4-yl)CH₂CH₂-, ethylmorpholin-1-yl, pyrrolidin-3-yl, ethyl-pyridin-2-yl, H₂NC(=NH)NH(CH₂)₂-, cyclopentyl or ethylcyclohexyl.

6. A compound according to any one of claims 1 to 5 wherein R² represents 2-(1-C₁₋₃alkyl-1H-imidazoyl-4-yl)CH₂CH₂-.

7. A compound according to any one of claims 1 to 6 wherein Z² represents C.

8. A compound according to any one of claims 1 to 7 wherein Z⁴ represents N.

9. A compound according to any one of claims 1 to 8 wherein R³ represents methyl, ethyl, n-propyl, isopropyl, cyclopropyl or CH₂OH (when Z² represents C).

10. A compound according to any one of claims 1 to 9 wherein R³ represents methyl, ethyl or cyclopropyl.

11. A compound according to any one of claims 1 to 10 wherein R³ represents ethyl.

12. A compound according to any one of claims 1 to 11 wherein R⁴ and R⁵ independently represent hydrogen or aryl or NR⁴R⁵ together may represent pyrrolidinyl, piperidinyl, morpholinyl, azetidinyl, azepinyl, piperazinyl or N-methylpiperazinyl.

13. A compound according to any one of claims 1 to 12 wherein R⁶ represents OH or NH₂.

14. A compound according to any one of claims 1 to 13 wherein X represents NR⁷, O, S or SO₂.

15. A compound according to any one of claims 1 to 14 wherein the moiety 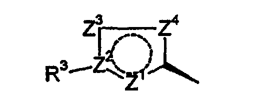 represents one of the following groups:

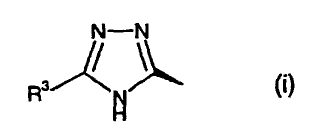

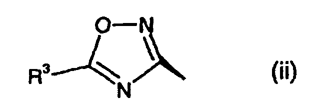

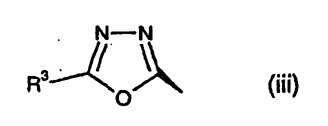

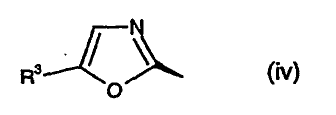

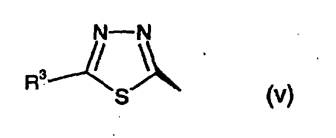

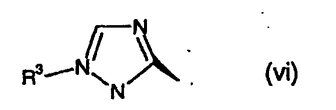

16. A compound according to any one of claims 1 to 15 wherein the moiety 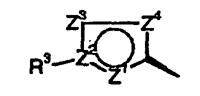 represents (i), (ii), (iii) and (vi) as defined in claim 15.

17. A compound according to any one of claims 1 to 16 wherein the moiety 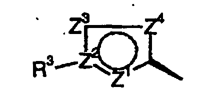 represents (i) as defined in claim 15.

18. A compound of formula (I) which is
(2R,3R,4S,5R)-2-[6-(2,2-Diphenyl-ethylamino)-2-(2-pyrrolidin-1-yl-ethylamino)-purin-9-yl]-5-(5-methyl-4H-[1,2,4]triazol-3-yl)-tetrahydro-furan-3,4-diol;
(2R,3R,4S,5R)-2-[6-(2,2-Diphenyl-ethylamino)-2-(1 S-hydroxymethyl-2-phenylethylamino)-purin-9-yl]-5-(5-methyl-4H-[1,2,4]triazol-3-yl)-tetrahydro-furan-3,4-diol;
(2R,3R,4S,5R)-2-{6-(2,2-Diphenyl-ethylamino)-2-[2-(1-methyl-1H-imidazol-4-yl)-ethylamino]-purin-9-yl}-5-(5-methyl-4H-[1,2,4]triazol-3-yl)-tetrahydro-furan-3,4-diol;
(2R,3R,4S,5R)-2-[2-(trans-4-Amino-cyclohexylamino)-6-(2,2-diphenyl-ethyl amino)-purin-9-yl]-5-(5-methyl-4H-[1,2,4]triazol-3-yl)-tetrahydro-furan-3,4-diol;
(2R,3R,4S,5R)-2-[6-(2,2-Diphenyl-ethylamino)-2-(1S-hydroxymethyl-2-phenylethylamino)-purin-9-yl]-5-(5-ethyl-4H-[1,2,4]triazol-3-yl)-tetrahydro-furan-3,4-diol;
(2R,3R,4S,5R)-2-[2-(trans-4-Amino-cyclohexylamino)-6-(2,2-diphenylethylamino)-purin-9-yl]-5-(5-ethyl-4H-[1,2,4]triazol-3-yl)-tetrahydro-furan-3,4-diol;
(2R,3R,4S,5R)-2-[6-(2,2-Diphenyl-ethylamino)-2-(1S-hydroxymethyl-2-methylpropylamino)-purin-9-yl]-5-(5-ethyl-4H-[1,2,4]triazol-3-yl)-tetrahydro-furan-3,4-diol;
(2R,3R,4S,5R)-2-[6-(2,2-Diphenyl-ethylamino)-2-(2-morpholin-4-yl-ethylamino)-purin-9-yl]-5-(5-ethyl-4H-[1,2,4]triazol-3-yl)-tetrahydro-furan-3,4-diol;
(2R,3R,4S,5R)-2-[6-(2,2-Diphenyl-ethylamino)-2-(pyrrolidin-3R-ylamino)-purin-9-yl]-5-(5-ethyl-4H-[1,2,4]triazol-3-yl)-tetrahydro-furan-3,4-diol;
(2R,3R,4S,5R)-2-[2-(trans-4-Amino-cyclohexylamino)-6-(2,2-diphenyl-ethyl amino)-purin-9-yl]-5-(5-isopropyl-4H-[1,2,4]triazol-3-yl)-tetrahydro-furan-3,4-diol;
(2R,3R,4S,5R)-2-[6-(2,2-Diphenyl-ethylamino)-2-(1S-hydroxymethyl-2-phenylethylamino)-purin-9-yl]-5-(5-isopropyl-4H-[1,2,4]triazol-3-yl)-tetrahydro-furan-3,4-diol;
(2R,3R,4S,5R)-2-[2-(trans-4-Amino-cyclohexylamino)-6-(2,2-diphenylethylamino)-purin-9-yl]-5-(5-ethyl-[1,2,4]oxadiazol-3-yl)-tetrahydro-furan-3,4-diol;
(2R,3R,4S,5R)-2-{6-(2,2-Diphenyl-ethylamino)-2-[2-(1-methyl-1H-imidazol-4-yl)-ethylamino]-purin-9-yl}-5-(5-ethyl-[1,2,4]oxadiazol-3-yl)-tetrahydro-furan-3,4-diol;
(2R,3R,4S,5R)-2-[6-(2,2-Diphenyl-ethylamino)-2-(2-piperidin-1-yl-ethylamino)-purin-9-yl]-5-(5-ethyl-[1,2,4]oxadiazol-3-yl)-tetrahydro-furan-3,4-diol;
(2R,3R,4S,5R)-2-[6-(2,2-Diphenyl-ethylamino)-2-(2-morpholin-4-yl-ethylamino)-purin-9-yl]-5-(5-ethyl-[1,2,4]oxadiazol-3-yl)-tetrahydro-furan-3,4-diol; (2R,3R,4S,5R)-2-[6-(2,2-Diphenyl-ethylamino)-2-(1S-hydroxymethyl-2-phenylethylamino)-purin-9-yl]-5-(5-ethyl-[1,2,4]oxadiazol-3-yl)-tetrahydro-furan-3,4-diol;
(2R,3R,4S,5S)-2-[6-(2-Cyclohexyl-ethylamino)-2-(1S-hydroxymethyl-2-phenylethylamino)-purin-9-yl]-5-(5-methyl-oxazol-2-yl)-tetrahydro-furan-3,4-diol;
(2R,3R,4S,5S)-2-[6-(2,2-biphenyl-ethylamino)-2-(pyrrolidin-3R-ylamino)-purin-9-yl]-5-(5-methyl-oxazol-2-yl)-tetrahydro-furan-3,4-diol;
(2R,3R,4S,5S)-2-{6-(2,2-Diphenyl-ethylamino)-2-[2-(1-methyl-1H-imidazol-4-yl)-ethylamino]-purin-9-yl}-5-(5-methyl-[1,3,4]thiadiazol-2-yl)-tetrahydro-furan-3,4-diol;
(2R,3R,4S,5S)-2-[6-(2,2-Diphenyl-ethylamino)-2-(1S-hydroxymethyl-2-phenylethylamino)-purin-9-yl]-5-(5-methyl-[1,3,4]thiadiazol-2-yl)-tetrahydro-furan-3,4-diol;
(2R,3R,4S,5S)-2-[6-(2,2-Diphenyl-ethylamino)-2-(1S-hydroxymethyl-2-phenylethylamino)-purin-9-yl]-5-(5-ethyl-[1,3,4]oxadiazol-2-yl)-tetrahydro-furan-3,4-diol;
(2R,3R,4S,5S)-2-[6-(2,2-Diphenyl-ethylamino)-2-(2-piperidin-1-yl-ethylamino)-purin-9-yl]-5-(5-ethyl-[1,3,4]oxadiazol-2-yl)-tetrahydro-furan-3,4-diol;
(2R,3R,4S,5S)-2-[6-(2,2-Diphenyl-ethylamino)-2-(2-morpholin-4-yl-ethylamino)-purin-9-yl]-5-(5-ethyl-[1,3,4]oxadiazol-2-yl)-tetrahydro-furan-3,4-diol;
(2R,3R,4S,5S)-2-[6-(2,2-Diphenyl-ethylamino)-2-(2-pyridin-2-yl-ethylamino)-purin-9-yl]-5-(5-ethyl-[1,3,4]oxadiazol-2-yl)-tetrahydro-furan-3,4-diol;
(2R,3R,4S,5S)-2-[2-(trans-4-Amino-cyclohexylamino)-6-(2,2-diphenylethylamino)-purin-9-yl]-5-(5-ethyl-[1,3,4]oxadiazol-2-yl)-tetrahydro-furan-3,4-diol;
(2R,3R,4S,5S)-2-[6-(2,2-Diphenyl-ethylamino)-2-(pyrrolidin-3R-ylamino)-purin-9-yl]-5-(5-ethyl-[1,3,4]oxadiazol-2-yl)-tetrahydro-furan-3,4-diol;
(2R,3R,4S,5R)-2-[6-(2,2-Diphenyl-ethylaminp)-2-(2-pyridin-2-yl-ethylamino)-purin-9-yl]-5-(5-ethyl-4H-[1,2,4]triazol-3-yl)-tetrahydro-furan-3,4-diol;
(2R,3R,4S,5S)-2-[6-(2,2-Diphenyl-ethylamino)-2-(2-piperidin-1-yl-ethylamino)-purin-9-yl]-5-(5-methyl-[1,3,4]thiadiazol-2-yl)-tetrahydro-furan-3,4-diol;
N-(2-{6-(2,2-Diphenyl-ethylamino)-9-[5R-(5-ethyl-[1,2,4]oxadiazol-3-yl)-3R,4S-dihydroxy-tetrahydro-furan-2R-yl]-9H-purin-2-ylamino}-ethyl)-guanidine;
(2R,3R,4S,5R)-2-[2-(trans-4-Amino-cyclohexylamino)-6-(2,2-diphenylethylamino)-purin-9-yl]-5-(1-ethyl-1H-[1,2,4]triazol-3-yl)-tetrahydro-furan-3,4-diol;
(2R,3R,4S,5R)-2-{6-(2,2-Diphenyl-ethylamino)-2-[2-(1-methyl-1H-imidazol-4-yl)-ethylamino]-purin-9-yl}-5-(1-ethyl-1H-[1,2,4]triazol-3-yl)-tetrahydro-furan-3,4-diol;
(2R,3R,4S,5R)-2-[6-(2,2-Diphenyl-ethylamino)-2-(2-piperidin-1-yl-ethylamino)-purin-9-yl]-5-(1-ethyl-1H-[1,2,4]triazol-3-yl)-tetrahydro-furan-3,4-diol;
(2R,3R,4S,5R)-2-[6-(2,2-Diphenyl-ethylamino)-2-(2-pyridin-2-yl-ethylamino)-purin-9-yl]-5-(1-ethyl-1H-[1,2,4]triazol-3-yl)-tetrahydro-furan-3,4-diol;
(2R,3R,4S,5R)-2-[6-(2,2-Diphenyl-ethylamino)-2-(pyrrolidin-3R-ylamino)-purin-9-yl]-5-(1-ethyl-1H-[1,2,4]triazol-3-yl)-tetrahydro-furan-3,4-diol;
(2R,3R,4S,5R)-2-[6-(2,2-Diphenyl-ethylamino)-2-(1R-hydroxy-2-phenylethylamino)-purin-9-yl]-5-(1-ethyl-1H-[1,2,4]triazol-3-yl)-tetrahydro-furan-3,4-diol;
(2R,3R,4S,5S)-2-[2-(trans-4-Amino-cyclohexylamino)-6-(1-ethyl-propylamino)-purin-9-yl]-5-(5-cyclopropyl-[1,3,4]oxadiazol-2-yl)-tetrahydro-furan-3,4-diol;
(2S,3S,4R,5R)-2-(5-Cyclopropyl-[1,3,4]oxadiazol-2-yl)-5-{6-(1-ethyl-propylamino) -2-[2-(1-methyl-1H-imidazol-4-yl)-ethylamino]-purin-9-yl}-tetrahydro-furan-3,4-diol;
(2S,3S,4R,5R)-2-(5-Cyclopropyl-[1,3,4]oxadiazol-2-yl)-5-[6-(1-ethyl-propyl amino)-2-(2-piperidin-1-yl-ethylamino)-purin-9-yl]-tetrahydro-furan-3,4-diol;
(2R,3R,4S,5S)-2-[2-Cyclopentylamino-6-(1-ethyl-propylamino)-purin-9-yl]-5-(5-cyclopropyl-[1,3,4]oxadiazol-2-yl)-tetrahydro-furan-3,4-diol;
(2S,3S,4R,5R)-2-(5-Cyclopropyl-[1,3,4]oxadiazol-2-yl)-5-[6-(1-ethylpropylamino)-2-(pyrrolidin-3R-ylamino)-purin-9-yl]-tetrahydro-furan-3,4-diol;
(2R,3R,4S,5S)-2-[2-(2-Cyclohexyl-ethylamino)-6-(1-ethyl-propylamino)-purin-9-yl]-5-(5-cyclopropyl-[1,3,4]oxadiazol-2-yl)-tetrahydro-furan-3,4-diol;
(2R,3R,4S,5S)-2-[6-(1-Ethyl-propylamino)-2-(1S-hydroxymethyl-2-methyl-propyl amino)-purin-9-yl]-5-(5-cyclopropyl-[1,3,4]oxadiazol-2-yl)-tetrahydro-furan-3,4-diol;
or a salt or solvate of any one thereof.

19. A compound of formula (I) which is
(2R,3R,4S,5R)-2-[6-(1-Ethyl-propylamino)-2-(2-piperidin-1-yl-ethylamino)-purin-9-yl]-5-(5-ethylisoxazol-3-yl)tetrahydrofuran-3,4-diol or a salt or solvate thereof.

20. A pharmaceutical composition comprising a compound of formula (I) as defined in any one of claims 1 to 19 or a pharmaceutically acceptable salt or solvate thereof in admixture with one or more pharmaceutically acceptable diluents or carriers.

21. A compound of formula (I) as defined in any one of claims 1 to 19 or a pharmaceutically acceptable salt or solvate thereof for use as a pharmaceutical.

22. Use of a compound of formula (I) as defined in any one of claims 1 to 19 or a pharmaceutically acceptable salt or solvate thereof in the manufacture of a medicament for the treatment of inflammatory diseases, eg asthma or chronic obstructive pulmonary disease (COPD).

23. A process for preparation of a compound of formula (I) as defined in any one of claims 1 to 19 which comprises reacting a compound of formula (II) 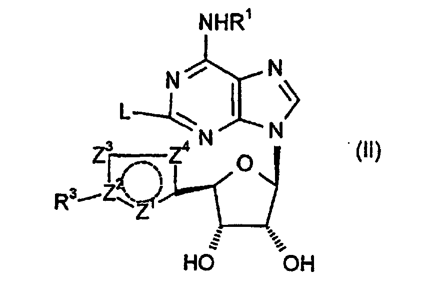 wherein L represents a leaving group or a protected derivative thereof with a compound of formula R²NH₂ or a protected derivative thereof, wherein R¹, R², R³, Z¹, Z², Z³ and Z⁴ are as defined in any one of claims 1 to 19.

24. A compound of formula (II) 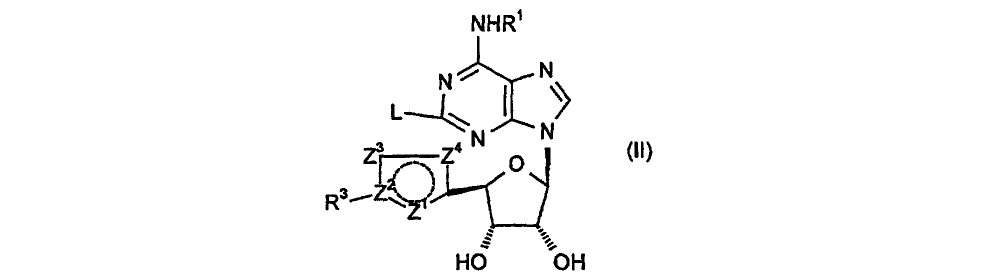 wherein L represents a leaving group or a protected derivative thereof and R¹, R³, Z¹, Z², Z³ and Z⁴ are as defined in any one of claims 1 to 19.

25. A compound of formula (III) 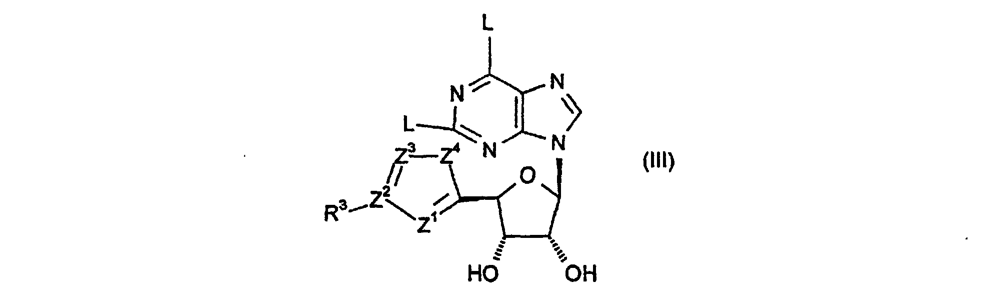 wherein L represents a leaving group or a protected derivative thereof and R³, Z¹, Z², Z³ and Z⁴ are as defined in any one of claims 1 to 19.

26. A compound of formula (IV) 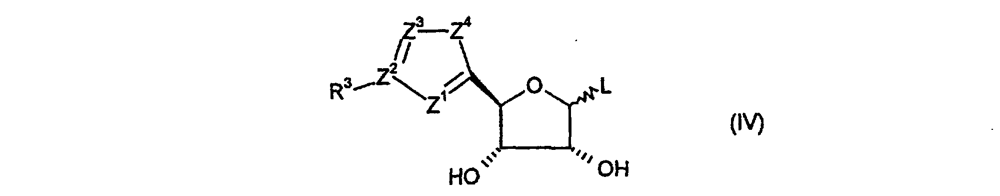 wherein L represents a leaving group or a protected derivative thereof and R³, Z¹, Z², Z³ and Z⁴ are as defined in any one of claims 1 to 19.

27. A compound of formula (V) 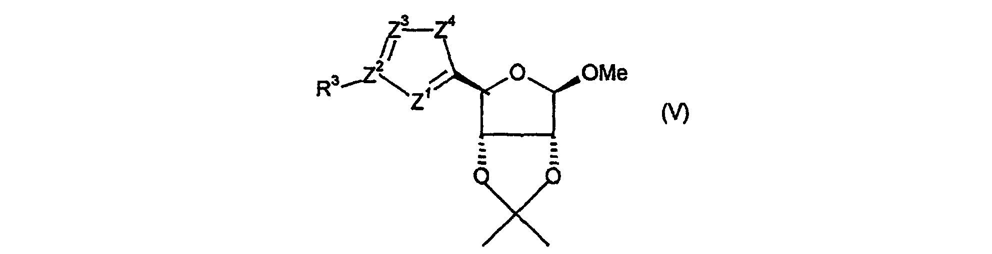 wherein R³, Z¹, Z², Z³ and Z⁴ are as defined in any one of claims 1 to 19.

28. A compound of formula (VI) 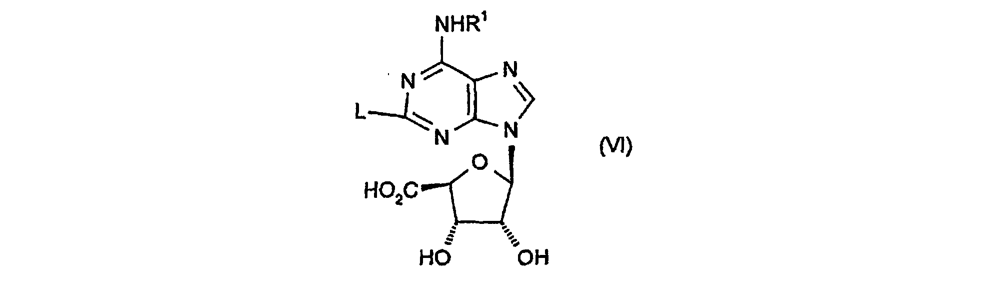 wherein L represents a leaving group or a protected derivative thereof and R¹ is as defined in any one of claims 1 to 19, with the proviso that when R¹ represents Ph₂CHCH₂, L does not represent chlorine.

## Patentansprüche

1. Verbindung der Formel (I): 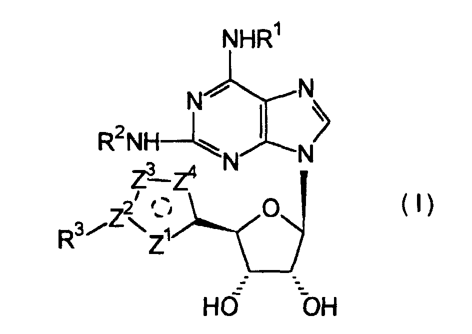 worin R¹ und R² unabhängig eine Gruppe darstellen, die aus den folgenden ausgewählt ist:
(i) C₃₋₈-Cycloalkyl-;
(ii) Wasserstoff;
(iii) Aryl₂CHCH₂-;
(iv) C₃₋₈-Cycloalkyl-C₁₋₆-alkyl-;
(v) C₁₋₈-Alkyl-;
(vi) Aryl-C₁₋₆-alkyl-;
(vii) R⁴R⁵N-C₁₋₆-Alkyl-;
(viii) C₁₋₆-Alkyl-CH(CH₂OH)-;
(ix) Aryl-C₁₋₅-alkyl-CH(CH₂OH)-;
(x) Aryl-C₁₋₅-alkyl-C(CH₂OH)₂-;
(xi) C₃₋₈-Cycloalkyl, das unabhängig mit einer oder mehreren (z.B. 1, 2 oder 3) -(CH₂)ₚR⁶-Gruppen substituiert ist;
(xii) H₂NC(=NH)NHC₁₋₆-Alkyl-;
(xiii) eine Gruppe der Formel 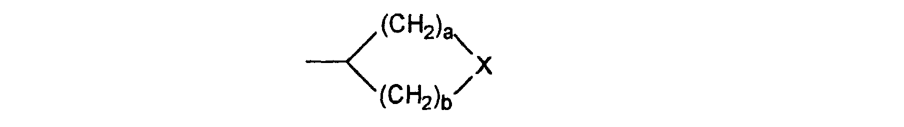 oder eine solche Gruppe, in der ein zu X benachbartes Methylen-Kohlenstoffatom oder beide, falls solche existieren, mit Methyl substituiert ist;
(xiv) -C₁₋₆-Alkyl-OH;
(xv) -C₁₋₈-Halogenalkyl;
(xvi) eine Gruppe der Formel 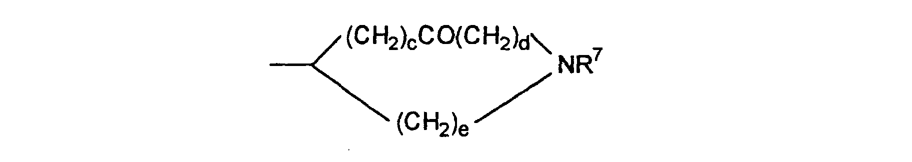
(xvii) Aryl; und
(xviii) -(CH₂)_{f}SO₂NH_{g}(C₁₋₄-Alkyl-)_{2-g} oder -(CH₂)_{f}SO₂NH_{g}(Aryl-C₁₋₄-alkyl-)_{2-g}, worin f 2 oder 3 ist und g eine ganze Zahl von 0 bis 2 ist;
Z² C oder N darstellt;
Z¹, Z³ und Z⁴ zusammen mit Z² und dem Kohlenstoffatom einen 5-gliedrigen heterocyclischen, aromatischen Ring bilden;
R³ C₁₋₃-Alkyl oder Cyclopropyl darstellt, ausser dass dann, wenn Z³ C darstellt, R³ ebenfalls CH₂OH darstellen kann;
R⁴ und R⁵ unabhängig Wasserstoff, C₁₋₆-Alkyl, Aryl oder Aryl-C₁₋₆-alkyl- darstellen oder NR⁴R⁵ zusammen Pyridinyl, Pyrrolidinyl, Piperidinyl, Morpholinyl, Azetidinyl, Azepinyl, Piperazinyl oder N-C₁₋₆-Alkylpiperazinyl darstellen kann;
R⁶ OH, NH₂, NHCOCH₃ oder Halogen darstellt;
R⁷ Wasserstoff, C₁₋₆-Alkyl, -C₁₋₆-Alkylaryl oder -COC₁₋₆-Alkyl darstellt;
X NR⁷, O, S, SO oder SO₂ darstellt;
p 0 oder 1 darstellt;
a und b unabhängig eine ganze Zahl von 0 bis 4 darstellen, mit der Massgabe, dass a + b im Bereich von 3 bis 5 ist;
c, d und e unabhängig eine ganze Zahl von 0 bis 3 darstellen, mit der Massgabe, dass c + d + e im Bereich von 2 bis 3 ist;
mit der Massgabe, dass die Einheit 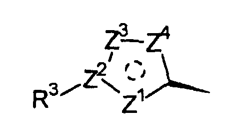 nicht eine der folgenden Gruppen darstellt: 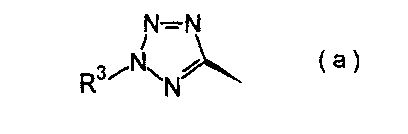 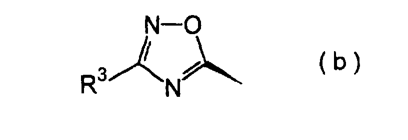 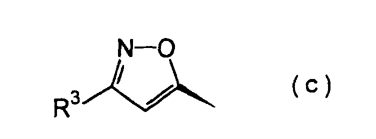 und beliebige Salze und Solvate davon.

2. Verbindung der Formel (I) gemäss Anspruch 1, worin R¹ und R² nicht beide Wasserstoff darstellen.

3. Verbindung gemäss Anspruch 1 oder 2, worin R¹ Aryl₂CHCH₂-C₁₋₈-alkyl-, Wasserstoff oder Aryl-C₁₋₆alkyl darstellt.

4. Verbindung gemäss einem der Ansprüche 1 bis 3, worin R¹ Ph₂CHCH₂- darstellt.

5. Verbindung gemäss einem der Ansprüche 1 bis 4, worin R² Ethyl-piperidin-1-yl, PhCH₂CH(CH₂OH)-, -CH(CH₂OH)(CH(CH₃)₂), trans-4-Amino-cyclohexyl, 2-(1-Methyl-1H-imidazolyl-4-yl)CH₂CH₂-, Ethyl-morpholin-1-yl, Pyrrolidin-3-yl, Ethyl-pyridin-2-yl, H₂NC(=NH)NH(CH₂)₂-, Cyclopentyl oder Ethylcyclohexyl darstellt.

6. Verbindung gemäss einem der Ansprüche 1 bis 5, worin R² 2-(1-C₁₋₃-Alkyl-1H-imidazolyl-4-yl)CH₂CH₂darstellt.

7. Verbindung gemäss einem der Ansprüche 1 bis 6, worin Z² C darstellt.

8. Verbindung gemäss einem der Ansprüche 1 bis 7, worin Z⁴ N darstellt.

9. Verbindung gemäss einem der Ansprüche 1 bis 8, worin R³ Methyl, Ethyl, n-Propyl, Isopropyl, Cyclopropyl oder CH₂OH (wenn Z² C darstellt) darstellt.

10. Verbindung gemäss einem der Ansprüche 1 bis 9, worin R³ Methyl, Ethyl oder Cyclopropyl darstellt.

11. Verbindung gemäss einem der Ansprüche 1 bis 10, worin R³ Ethyl darstellt.

12. Verbindung gemäss einem der Ansprüche 1 bis 11, worin R⁴ und R⁵ unabhängig Wasserstoff oder Aryl darstellen oder NR⁴R⁵ zusammen Pyrrolidinyl, Piperidinyl, Morpholinyl, Azetidinyl, Azepinyl, Piperazinyl oder N-Methylpiperazinyl darstellen kann.

13. Verbindung gemäss einem der Ansprüche 1 bis 12, worin R⁶ OH oder NH₂ darstellt.

14. Verbindung gemäss einem der Ansprüche 1 bis 13, worin X NR⁷, O, S oder SO₂ darstellt.

15. Verbindung gemäss einem der Ansprüche 1 bis 14, worin die Einheit 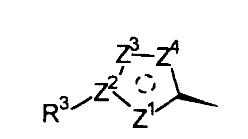 eine der folgenden Gruppen darstellt:

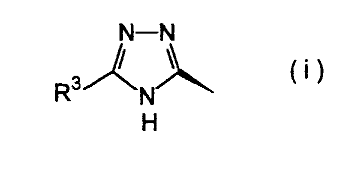

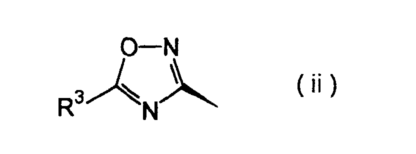

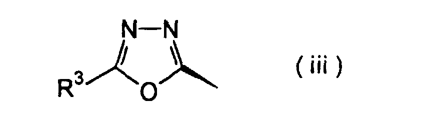

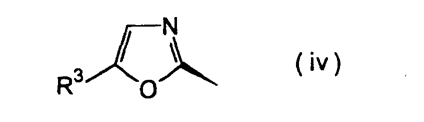

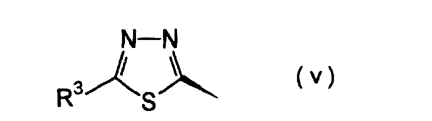

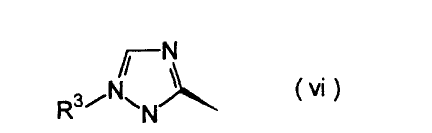

16. Verbindung gemäss einem der Ansprüche 1 bis 15, worin die Einheit 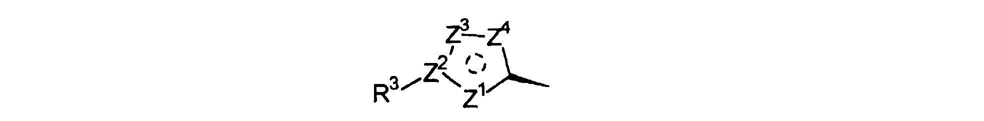 (i), (ii), (iii) und (iv) wie in Anspruch 15 definiert darstellt.

17. Verbindung gemäss einem der Ansprüche 1 bis 16, worin die Einheit 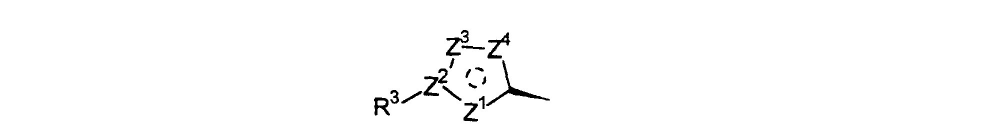
(i) wie in Anspruch 15 definiert darstellt.

18. Verbindung der Formel (I), die:
(2R,3R,4S,5R)-2-[6-(2,2-Diphenyl-ethylamino)-2-(2-pyrrolidin-1-yl-ethylamino)-purin-9-yl]-5-(5-methyl-4H-[1,2,4]triazol-3-yl)-tetrahydro-furan-3,4-diol;
(2R,3R,4S,5R)-2-[6-(2,2-Diphenyl-ethylamino)-2-(1Shydroxymethyl-2-phenyl-ethylamino)-purin-9-yl]-5-(5-methyl-4H-[1,2,4]triazol-3-yl)-tetrahydro-furan-3,4-diol;
(2R,3R,4S,5R)-2-{6-(2,2-Diphenyl-ethylamino)-2-[2-(1-methyl-1H-imidazol-4-yl)-ethylamino]-purin-9-yl}-5-(5-methyl-4-H-[1,2,4]triazol-3-yl)-tetrahydro-furan-3,4-diol;
(2R,3R,4S,5R)-2-[2-(trans-4-Amino-cyclohexylamino)-6-(2,2-diphenyl-ethylamino)-purin-9-yl]-5-(5-methyl-4H-[1,2,4]triazol-3-yl)-tetrahydro-furan-3,4-diol;
(2R,3R,4S,5R)-2-[6-(2,2-Diphenyl-ethylamino)-2-(1Shydroxymethyl-2-phenyl-ethylamino)-purin-9-yl]-5-(5-ethyl-4H-[1,2,4]triazol-3-yl)-tetrahydro-furan-3,4-diol;
(2R,3R,4S,5R)-2-[2-(trans-4-Amino-cyclohexylamino)-6-(2,2-diphenyl-ethylamino)-purin-9-yl]-5-(5-ethyl-4H-[1,2,4]triazol-3-yl)-tetrahydro-furan-3,4-diol;
(2R,3R,4S,5R)-2-[6-(2,2-Diphenyl-ethylamino)-2-(1Shydroxymethyl-2-methyl-propylamino)-purin-9-yl]-5-(5-ethyl-4H-[1,2,4]triazol-3-yl)-tetrahydro-furan-3,4-diol;
(2R,3R,4S,5R)-2-[6-(2,2-Diphenyl-ethylamino)-2-(2-morpholin-4-yl-ethylamino)-purin-9-yl]-5-(5-ethyl-4H-[1,2,4]triazol-3-yl)-tetrahydro-furan-3,4-diol;
(2R,3R,4S,5R)-2-[6-(2,2-Diphenyl-ethylamino)-2-(pyrrolidin-3R-ylamino)-purin-9-yl]-5-(5-ethyl-4H-[1,2,4]triazol-3-yl)-tetrahydro-furan-3,4-diol;
(2R,3R,4S,5R)-2-[2-(trans-4-Amino-cyclohexylamino)-6-(2,2-diphenyl-ethylamino)-purin-9-yl]-5-(5-isopropyl-4H-[1,2,4]triazol-3-yl)-tetrahydro-furan-3,4-diol;
(2R,3R,4S,5R)-2-[6-(2,2-Diphenyl-ethylamino)-2-(1Shydroxymethyl-2-phenyl-ethylamino)-purin-9-yl]-5-(5-isopropyl-4H-[1,2,4]triazol-3-yl)-tetrahydro-furan-3,4-diol;
(2R,3R,4S,5R)-2-[2-(trans-4-Amino-cyclohexylamino)-6-(2,2-diphenyl-ethylamino)-purin-9-yl]-5-(5-ethyl-[1,2,4]oxadiazol-3-yl)-tetrahydro-furan-3,4-diol;
(2R,3R,4S,5R)-2-{6-(2,2-Diphenyl-ethylamino)-2-[2-(1-methyl-1H-imidazol-4-yl)-ethylamino]-purin-9-yl}-5-(5-ethyl-[1,2,4]oxadiazol-3-yl)-tetrahydro-furan-3,4-diol;
(2R,3R,4S,5R)-2-[6-(2,2-Diphenyl-ethylamino)-2-(2-piperidin-1-yl-ethylamino)-purin-9-yl]-5-(5-ethyl-[1,2,4]oxadiazol-3-yl)-tetrahydro-furan-3,4-diol;
(2R,3R,4S,5R)-2-[6-(2,2-Diphenyl-ethylamino)-2-(2-morpholin-4-yl-ethylamino)-purin-9-yl]-5-(5-ethyl-[1,2,4]oxadiazol-3-yl)-tetrahydro-furan-3,4-diol;
(2R,3R,4S,5R)-2-[6-(2,2-Diphenyl-ethylamino)-2-(1Shydroxymethyl-2-phenyl-ethylamino)-purin-9-yl]-5-(5-ethyl-[1,2,4]oxadiazol-3-yl)-tetrahydro-furan-3,4-diol;
(2R,3R,4S,5S)-2-[6-(2-Cyclohexyl-ethylamino)-2-(1Shydroxymethyl-2-phenyl-ethylamino)-purin-9-yl]-5-(5-methyl-oxazol-2-yl)-tetrahydro-furan-3,4-diol;
(2R,3R,4S,5S)-2-[6-(2,2-Diphenyl-ethylamino)-2-(pyrrolidin-3R-ylamino)-purin-9-yl]-5-(5-methyloxazol-2-yl)-tetrahydro-furan-3,4-diol;
(2R,3R,4S,5S)-2-{6-(2,2-Diphenyl-ethylamino)-2-[2-(1-methyl-1H-imidazol-4-yl)-ethylamino]-purin-9-yl}-5-(5-methyl-[1,3,4]thiadiazol-2-yl)-tetrahydro-furan-3,4-diol;
(2R,3R,4S,5S)-2-[6-(2,2-Diphenyl-ethylamino)-2-(1Shydroxymethyl-2-phenyl-ethylamino) -purin-9-yl]-5- (5-methyl-[1,3,4]thiadiazol-2-yl)-tetrahydro-furan-3,4-diol;
(2R,3R,4S,5S)-2-[6-(2,2-Diphenyl-ethylamino)-2-(1Shydroxymethyl-2-phenyl-ethylamino)-purin-9-yl]-5-(5-ethyl-[1,3,4]oxadiazol-2-yl)-tetrahydro-furan-3,4-diol;
(2R,3R,4S,5S)-2-[6-(2,2-Diphenyl-ethylamino)-2-(2-piperidin-1-yl)-ethylamino)-purin-9-yl]-5-(5-ethyl-[1,3,4]oxadiazol-2-yl)-tetrahydro-furan-3,4-diol;
(2R,3R,4S,5S)-2-[6-(2,2-Diphenyl-ethylamino)-2-(2-morpholin-4-yl-ethylamino)-purin-9-yl]-5-(5-ethyl-[1,3,4]oxadiazol-2-yl)-tetrahydro-furan-3,4-diol;
(2R,3R,4S,5S)-2-[6-(2,2-Diphenyl-ethylamino)-2-(2-pyridin-2-yl-ethylamino)-purin-9-yl]-5-(5-ethyl-[1,3,4]oxadiazol-2-yl)-tetrahydro-furan-3,4-diol;
(2R,3R,4S,5S)-2-[2-(trans-4-Amino-cyclohexylamino)-6-(2,2-diphenyl-ethylamino)-purin-9-yl]-5-(5-ethyl-[1,3,4]oxadiazol-2-yl)-tetrahydro-furan-3,4-diol;
(2R,3R,4S,5S)-2-[6-(2,2-Diphenyl-ethylamino)-2-(pyrrolidin-3R-ylamino)-purin-9-yl]-5-(5-ethyl-[1,3,4]oxadiazol-2-yl)-tetrahydro-furan-3,4-diol;
(2R,3R,4S,5R)-2-[6-(2,2-Diphenyl-ethylamino)-2-(2-pyridin-2-yl-ethylamino)-purin-9-yl]-5-(5-ethyl-4H-[1,2,4]triazol-3-yl)-tetrahydro-furan-3,4-diol;
(2R,3R,4S,5S)-2-[6-(2,2-Diphenyl-ethylamino)-2-(2-piperidin-1-yl-ethylamino)-purin-9-yl]-5-(5-methyl-[1,3,4]thiadiazol-2-yl)-tetrahydro-furan-3,4-diol;
N-(2-{6-(2,2-Diphenyl-ethylamino)-9-[5R-(5-ethyl-[1,2,4]oxadiazol-3-yl)-3R,4S-dihydroxy-tetrahydrofuran-2R-yl]-9H-purin-2-ylamino}-ethyl)-guanidin;
(2R,3R,4S,5R)-2-[2-(trans-4-Amino-cyclohexylamino)-6-(2,2-diphenyl-ethylamino)-purin-9-yl]-5-(1-ethyl-1H-[1,2,4]triazol-3-yl)-tetrahydro-furan-3,4-diol;
(2R,3R,4S,5R)-2-{6-(2,2-Diphenyl-ethylamino)-2-[2-(1-methyl-1H-imidazol-4-yl)-ethylamino]-purin-9-yl}-5-(1-ethyl-1H-[1,2,4]triazol-3-yl)-tetrahydro-furan-3,4-diol;
(2R,3R,4S,5R)-2-[6-(2,2-Diphenyl-ethylamino)-2-(2-piperidin-1-yl-ethylamino)-purin-9-yl]-5-(1-ethyl-1H-[1,2,4]triazol-3-yl)-tetrahydro-furan-3,4-diol;
(2R,3R,4S,5R)-2-[6-(2,2-biphenyl-ethylamino)-2-(2-pyridin-2-yl-ethylamino)-purin-9-yl]-5-(1-ethyl-1H-[1,2,4]triazol-3-yl)-tetrahydro-furan-3,4-diol;
(2R,3R,4S,5R)-2-[6-(2,2-Diphenyl-ethylamino)-2-(pyrrolidin-3R-ylamino)-purin-9-yl]-5-(1-ethyl-1H-[1,2,4]triazol-3-yl)-tetrahydro-furan-3,4-diol;
(2R,3R,4S,5R)-2-[6-(2,2-Diphenyl-ethylamino)-2-(1Rhydroxy-2-phenyl-ethylamino)-purin-9-yl]-5-(1-ethyl-1H-[1,2,4]triazol-3-yl)-tetrahydro-furan-3,4-diol;
(2R,3R,4S,5S)-2-[2-(trans-4-Amino-cyclohexylamino)-6-(1-ethyl-propylamino)-purin-9-yl]-5-(5-cyclopropyl-[1,3,4]oxadiazol-2-yl)-tetrahydro-furan-3,4-diol;
(2S,3S,4R,5R)-2-(5-Cyclopropyl-[1,3,4]oxadiazol-2-yl)-5-{6-(1-ethyl-propylamino)-2-[2-(1-methyl-1Himidazol-4-yl)-ethylamino]-purin-9-yl}-tetrahydrofuran-3,4-diol;
(2S,3S,4R,5R)-2-(5-Cyclopropyl-[1,3,4]oxadiazol-2-yl)-5-[6-(1-ethyl-propylamino)-2-(2-piperidin-1-ylethylamino)-purin-9-yl]-tetrahydro-furan-3,4-diol;
(2R,3R,4S,5S)-2-[2-Cyclopentylamino-6-(1-ethylpropylamino) -purin-9-yl]-5-(5-cyclopropyl-[1,3,4]oxadiazol-2-yl)-tetrahydro-furan-3,4-diol;
(2S,3S,4R,5R)-2-(5-Cyclopropyl-[1,3,4]oxadiazol-2-yl)-5-[6-(1-ethyl-propylamino)-2-(pyrrolidin-3Rylamino)-purin-9-yl]-tetrahydro-furan-3,4-diol;
(2R,3R,4S,5S)-2-[2-(2-Cyclohexyl-ethylamino)-6-(1-ethyl-propylamino)-purin-9-yl]-5-(5-cyclopropyl-[1,3,4]oxadiazol-2-yl)-tetrahydro-furan-3,4-diol;
(2R,3R,4S,5S)-2-[6-(1-Ethyl-propylamino)-2-(1Shydroxymethyl-2-methyl-propylamino)-purin-9-yl]-5-(5-cyclopropyl-[1,3,4]oxadiazol-2-yl)-tetrahydro-furan-3,4-diol;
oder ein Salz oder Solvat eines beliebigen davon ist.

19. Verbindung der Formel (I), die (2R,3R,4S,5S)-2-[6-(1-Ethyl-propylamino)-2-(2-piperidin-1-yl-ethylamino)-purin-9-yl]-5-(5-ethylisoxazol-3-yl)tetrahydrofuran-3,4-diol oder ein Salz oder Solvat davon ist.

20. Pharmazeutische Zusammensetzung, die eine Verbindung der Formel (I) wie in einem der Ansprüche 1 bis 19 definiert oder ein pharmazeutisch akzeptables Salz oder Solvat davon im Gemisch mit einem oder mehreren pharmazeutisch akzeptablen Verdünnungsstoffen oder Trägern umfasst.

21. Verbindung der Formel (I) wie in einem der Ansprüche 1 bis 19 definiert oder ein pharmazeutisch akzeptables Salz oder Solvat davon zur Verwendung als Pharmazeutikum.

22. Verwendung einer Verbindung der Formel (I) wie in einem der Ansprüche 1 bis 19 definiert oder eines pharmazeutisch akzeptablen Salzes oder Solvats davon in der Herstellung eines Medikaments zur Behandlung von entzündlichen Erkrankungen, z.B. Asthma oder chronisch obstruktiver Lungenerkrankung (COPD).

23. Verfahren zur Herstellung einer Verbindung der Formel (I) wie in einem der Ansprüche 1 bis 19 definiert, welches das Umsetzen einer Verbindung der Formel (II): 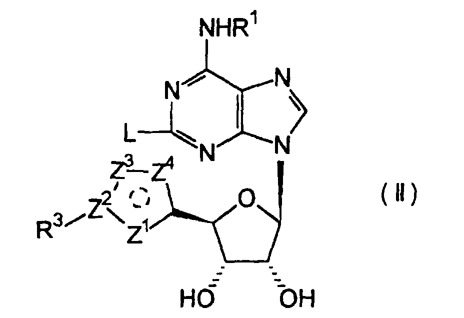 worin L eine Abgangsgruppe darstellt, oder eines geschützten Derivats davon mit einer Verbindung der Formel R²NH₂ oder einem geschützten Derivat davon umfasst, worin R¹, R², R³, Z¹, Z², Z³ und Z⁴ wie in einem der Ansprüche 1 bis 19 definiert sind.

24. Verbindung der Formel (II) 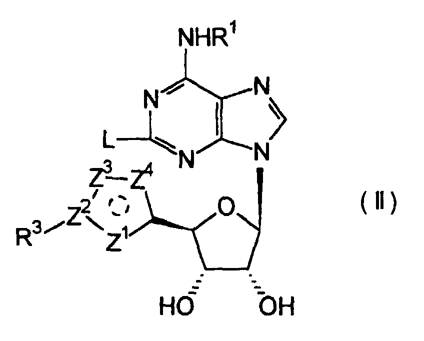 worin L eine Abgangsgruppe darstellt oder ein geschütztes Derivat davon und R¹, R³, Z¹, Z², Z³ und Z⁴ wie in einem der Ansprüche 1 bis 19 definiert sind.

25. Verbindung der Formel (III): 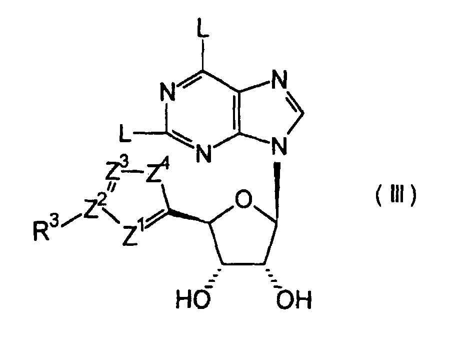 worin L eine Abgangsgruppe darstellt oder ein geschütztes Derivat davon und R³, Z¹, Z², Z³ und Z⁴ wie in einem der Ansprüche 1 bis 19 definiert sind.

26. Verbindung der Formel (IV): 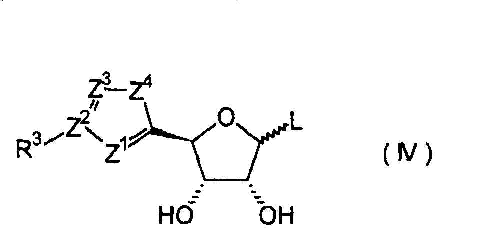 worin L eine Abgangsgruppe darstellt oder ein geschütztes Derivat davon und R³, Z¹, Z², Z³ und Z⁴ wie in einem der Ansprüche 1 bis 19 definiert sind.

27. Verbindung der Formel (V): 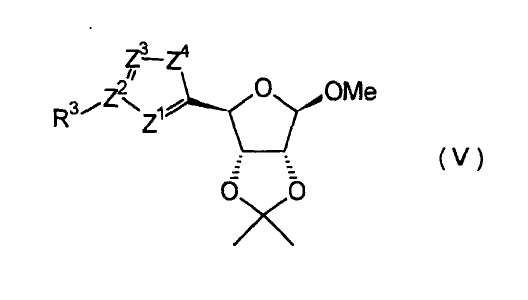 worin R³, Z¹, Z², Z³ und Z⁴ wie in einem der Ansprüche 1 bis 19 definiert sind.

28. Verbindung der Formel (VI): 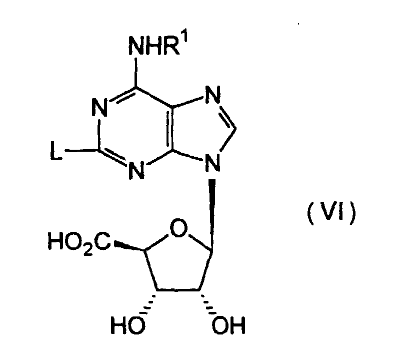 worin L eine Abgangsgruppe darstellt oder ein geschütztes Derivat davon und R¹ wie in einem der Ansprüche 1 bis 19 definiert ist, mit der Massgabe, dass L nicht Chlor darstellt, wenn R¹ Ph₂CHCH₂ darstellt.

## Revendications

1. Composé de formule (I) : 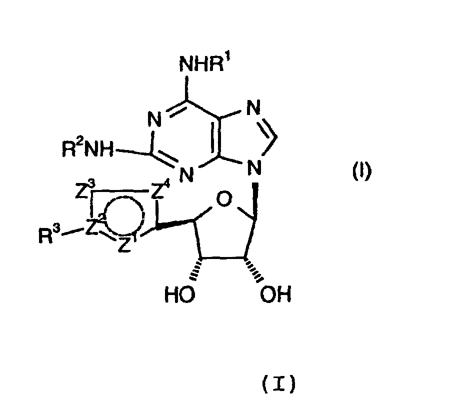 dans laquelle R¹ et R² représentent indépendamment un groupe choisi parmi :
(i) (cycloalkyle en C₃₋₈)- ;
(ii) l'hydrogène ;
(iii) aryl₂CHCH₂- ;
(iv) (cycloalkyle en C₃₋₈)-(alkyle en C₁₋₆)- ;
(v) (alkyle en C₁₋₈)- ;
(vi) aryl(alkyle en C₁₋₆)- ;
(vii) R⁴R⁵N-(alkyle en C₁₋₆)- ;
(viii) (alkyle en C₁₋₆)-CH(CH₂OH)- ;
(ix) aryl(alkyle en C₁₋₅)-CH(CH₂OH)- ;
(x) aryl(alkyle en C₁₋₅)-C(CH₂OH)₂- ;
(xi) un cycloalkyle en C₃₋₈ substitué indépendamment par un ou plusieurs (par exemple, 1, 2 ou 3) groupes -(CH₂)ₚR⁶ ;
(xii) H₂NC(=NH)NH(alkyle en C₁₋₆)- ;
(xiii) un groupe de formule 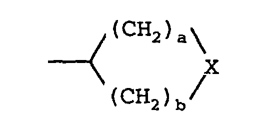 ou un tel groupe dans lequel un atome de carbone de méthylène contigu à X, ou les deux, le cas échéant, est substitué par un méthyle ;
(xiv) -(alkyle en C₁₋₆)-OH ;
(xv) un halogénoalkyle en C₁₋₈ ;
(xvi) un groupe de formule 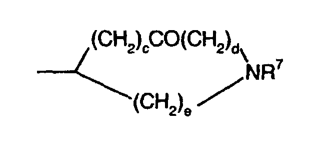
(xvii) un aryle ; et
(xviii) -(CH₂)_{f}SO₂NH_{g}(alkyle en C₁₋₄)_{2-g} ou -(CH₂)_{f}SO₂NH_{g}(aryl(alkyle en C₁₋₄))_{2-g} où f est 2 ou 3 et g est un entier de 0 à 2 ;
Z² représente C ou N ;
Z¹, Z³ et Z⁴ conjointement avec Z² et l'atome de carbone forment un cycle aromatique hétérocyclique à 5 membres ;
R³ représente un alkyle en C₁₋₃ ou un cyclopropyle, sauf que lorsque Z² représente C, R³ peut également représenter CH₂OH ;
R⁴ et R⁵ représentent indépendamment l'hydrogène, un alkyle en C₁₋₆, un aryle, un aryl(alkyle en C₁₋₆)- ou NR⁴R⁵ peut représenter conjointement le pyridinyle, le pyrrolidinyle, le pipéridinyle, le morpholinyle, l'azétidinyle, l'azépinyle, le pipérazinyle ou un N-(alkyle en C₁₋₆)pipérazinyle ;
R⁶ représente OH,NH₂, NHCOCH₃ ou un halogène ;
R⁷ représente l'hydrogène, un alkyle en C₁₋₆, un aryl(alkyle en C₁₋₆)- ou -CO(alkyle en C₁₋₆) ;
X représente NR⁷, O, S, SO ou SO₂ ;
p représente 0 ou 1 ;
a et b représentent indépendamment un entier de 0 à 4 à condition que a + b soit dans l'intervalle de 3 à 5 ;
c, d et e représentent indépendamment un entier de 0 à 3 à condition que c + d + e soit dans l'intervalle de 2 à 3 ;
à condition que le motif 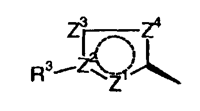 ne représente pas un des groupes suivants : 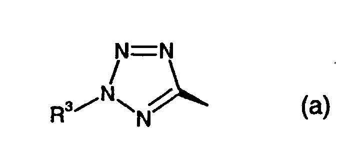 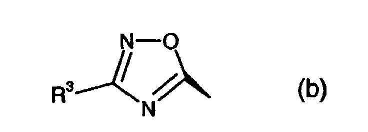 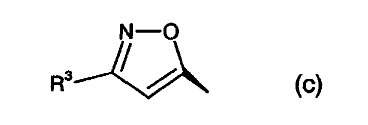
et des sels ou solvates quelconques de ceux-ci.

2. Composé de formule (I) selon la revendication 1 dans laquelle R¹ et R² ne représentent pas tous deux un hydrogène.

3. Composé selon la revendication 1 ou la revendication 2 dans lequel R¹ représente un aryl₂CHCH₂-(alkyle en C₁₋₈)-, l'hydrogène ou un aryl(alkyle en C₁₋₆)-.

4. Composé selon l'une quelconque des revendications 1 à 3 dans lequel R¹ représente Ph₂CHCH₂.

5. Composé selon l'une quelconque des revendications 1 à 4 dans lequel R² représente l'éthyl-pipéridin-1-yle, PhCH₂CH(CH₂OH)-, -CH(CH₂OH)(CH(CH₃)₂), le trans-4-amino-cyclohexyle, 2-(1-méthyl-1H-imidazoyl-4-yl)CH₂CH₂-, l'éthyl-morpholin-1-yle, le pyrrolidin-3-yle, l'éthyl-pyridin-2-yle, H₂NC(=NH)NH(CH₂)₂-, le cyclopentyle ou l'éthylcyclohexyle.

6. Composé selon l'une quelconque des revendications 1 à 5 dans lequel R² représente 2-(1-(alkyle en C₁₋₃)-1H-imidazoyl-4-yl)CH₂CH₂-.

7. Composé selon l'une quelconque des revendications 1 à 6 dans lequel Z² représente C.

8. Composé selon l'une quelconque des revendications 1 à 7 dans lequel Z⁴ représente N.

9. Composé selon l'une quelconque des revendications 1 à 8 dans lequel R³ représente le méthyle, l'éthyle, le n-propyle, l'isopropyle, le cyclopropyle ou CH₂OH (lorsque Z² représente C).

10. Composé selon l'une quelconque des revendications 1 à 9 dans lequel R³ représente le méthyle, l'éthyle ou le cyclopropyle.

11. Composé selon l'une quelconque des revendications 1 à 10 dans lequel R³ représente l'éthyle.

12. Composé selon l'une quelconque des revendications 1 à 11 dans lequel R⁴ et R⁵ représentent indépendamment l'hydrogène ou un aryle ou NR⁴R⁵ peut représenter conjointement le pyrrolidinyle, le pipéridinyle, le morpholinyle, l'azétidinyle, l'azépinyle, le pipérazinyle ou le N-méthylpipérazinyle.

13. Composé selon l'une quelconque des revendications 1 à 12 dans lequel R⁶ représente OH ou NH₂.

14. Composé selon l'une quelconque des revendications 1 à 13 dans lequel X représente NR⁷, O, S ou SO₂.

15. Composé selon l'une quelconque des revendications 1 à 14 dans lequel le motif 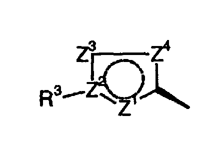 représente un des groupes suivants :

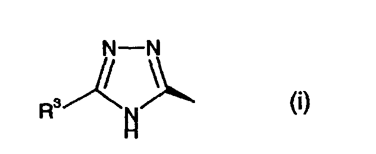

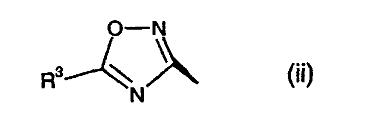

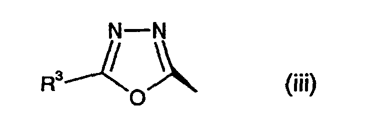

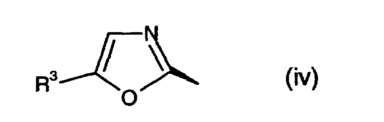

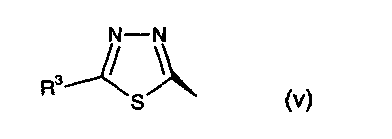

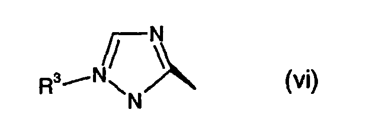

16. Composé selon l'une quelconque des revendications 1 à 15 dans lequel le motif 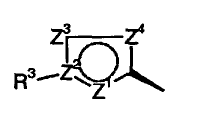 représente (i), (ii), (iii) et (vi) comme définis dans la revendication 15.

17. Composé selon l'une quelconque des revendications 1 à 16 dans lequel le motif 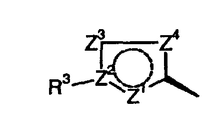 représente (i) comme défini dans la revendication 15.

18. Composé de formule (I) qui est
(2R,3R,4S,5R)-2-[6-(2,2-Diphényléthylamino)-2-(2-pyrrolidin-1-yl-éthylamino)-purin-9-yl]-5-(5-méthyl-4H-[1,2,4]triazol-3-yl)-tétrahydrofuran-3,4-diol ;
(2R,3R,4S,5R)-2-[6-(2,2-Diphényléthylamino)-2-(1S-hydroxyméthyl-2-phényléthylamino)-purin-9-yl]-5-(5-méthyl-4H-[1,2,4]triazol-3-yl)-tétrahydrofuran-3,4-diol ;
(2R,3R,4S,5R)-2-[6-(2,2-Diphényléthylamino)-2-[2-(1-méthyl-1H-imidazol-4-yl)éthylamino]-purin-9-yl]-5-(5-méthyl-4H-[1,2,4]triazol-3-yl)-tétrahydrofuran-3,4-diol ;
(2R,3R,4S,5R)-2-[2-(trans-4-Aminocyclohexylamino)-6-(2,2-diphényléthylamino)-purin-9-yl)]-5-(5-méthyl-4H-[1,2,4]triazol-3-yl)-tétrahydrofuran-3,4-diol ;
(2R,3R,4S,5R)-2-[6- (2,2-Diphényléthylamino)-2-(1S-hydroxyméthyl-2-phényléthylamino)-purin-9-yl]-5-(5-éthyl-4H-[1,2,4]triazol-3-yl)-tétrahydrofuran-3,4-diol ;
(2R,3R,4S,5R)-2-[2-(trans-4-Aminocyclohexylamino) -6- (2,2-diphényléthylamino)-purin-9-yl)]-5-(5-éthyl-4H-[1,2,4]triazol-3-yl)-tétrahydrofuran-3,4-diol ;
(2R,3R,4S,5R)-2-[6-(2,2-Diphényléthylamino)-2-(1S-hydroxyméthyl-2-méthylpropylamino)-purin-9-yl]-5-(5-éthyl-4H-[1,2,4]triazol-3-yl)-tétrahydrofuran-3,4-diol ;
(2R,3R,4S,5R)-2-[6-(2,2-Diphényléthylamino)-2-(2-morpholin-4-yl-éthylamino)-purin-9-yl]-5-(5-éthyl-4H-[1,2,4]triazol-3-yl)-tétrahydrofuran-3,4-diol ;
(2R,3R,4S,5R)-2-[6-(2,2-Diphényléthylamino)-2-(pyrrolidin-3R-ylamino)-purin-9-yl]-5-(5-éthyl-4H-[1,2,4]triazol-3-yl)-tétrahydrofuran-3,4-diol;
(2R,3R,4S,5R)-2-[2-(trans-4-Aminocyclohexylamino)-6-(2,2-diphényléthylamino)-purin-9-yl]-5-(5-isopropyl-4H-[1,2,4]triazol-3-yl)-tétrahydrofuran-3,4-diol ;
(2R,3R,4S,5R)-2-[6-(2,2-Diphényléthylamino)-2-(1S-hydroxyméthyl-2-phényléthylamino)-purin-9-yl]-5-(5-isopropyl-4H-[1,2,4]triazol-3-yl)-tétrahydrofuran-3,4-diol ;
(2R,3R,4S,5R)-2-[2-(trans-4-Aminocyclohexylamino)-6-(2,2-diphényléthylamino)-purin-9-yl]-5-(5-éthyl-[1,2,4]oxadiazol-3-yl)-tétrahydrofuran-3,4-diol ;
(2R,3R,4S,5R)-2-[6-(2,2-Diphényléthylamino)-2-[2-(1-méthyl-1H-imidazol-4-yl)-éthylamino]-purin-9-yl]-5-(5-éthyl-[1,2,4]oxadiazol-3-yl)-tétrahydrofuran-3,4-diol ;
(2R,3R,4S,5R)-2-[6-(2,2-Diphényléthylamino)-2-(2-pipéridin-1-yl-éthylamino)-purin-9-yl]-5-(5-éthyl-[1,2,4]oxadiazol-3-yl)-tétrahydrofuran-3,4-diol ;
(2R,3R,4S,5R)-2-[6-(2,2-Diphényléthylamino)-2-(2-morpholin-4-yl-éthylainino)-purin-9-yl]-5- (5-éthyl-[1,2,4]oxadiazol-3-yl)-tétrahydrofuran-3,4-diol ;
(2R,3R,4S,5R)-2-[6-(2,2-Diphényléthylamino)-2-(1S-hydroxyméthyl-2-phényléthylamino)-purin-9-yl]-5-(5-éthyl-[1,2,4]oxadiazol-3-yl)-tétrahydrofuran-3,4-diol ;
(2R,3R,4S,5S)-2-[6-(2-Cyclohexyl-éthylamino)-2-(1S-hydroxyméthyl-2-phényléthylamino)-purin-9-yl]-5-(5-méthyl-oxazol-2-yl)-tétrahydrofuran-3,4-diol ;
(2R,3R,4S,5S)-2-[6-(2,2-Diphényléthylamino)-2-(pyrrolidin-3R-ylamino)-purin-9-yl]-5-(5-méthyl-oxazol-2-yl)-tétrahydrofuran-3,4-diol ;
(2R,3R,4S,5S)-2-[6-(2,2-Diphényléthylamino)-2-[2-(1-méthyl-1H-imidazol-4-yl)-éthylamino]-purin-9-yl]-5-(5-méthyl-[1,3,4]thiadiazol-2-yl)-tétrahydrofuran-3,4-diol ;
(2R,3R,4S,5S)-2-[6-(2,2-Diphényléthylamino)-2-(1S-hydroxyméthyl-2-phényléthylamino)-purin-9-yl]-5-(5-méthyl-[1,3,4]thiadiazol-2-yl)-tétrahydrofuran-3,4-diol ;
(2R,3R,4S,5S)-2-[6-(2,2-Diphényléthylamino)-2-(1S-hydroxyméthyl-2-phényléthylamino)-purin-9-yl]-5-(5-éthyl-[1,3,4]oxadiazol-2-yl)-tétrahydrofuran-3,4-diol;
(2R,3R,4S,5S)-2-[6-(2,2-Diphényléthylamino)-2-(2-pipéridin-1-yl-éthylamino)-purin-9-yl]-5-(5-éthyl-[1,3,4]oxadiazol-2-yl)-tétrahydrofuran-3,4-diol ;
(2R,3R,4S,5S)-2-[6-(2,2-Diphényléthylamino)-2-(2-morpholin-4-yl-éthylamino)-purin-9-yl]-5-(5-éthyl-[1,3,4]oxadiazol-2-yl)-tétrahydrofuran-3,4-diol ;
(2R,3R,4S,5S)-2-[6-(2,2-Diphényléthylamino)-2-(2-pyridin-2-yl-éthylamino)-purin-9-yl]-5-(5-éthyl-[1,3,4]oxadiazol-2-yl)-tétrahydrofuran-3,4-diol ;
(2R,3R,4S,5S)-2-[2-(trans-4-Aminocyclohexylamino)-6-(2,2-diphényléthylamino)-purin-9-yl]-5-(5-éthyl-[1,3,4]oxadiazol-2-yl)-tétrahydrofuran-3,4-diol ;
(2R,3R,4S,5S)-2-[6-(2,2-diphényléthylamino)-2-(pyrrolidin-3R-ylamino)-purin-9-yl]-5-(5-éthyl-[1,3,4]oxadiazol-2-yl)-tétrahydrofuran-3,4-diol ;
(2R,3R,4S,5R)-2-[6-(2,2-diphényléthylamino)-2-(2-pyridin-2-yl-éthylamino)-purin-9-yl]-5-(5-éthyl-4H-[1,2,4]triazol-3-yl)-tétrahydrofuran-3,4-diol ;
(2R,3R,4S,5S)-2-[6-(2,2-diphényléthylamino)-2-(2-pipéridin-1-yl-éthylamino)-purin-9-yl]-5-(5-méthyl-[1,3,4]thiadiazol-2-yl)-tétrahydrofuran-3,4-diol ;
N-(2-[6-(2,2-Diphényléthylamino)-9-[5R-(5-éthyl-[1,2,4]oxadiazol-3-yl)-3R,4S-dihydroxy-tétrahydro-furan-2R-yl]-9H-purin-2-ylamino)-éthyl)-guanidine ;
(2R,3R,4S,5R)-2-[2-(trans-4-Aminocyclohexylamino)-6-(2,2-diphényléthylamino)-purin-9-yl]-5-(1-éthyl-1H-[1,2,4]triazol-3-yl)-tétrahydrofuran-3,4-diol ;
(2R,3R,4S,5R)-2-[6-(2,2-Diphényléthylamino)-2-[2-(1-méthyl-1H-imidazol-4-yl)éthylamino]-purin-9-yl]-5-(1-éthyl-1H-[1,2,4]triazol-3-yl)-tétrahydrofuran-3,4-diol ;
(2R,3R,4S,5R)-2-[6-(2,2-Diphényléthylamino)-2-(2-pipéridin-1-yl-éthylamino)-purin-9-yl]-5-(1-éthyl-1H-[1,2,4]triazol-3-yl)-tétrahydrofuran-3,4-diol ;
(2R,3R,4S,5R)-2-[6-(2,2-Diphényléthylamino)-2-(2-pyridin-2-yl-éthylamino) -purin-9-yl]-5-(1-éthyl-1H-[1,2,4]triazol-3-yl)-tétrahydrofuran-3,4-diol;
(2R,3R,4S,5R)-2-[6-(2,2-Diphényléthylamino)-2-(pyrrolidin-3R-ylamino)-purin-9-yl]-5-(1-éthyl-1H-[1,2,4]triazol-3-yl)-tétrahydrofuran-3,4-diol;
(2R,3R,4S,5R)-2-[6-(2,2-Diphényléthylamino)-2-(1R-hydroxy-2-phényl-éthylamino)-purin-9-yl]-5-(1-éthyl-1H-[1,2,4]triazol-3-yl)-tétrahydrofuran-3,4-diol ;
(2R,3R,4S,5S)-2-[2-(trans-4-Aminocyclohexylamino)-6-(1-éthyl-propylamino)-purin-9-yl]-5-(5-cyclopropyl-[1,3,4]oxadiazol-2-yl)-tétrahydrofuran-3,4-diol ;
(2S,3S,4R,5R)-2-(5-Cyclopropyl-[1,3,4]oxadiazol-2-yl)-5-[6-(1-éthyl-propylamino)-2-[2-(1-méthyl-1H-imidazol-4-yl)-éthylamino]-purin-9-yl]-tétrahydrofuran-3,4-diol ;
(2S,3S,4R,5R)-2-(5-Cyclopropyl-[1,3,4]oxadiazol-2-yl)-5-[6-(1-éthyl-propylamino)-2-(2-pipéridin-1-yl-éthylamino)-purin-9-yl]-tétrahydrofuran-3,4-diol ;
(2R,3R,4S,5S)-2-[2-(Cyclopentylamino-6-(1-éthylpropylamino)-purin-9-yl]-5-(5-cyclopropyl-[1,3,4]oxadiazol-2-yl)-tétrahydrofuran-3,4-diol ;
(2S,3S,4R,5R)-2-(5-Cyclopropyl-[1,3,4]oxadiazol-2-yl)-5-[6-(1-éthyl-propylamino)-2-(pyrrolidin-3R-ylamino)-purin-9-yl]-tétrahydrofuran-3,4-diol ;
(2R,3R,4S,5S)-2-[2-(2-Cyclohexyl-éthylamino)-6- (1-éthyl-propylamino)-purin-9-yl]-5-(5-cyclopropyl-[1,3,4]oxadiazol-2-yl)-tétrahydrofuran-3,4-diol ;
(2R,3R,4S,5S)-2-[6- (1-Ethyl-propylamino)-2-(1S-hydroxyméthyl-2-méthyl-propylamino)-purin-9-yl]-5- (5-cyclopropyl-[1,3,4]oxadiazol-2-yl)-tétrahydrofuran-3,4-diol ;
ou un sel ou solvate de l'un quelconque de ceux-ci.

19. Composé de formule (I) qui est le
(2R,3R,4S,5R)-2-[6-(1-éthyl-propylamino)-2-(2-pipéridin-1-yl-éthylamino)-purin-9-yl]-5-(5-éthylisoxazol-3-yl)tétrahydrofuran-3,4-diol ou un sel ou solvate de celui-ci.

20. Composition pharmaceutique comprenant un composé de formule (I) tel que défini dans l'une quelconque des revendications 1 à 19 ou un sel ou solvate acceptable pharmaceutiquement de celui-ci en mélange avec un ou plusieurs diluants ou supports acceptables pharmaceutiquement.

21. Composé de formule (I) tel que défini dans l'une quelconque des revendications 1 à 19 ou un sel ou solvate acceptable pharmaceutiquement de celui-ci pour utilisation en tant que substance pharmaceutique.

22. Utilisation d'un composé de formule (I) tel que défini dans l'une quelconque des revendications 1 à 19 ou un sel ou solvate acceptable pharmaceutiquement de celui-ci dans la fabrication d'un médicament pour le traitement de maladies inflammatoires, par exemple l'asthme ou la bronchopneumopathie chronique obstructive (COPD).

23. Procédé pour la préparation d'un composé de formule (I) tel que défini dans l'une quelconque des revendications 1 à 19 qui comprend la réaction d'un composé de formule (II) 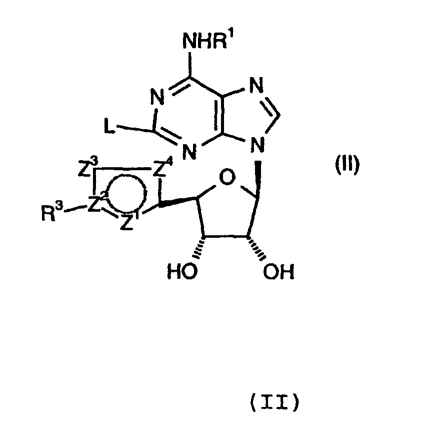 dans laquelle L représente un groupe labile ou un dérivé protégé de celui-ci avec un composé de formule R²NH₂ ou un dérivé protégé de celui-ci, dans lequel R¹, R², R³, Z¹, Z², Z³ et Z⁴ sont comme définis dans l'une quelconque des revendications 1 à 19.

24. Composé de formule (II) 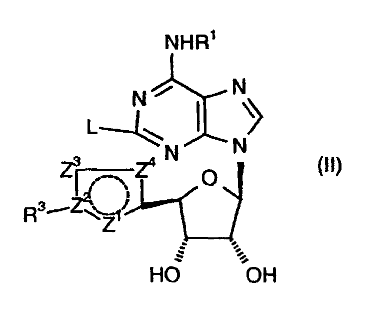 dans laquelle L représente un groupe labile ou un dérivé protégé de celui-ci et R¹, R³, Z¹, Z², Z³ et Z⁴ sont comme définis dans l'une quelconque des revendications 1 à 19.

25. Composé de formule (III) 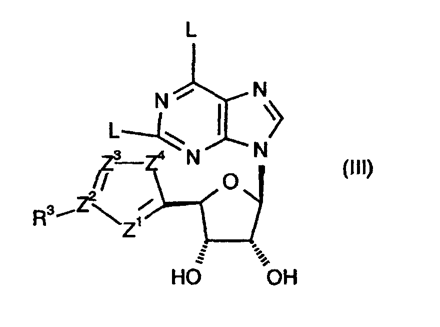 dans laquelle L représente un groupe labile ou un dérivé protégé de celui-ci et R³, Z¹, Z², Z³ et Z⁴ sont comme définis dans l'une quelconque des revendications 1 à 19.

26. Composé de formule (IV) 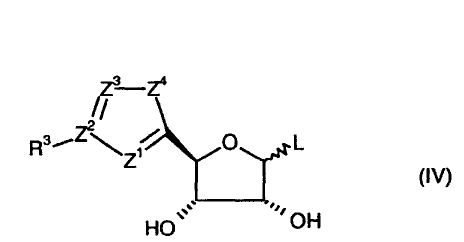 dans laquelle L représente un groupe labile ou un dérivé protégé de celui-ci et R³, Z¹, Z², Z³ et Z⁴ sont comme définis dans l'une quelconque des revendications 1 à 19.

27. Composé de formule (V) 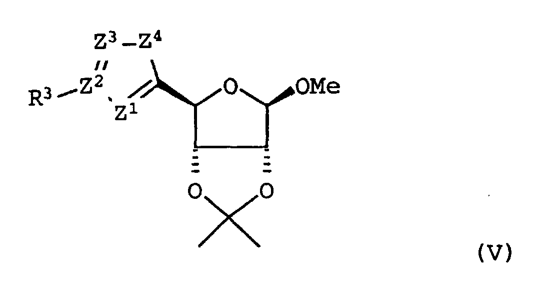 dans laquelle R³, Z¹, Z², Z³ et Z⁴ sont comme définis dans l'une quelconque des revendications 1 à 19.

28. Composé de formule (VI) 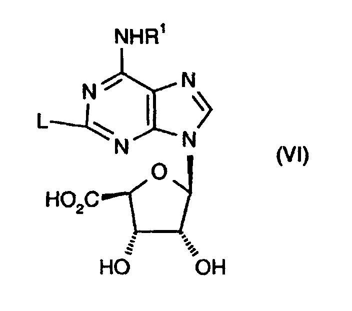 dans laquelle L représente un groupe labile ou un dérivé protégé de celui-ci et R¹ est comme défini dans l'une quelconque des revendications 1 à 19, à condition que lorsque R¹ représente Ph₂CHCH₂, L ne représente pas le chlore.
